(19) Europäisches Patentamt
European Patent Office
Office européen des brevets

(11) EP 4 703 361 A1

(12) **EUROPEAN PATENT APPLICATION**
published in accordance with Art. 153(4) EPC

(43) Date of publication:
**04.03.2026 Bulletin 2026/10**

(21) Application number: **24795760.8**

(22) Date of filing: **29.03.2024**

(51) International Patent Classification (IPC):
***C07D 487/04*** *(2006.01)* ***A61K 31/4985*** *(2006.01)*
***A61P 13/12*** *(2006.01)* ***A61P 25/18*** *(2006.01)*
***A61P 25/28*** *(2006.01)* ***A61P 1/16*** *(2006.01)*

(52) Cooperative Patent Classification (CPC):
**A61K 31/4985; A61K 31/501; A61P 1/16; A61P 9/00; A61P 9/06; A61P 9/10; A61P 9/12; A61P 13/12; A61P 17/00; A61P 17/06; A61P 25/00; A61P 25/04; A61P 25/08; A61P 25/14; A61P 25/16;**
(Cont.)

(86) International application number:
**PCT/CN2024/084776**

(87) International publication number:
**WO 2024/222379 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.04.2023 CN 202310462302**

(71) Applicants:
- **Shanghai Institute of Materia Medica, Chinese Academy of Sciences**
  **Shanghai 201203 (CN)**
- **China Pharmaceutical University**
  **Nanjing, Jiangsu 210009 (CN)**

(72) Inventors:
- **SHEN, Jianhua**
  **Shanghai 201203 (CN)**
- **CAO, Zhengyu**
  **Nanjing, Jiangsu 210009 (CN)**
- **WANG, Kai**
  **Shanghai 201203 (CN)**
- **LIU, Mengru**
  **Nanjing, Jiangsu 210009 (CN)**
- **XU, Yuanyuan**
  **Shanghai 201203 (CN)**
- **NIU, Bo**
  **Nanjing, Jiangsu 210009 (CN)**
- **XU, Tifei**
  **Shanghai 201203 (CN)**
- **ZHAO, Fang**
  **Nanjing, Jiangsu 210009 (CN)**
- **HU, Yixin**
  **Nanjing, Jiangsu 210009 (CN)**
- **REN, Younan**
  **Nanjing, Jiangsu 210009 (CN)**

(74) Representative: **dompatent Partnerschaft von Patentanwälten und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

# (54) PYRIDAZINONE TRPC4/5 INHIBITOR AND USE THEREOF

(57) Disclosed in the present invention is a pyridazinone TRPC4/5 inhibitor; the structure thereof is as shown in formula I. In the formula, the definition of each substituent is as stated in the description and the claims. The compound of the present invention has strong inhibitory activity on TRPC4 and TRPC5. After oral administration, the compound shows a remarkable treatment effect on a hypertensive nephropathy model and a hepatic fibrosis model, and therefore has good potential to be developed as a drug for treating kidney and liver diseases.



Processed by Luminess, 75001 PARIS (FR)

(Cont. next page)

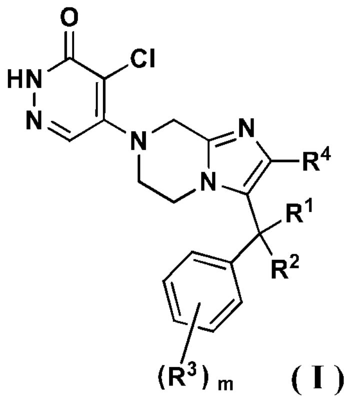

(I)

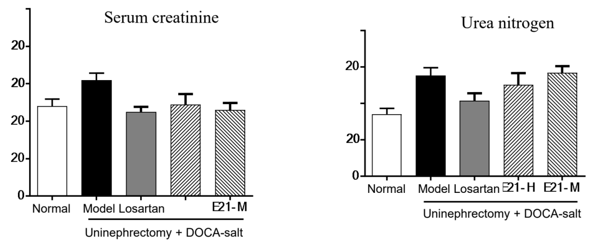


Figure 3

(52) Cooperative Patent Classification (CPC): (Cont.)
**A61P 25/18; A61P 25/22; A61P 25/24; A61P 25/28; A61P 29/00; A61P 35/00; C07D 487/04**

## Description

### Technical Field

The present invention relates to the field of medicinal chemistry, and in particular to pyridazinone compounds, preparation methods and uses thereof. The compound is an inhibitor of TRPC4 and TRPC5 ion channels and has potential application value in preventing, delaying or treating a disease related to abnormal TRPC4/5 function or expression.

### Background Art

Transient receptor potential (TRP) ion channels are a class of six-transmembrane channel proteins that are widely distributed in the peripheral and central nervous systems. They are molecular sensors that sense changes in the cellular environment, transmit signals, and maintain cellular homeostasis. Based on sequence similarity, mammalian TRP ion channels can be divided into six subfamilies: TRPA, TRPC, TRPM, TRPML, TRPP, and TRPV. Classical transient receptor potential channel (TRPC) is non-selective cation channel that allows $Ca^{2+}$ and $Na^{+}$ to pass through. Mammalian TRPC is composed of six members: TRPC1, TRPC3, TRPC4, TRPC5, TRPC6, and TRPC7. The amino acid sequences of TRPC3, TRPC6, and TRPC7 have high consistency, while the sequences of TRPC1, TRPC4, and TRPC5 have high consistency. TRPC family proteins exist in the form of homo- or hetero-tetramers and function as ion channels. TRPC5 easily forms hetero-tetramers with TRPC 1 and TRPC4.

TRPC4/5 is expressed in multiple parts of the brain, including the hippocampus, amygdala, frontal cortex, etc., and is related to the occurrence of neurological diseases such as anxiety, depression, epilepsy, and addiction. TRPC4 or TRPC5 gene knockout or application of TPRC4/5 inhibitors can alleviate anxiety and depression symptoms in mice. BI-1358894, a TRPC4/5 inhibitor developed by Boehringer Ingelheim Pharmaceuticals, is currently undergoing clinical trials for the treatment of mental illnesses such as depression, borderline personality disorder, and post-traumatic stress disorder.

Podocytes are special cells that form the glomerular filtration barrier. TRPC5 is also expressed in glomerular podocytes, and its abnormal function is associated with the development of chronic kidney disease. TRPC5 is involved in the regulation of angiotensin-induced cell migration and actin remodeling. In various animal models of kidney disease, knocking out TRPC5 or inhibiting TPRC5 activity can significantly reduce proteinuria levels, protect podocytes from damage, and inhibit the progression of kidney disease. Goldfinch Biotechnology Company has launched a Phase II clinical trial of GFB-887, a TRPC4/5 inhibitor, for the treatment of diabetic nephropathy and focal segmental glomerulosclerosis.

TRPC4/5 is expressed in skin keratinocytes and plays an important role in regulating keratinocyte differentiation. Therefore, targeted regulation of TRPC4/5 may be used to treat skin lesions with abnormal keratinocyte function as the pathological basis.

TRPC5 is expressed in the liver and portal vein bed. A series of endogenous phospholipids that play an important role in the formation of liver disease, such as lysolecithin (LPC), can significantly activate TRPC5. In a mouse model having cholestatic liver disease induced by bile acid feeding, TRPC5 gene knockout can alleviate liver damage and abnormal hepatic lipid metabolism. It can be seen that TRPC5 is also a potential target for the treatment of liver diseases.

TRPC4/5 are also expressed in peripheral sensory neurons and are involved in pain perception. $TRPC4^{-/-}$ mice are resistant to mustard oil-induced pain and have an increased pain threshold compared to wild-type animals. In various mouse models having neuropathic pain, inflammatory pain, spontaneous pain, etc., TRPC5 gene knockout or TRPC5 inhibitors can effectively alleviate mechanical allodynia, and this effect is intrinsically related to the biological function regulated by LPC.

TRPC4/5 is also associated with cardiovascular disease. Activation of TRPC5 channel can significantly promote the proliferation and migration of vascular smooth muscle cells. Inhibition of TRPC5 can significantly reduce hypoxia-ischemia-induced cell apoptosis and oxidative stress in endothelial cells, reduce endothelium-dependent vasoconstriction, and inhibit the formation of atherosclerotic plaques. It can also reduce the expression of genes related to myocardial hypertrophy and maintain the stability of cardiac electrical activity, thereby playing an anti-atherosclerotic, anti-myocardial hypertrophy and anti-arrhythmia role. Furthermore, TRPC5-mediated calcium influx is a key signaling mechanism for the release of multiple endothelium-derived contractile factors, contributing to the development of hypertension. TRPC5 promotes the proliferation of vascular smooth muscle cells and increases oxidative stress, thereby promoting the occurrence of hypertension.

TRPC4/5 are associated with tumor angiogenesis, tumor drug resistance and metastasis. TRPC5 is highly expressed in colorectal cancer, promoting extracellular calcium influx and thus activating the Wnt5a/β-catenin pathway, reducing the tumor differentiation level and increasing cancer stem cells. TRPC5 expression level is negatively correlated with the prognosis of colorectal cancer patients. TRPC5 can also reduce E-cadherin, increase the expression of mesenchymal markers, and promote tumor cell migration, invasion, and proliferation. In addition, calcium ion dysregulation caused by activation of TRPC5 can promote anaerobic inducible factor 1 (HIF-1)-mediated tumor angiogenesis,

allowing cancer cells to escape from sites exposed to high doses of anticancer drugs and obtain additional nutrients to help tumor survival, thereby reducing the effectiveness of chemotherapy. Inhibition of TRPC5 reduces chemotherapy resistance in breast cancer models. On the other hand, activation of TRPC4/5 can also induce tumor cell death through calcium overload. For example, the natural product (-)-Englerin A is a potent TRPC4/5 agonist that exhibits anti-proliferative effects on various tumor cells such as renal cancer, lung cancer, breast cancer, and skin cancer.

WO2019055966 discloses pyridazinone compounds represented by the following general formula (A) as TRPC4/5 inhibitors and discloses example compounds JO and QF, wherein the heterocyclic rings in the middle part of the molecules are '[1,2,4]triazolo[4,3-a]tetrahydropyrazine' and 'imidazo[4,5-c]tetrahydropyridine', respectively.

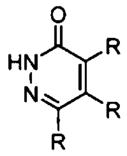

(A)

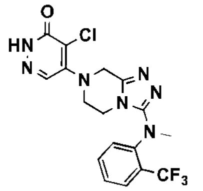

JO

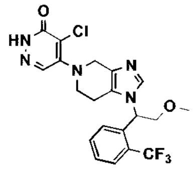

QF

WO2020191056 discloses pyridazinone compounds represented by the following general formula (I) as TRPC4/5 inhibitors, and discloses example compound 100 (i.e., the aforementioned drug GFB-887), wherein the heterocyclic ring in the middle part of the molecule is 'tetrahydropyrido[3,4-d]pyrimidine'.

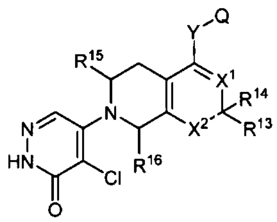

(I)

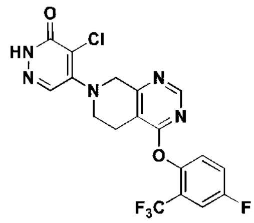

Compound 100

WO2022001767 discloses pyridazinone compounds represented by the following general formula (I) as TRPC4/5 inhibitors, wherein Y is defined as -O-, -S-, -$NR^8$-, -$CH_2$-, -C(=O)- or - S(=O)-. WO2022001767 discloses example compound L002, wherein the heterocyclic ring in the middle of the molecule is 'imidazo[1,2-a]tetrahydropyrazine'.

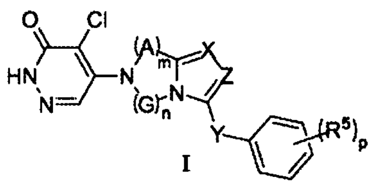

I

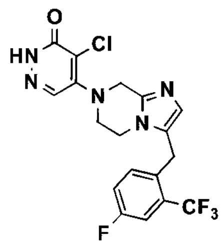

L002

## Summary of the Invention

The main object of the present invention is to provide a class of pyridazinone compounds used as TRPC4 and TRPC5 inhibitors, preparation method and therapeutic use thereof.

The first aspect of the present invention provides a compound represented by general formula (I), a stereoisomer, deuterated derivative and pharmaceutically acceptable salt thereof;

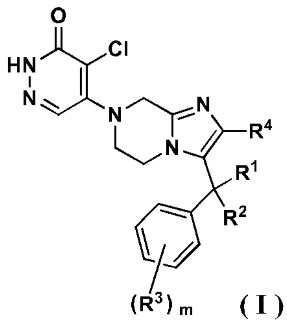

( I )

wherein $R^1$ and $R^2$ are each independently H, C1-C6 alkyl, C1-C6 alkoxy, azido, or hydroxyl; or $R^1$ and $R^2$ together with a carbon atom to which they are attached form

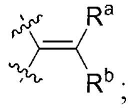

the C1-C6 alkyl or C1-C6 alkoxy is unsubstituted or further substituted with one or more substituents selected from the group consisting of a C3-C8 cycloalkyl, a C6-C10 aryl, a halogenated C6-C10 aryl, a deuterium atom, or a halogen; and $R^a$ and $R^b$ are each independently H, C1-C6 alkyl, or cyano.

the premise is that $R^1$ and $R^2$ are not H at the same time;

m is an integer from 1 to 4 (1, 2, 3 or 4), each $R^3$ is independently H, halogen, C1-C6 alkyl, C1-C6 haloalkyl, C1-C6 alkoxy, C1-C6 haloalkoxy, or nitro;

$R^4$ is H, halogen, C1-C6 alkyl, or C1-C6 haloalkyl.

In another preferred embodiment, $R^1$ and $R^2$ are each independently H, C1-C3 alkyl, or C1-C3 alkoxy, and the C1-C3 alkoxy is unsubstituted or further substituted with a deuterium atom;

the premise is that $R^1$ and $R^2$ are not H at the same time;

or $R^1$ and $R^2$ together constitute $=CH_2$.

In a preferred embodiment, $R^1$ is H, and $R^2$ is methoxy or deuterated methoxy.

In another preferred embodiment, each $R^3$ is independently H, halogen, C1-C3 alkyl, C1-C3 haloalkyl, or nitro.

In another preferred embodiment, $R^4$ is H.

In another preferred embodiment, the compound has a structure as shown in general formula (I-a);

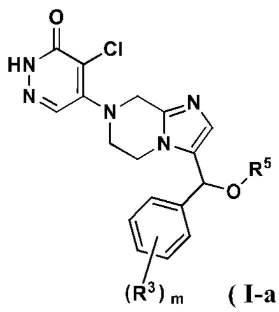

( I-a )

wherein m is 1, 2 or 3; each $R^3$ is independently H, halogen, C1-C3 alkyl, C1-C3 haloalkyl, or nitro; and $R^5$ is C1-C3 alkyl or C1-C3 deuterated alkyl.

In another preferred embodiment, the compound is selected from the compounds listed in claim 6.

The second aspect of the present invention provides a pharmaceutical composition comprising the compound according to the first aspect of claim 1, the stereoisomer, deuterated derivatives and pharmaceutically acceptable salt thereof, and a pharmaceutically acceptable excipient.

The pharmaceutical excipients include pharmaceutically acceptable carriers, excipients, sustained-release agents, odorants, flavoring agents, etc.

In the pharmaceutical composition, the compound of the present invention is the active ingredient, and its weight

accounts for 0.1 to 99.9% of the total weight of the pharmaceutical composition, and the rest is pharmaceutically acceptable excipient; the preferred ratio of the compound of the present invention to the excipient is that the compound of the present invention as the active ingredient accounts for more than 60% of the total weight, and the remaining part accounts for 0-40% of the total weight, and the amount of the remaining part is preferably 1-20%, and most preferably 1-10%.

The compound or pharmaceutical compositions of the present invention can be prepared into various dosage forms, such as tablets, capsules, powders, syrups, solutions, suspensions, sprays, creams, ointments, gels, transdermal patches, etc., based on conventional processes in the field of pharmaceutical preparations, and can be present in suitable solid or liquid carriers or diluents. The pharmaceutical composition of the present invention can also be stored in a suitable sterile device for injection or infusion.

The compound or pharmaceutical compositions of the present invention can be administered to mammals, including humans and animals. The routes of administration include oral administration, nasal inhalation, topical skin administration, intravenous injection, intramuscular injection, subcutaneous injection, etc. In another preferred embodiment, the preferred route of administration of the compound or pharmaceutical composition of the present invention is oral administration.

Solid dosage forms for oral administration of the compound or pharmaceutical composition of the present invention include capsules, tablets, pills, powders and granules. Solid carriers include starch, lactose, dicalcium phosphate, microcrystalline cellulose, sucrose and kaolin, while liquid carriers include sterile water, polyethylene glycol, non-ionic surfactants and edible oils (such as corn oil, peanut oil and sesame oil), as long as they are suitable for the characteristics of the active ingredient and the specific administration method required. Adjuvants commonly used in preparing pharmaceutical compositions may also advantageously be included, such as flavorings, colorings, preservatives and antioxidants such as vitamin E, vitamin C, BHT and BHA.

Liquid dosage forms for oral administration include pharmaceutically acceptable emulsions, solutions, suspensions, syrups or tinctures. In addition to the active ingredient, the liquid dosage form may contain inert diluents conventionally used in the art, such as water or other solvents, solubilizers and emulsifiers, for example, ethanol, isopropyl alcohol, ethyl carbonate, ethyl acetate, propylene glycol, 1,3-butylene glycol, dimethylformamide and oils, in particular cottonseed oil, peanut oil, corn germ oil, olive oil, castor oil and sesame oil or mixtures of these substances. Besides such inert diluents, the composition may also include additives such as wetting agents, emulsifying and suspending agents, sweeteners, flavoring and perfuming agents.

In addition to the active ingredient, suspensions may contain suspending agents such as, for example, ethoxylated isostearyl alcohol, polyoxyethylene sorbitol and sorbitan ester, microcrystalline cellulose, aluminum methoxide and agar, or mixtures of these substances.

When used as a pharmaceutical preparation, the compound of the present invention is preferably in a unit dose, each dose containing 0.01 mg to 200 mg, preferably 0.5 mg to 50 mg, of the active ingredient, for single or divided use. Regardless of the route of administration, the optimal individual dose should be determined based on the specific treatment. Generally, the treatment starts with a low dose and gradually increases until the optimal dosage is reached.

The third aspect of the present invention provides use of a compound represented by general formula (I) or (I-a), a stereoisomer thereof, or a pharmaceutically acceptable salt thereof, or a pharmaceutical composition thereof, as an inhibitor of TRPC4 and/or TRPC5 ion channels, for preparing a medicament for preventing, delaying or treating a disease related to abnormal function or expression of TRPC4 and/or TRPC5.

In the present invention, the disease related to the abnormal function and expression of TRPC4 and/or TRPC5 includes but is not limited to epilepsy, mental illness, neurodegenerative disease, kidney disease, pain, epilepsy, cardiovascular disease, cancer, skin disease, liver disease, and the like.

In a preferred embodiment, the disease associated with abnormal function or expression of TRPC4 and/or TRPC5 is a mental illness. The mental illness includes a disease related to low mood, such as borderline personality disorder, depression, dysthymia, postpartum depression, bipolar disorder, etc.; the mental illness also includes a condition related to anxiety and fear, such as post-traumatic stress disorder, panic disorder, agoraphobia, social phobia, generalized anxiety disorder, social anxiety disorder, separation anxiety, etc.; the mental illness also includes affective disorders and mental disorders caused by various other reasons, such as schizophrenia, mania, obsessive-compulsive disorder, apathy, neurasthenia, paranoia, etc.

In another preferred embodiment, the mental illness is borderline personality disorder, depression, anxiety disorder, or post-traumatic stress disorder.

The compound or pharmaceutical composition thereof of the present invention can be used alone or in combination with drugs with other mechanisms for treating mental illness. Drugs with other mechanisms include but are not limited to tricyclic antidepressant (TCA), monoamine oxidase inhibitor (MAOI), serotonin (5-HT) reuptake inhibitor (SSRI), serotonin and norepinephrine reuptake inhibitor (SNRI), norepinephrine-specific 5-HT antidepressant (NaSSA), 5-HT receptor antagonist and reuptake inhibitor (SARI), etc. SSRI and SNRI are preferred. Representative SSRI drugs include fluoxetine, paroxetine, citalopram, sertraline, fluvoxamine, etc. Representative SNRI drugs include duloxetine,

venlafaxine, milnacipran and the like.

In another preferred embodiment, the disease associated with abnormal function or expression of TRPC4 and/or TRPC5 is a neurodegenerative disease. The neurodegenerative disease includes Alzheimer's disease (AD), Parkinson's disease, Huntington's disease, amyotrophic lateral sclerosis (ALS), memory impairment, amnesia, aphasia, chronic fatigue syndrome, Creutzfeldt-Jakob disease, dissociative amnesia, fugue amnesia, learning disorder, sleep disorder and other brain disorders caused by trauma or aging.

In another preferred embodiment, the disease associated with abnormal function or expression of TRPC4 and/or TRPC5 is pain disorder. The pain syndrome includes nociceptive pain, mechanical pain, inflammatory pain, cancer pain, and neuropathic pain (e.g., osteoarthritis pain, rheumatoid arthritis pain, postherpetic neuralgia, and pain caused by burns). The pain can be chronic pain or acute pain.

In another preferred embodiment, the disease associated with abnormal function or expression of TRPC4 and/or TRPC5 is epilepsy. Epilepsy can be caused by excitotoxicity of various origins. In general, excessive neuronal firing can drive epileptic activity. Compounds that reduce the hyperexcitability of relevant neuronal populations have significant potential in reducing epileptic activity. Inhibition of TRPC5-mediated calcium influx may reduce hyperexcitability and thus reduce epileptic activity.

In another preferred embodiment, the disease associated with abnormal function or expression of TRPC4 and/or TRPC5 is kidney disease. The kidney disease includes extreme kidney damage and chronic kidney damage caused by various reasons, such as kidney damage caused by toxic substances, kidney damage caused by viral or bacterial infection, hypertensive nephropathy, diabetic nephropathy, glomerulonephritis, lupus erythematosus nephritis, IgA nephropathy, nephrotic syndrome, membranous nephropathy, minimal change nephropathy, and also includes hereditary kidney diseases such as polycystic kidney disease, focal segmental glomerulosclerosis, etc.

In another preferred embodiment, the disease related to the abnormal function or expression of TRPC4 and/or TRPC5 is a skin disease. Furthermore, the skin disease is associated with abnormal function of keratinocytes, such as but not limited to psoriasis, ichthyosis, palmoplantar keratoderma, Olmsted's disease, toad skin disease or menopausal keratoderma.

In another preferred embodiment, the disease related to the abnormal function or expression of TRPC4 and/or TRPC5 is a disease related to the cardiovascular system. The disease includes but is not limited to hypertension, atherosclerosis, coronary heart disease, angina pectoris, myocardial infarction, arrhythmia, stroke, pulmonary hypertension, etc.

In another preferred embodiment, the disease associated with abnormal function or expression of TRPC4 and/or TRPC5 is liver disease. The disease includes but is not limited to cholestatic liver disease, alcoholic steatohepatitis, non-alcoholic steatohepatitis, viral hepatitis, autoimmune liver disease, chemical liver injury, liver fibrosis, and cirrhosis.

Cholestatic liver disease is a general term for hepatobiliary diseases caused by various reasons and characterized by cholestasis as the main manifestation. Cholestasis refers to disorders in the formation, secretion and excretion of bile caused by various reasons inside and outside the liver. Clinically, it can manifest as itching, fatigue, darker urine and jaundice. Persistent cholestasis can cause hepatitis and liver fibrosis, and in severe cases can lead to cirrhosis, liver failure and even death. The main causes of cholestasis include genetic factors, pregnancy, drugs, alcohol, hepatitis B, hepatitis C, primary biliary cholangitis (PBC), primary sclerosing cholangitis (PSC), autoimmune hepatitis, metabolic-related fatty liver disease, etc. Among them, PBC and PSC are the most important cholestatic liver diseases and the main causes of cholestatic liver fibrosis in adults. Ursodeoxycholic acid is the main drug for the treatment of PBC, but there are still many unmet needs for the treatment of patients with poor response, and there is no effective treatment for PSC.

TRPC5 is expressed in the liver. Knocking out the TRPC5 gene in mice with cholestatic liver disease can effectively alleviate liver damage and abnormal liver lipid metabolism, suggesting that TRPC5 inhibitors have the potential to treat cholestatic liver disease. In a preferred embodiment, the cholestatic liver disease is PBC and PSC.

In another preferred embodiment, the disease associated with abnormal function or expression of TRPC4 and/or TRPC5 is cancer. The disease includes but is not limited to renal cell carcinoma, breast cancer, colorectal cancer, hepatocellular carcinoma, etc.

Lysolecithin (LPC) can directly activate TRPC5, independent of other indirect activation mechanisms such as the GPCR-PLC pathway. A series of biological effects of LPC are related to its activation of TRPC5. Therefore, TRPC5 inhibitors may be used to combat diseases related to LPC level disorders. The diseases include but are not limited to atherosclerosis, cardiovascular disease, central and peripheral nervous system diseases, osteoarthritis, hepatitis, hepatocellular carcinoma, obesity, bacterial infection, parasitic disease, diabetes, tumors, pain, etc.

The fourth aspect of the present invention provides a method for preparing a compound represented by general formula (I-a), a stereoisomer, deuterated derivative and pharmaceutically acceptable salt thereof, as shown in Route A. Wherein, halo is a halogen atom, THP represents a tetrahydropyranyl protecting group, Boc represents a tert-butyloxycarbonyl protecting group, and the other substituents are defined the same as those in the aforementioned general formula (I-a);

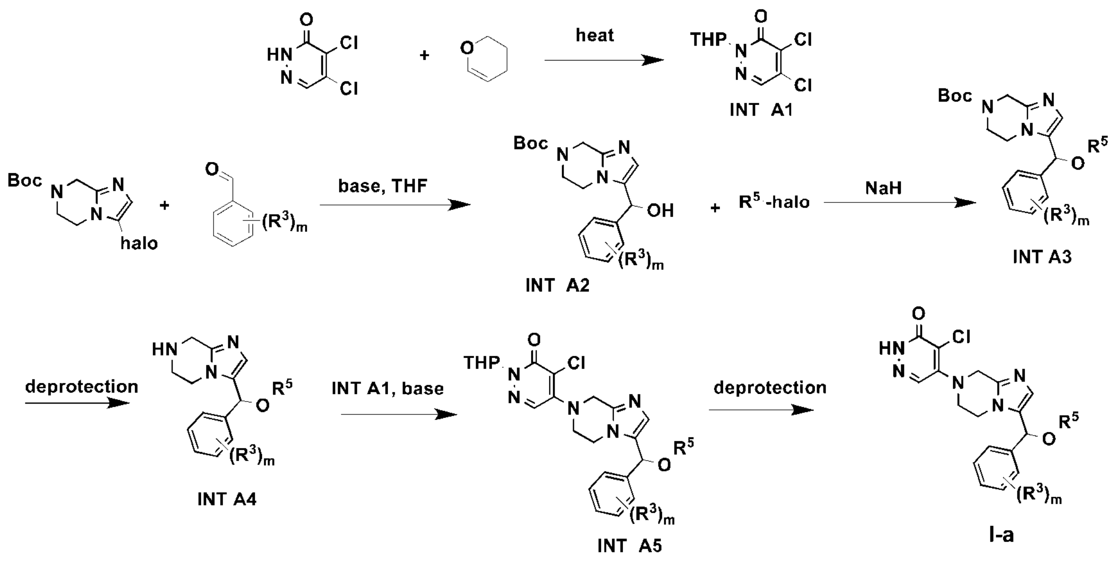


**Route A**

The synthesis method shown in Route A comprises the following steps:

(1.1) Chloropyridazinone (commercially available) is THP-protected to produce an intermediate INT-A1. The reaction is typically carried out in tetrahydrofuran (THF) using p-toluenesulfonic acid as a catalyst. The reaction typically requires heating at 50-100°C.

(1.2) Tert-butyl 3-halo-5,6-dihydroimidazo[1,2-a]pyrazine-7(8H)-carboxylate (commercially available) is dehalogenated and activated by a strong organic base, followed by a nucleophilic addition reaction with a benzaldehyde starting material (commercially available) to produce an intermediate INT-A2. The reaction is carried out in an ultra-dry aprotic solvent; the solvent may be, for example, but not limited to, dichloromethane (DCM) or tetrahydrofuran (THF), preferably tetrahydrofuran; the organic base may include, but not be limited to, an alkyl lithium reagent or a Grignard reagent; the reaction temperature may be from -80°C to room temperature;

(1.3) Intermediate INT-A2 is reacted with $R^5$-halo under the action of NaH to generate an intermediate INT-A3; the reaction is carried out in an aprotic solvent, such as but not limited to tetrahydrofuran (THF) and N,N-dimethylformamide (DMF); the reaction temperature is generally 0 °C to room temperature;

(1.4) Boc protecting group is removed from the intermediate INT-A3 to generate an intermediate INT-A4; the reaction is usually carried out in the presence of an acidic reagent; the acidic reagent may be, but is not limited to, trifluoroacetic acid or hydrogen chloride;

(1.5) Intermediate INT-A4 undergoes a substitution reaction with INT-A1 in the presence of a base to yield an intermediate INT-A5. The reaction is carried out in a polar solvent such as DMF or acetonitrile or without a solvent. The base is typically an organic base such as, but not limited to, triethylamine or diisopropylethylamine. The reaction requires heating, typically at a temperature of 50-120°C.

(1.6) The THP protecting group of the intermediate INT-A5 is removed to generate a compound represented by the general formula (I-a); the deprotection reaction is usually carried out under the action of an acidic reagent; the acidic reagent is for example but not limited to trifluoroacetic acid, hydrogen chloride, and p-toluenesulfonic acid.

Those skilled in the art can adjust the reaction conditions appropriately based on literature data or actual conditions encountered during the synthesis process.

It should be understood that within the scope of the present invention, the above-mentioned technical features of the present invention and the technical features specifically described below (such as examples) can be combined with each other to form new or preferred technical solutions. Each feature disclosed in the specification may be replaced by any alternative feature that provides the same, equal or similar purpose. Due to limited space, they will not be elaborated on herein one by one.

## Brief Description of the Drawings

Figure 1 shows the effects of compound E21 on UACR in nephrotic rats (Mean ± SEM for each group; #, P<0.05 vs

normal group; *, P<0.05 vs model group; **, P<0.01 vs model group; n=8-10).

Figure 2 shows the effects of compound E21 on the total 24-hour urine protein in nephrotic rats (Mean ± SEM for each group; #, P < 0.05 vs normal group; ##, P < 0.01 vs normal group; *, P < 0.05 vs model group; **, P < 0.01 vs model group; n = 8-10).

Figure 3 shows the effects of compound E21 on serum creatinine (Scr) and blood urea nitrogen (BUN) in rats (Mean ± SEM for each group; #, P < 0.05 vs normal group; *, P < 0.05 vs model group; n = 8-10).

Figure 4 shows the effects of compound E21 on the mRNA levels of nephrin and podocin, slit diaphragm proteins in rat kidney (Mean + SEM for each group; #, P < 0.05 vs normal group; ##, P < 0.01 vs normal group; *, P < 0.05 vs model group; **, P < 0.01 vs model group; n = 8-10).

Figure 5 shows the effects of compound E21 on serum transaminases ALT and AST in mice with liver fibrosis induced by bile duct ligation (BDL) (Mean ± SEM for each group; ##, P<0.01 vs blank control; *, P<0.05 vs BDL; n=4-5).

Figure 6 shows pathological images of a liver section stained with HE under a microscope (10× eyepiece, 10× objective lens, scale bar = 250 μm).

Figure 7 shows pathological images of a liver section stained with Masson under a microscope and the statistics of the percentage of collagen-positive area (Mean ± SEM for each group; ##, P<0.01 vs blank control; **, P<0.01 vs BDL; n=4-5).

Figure 8 shows pathological images of a liver section stained with Sirius red under a microscope and the statistics of the percentage of collagen-positive area (Mean ± SEM for each group; ##, P<0.01 vs blank control; **, P<0.01 vs BDL; n=4-5).

Figure 9 shows the effects of compound E21 on the mRNA levels of four fibrosis markers (ACTA2, COL1a1, TIMP-1, PDGFR) in liver tissue of BDL mice (Mean ± SEM for each group; #, P < 0.05 vs blank control; ##, P < 0.01 vs blank control; *, P < 0.05 vs BDL; **, P < 0.01 vs BDL; n = 4-5).

**Detailed Description**

After extensive and in-depth research, the inventors of the present application have developed a pyridazinone compound, which contains 'imidazo[1,2-a]tetrahydropyrazine', and the methylene group at the 5-position of the imidazole ring is substituted by a C1-C6 alkyl, a C1-C6 alkoxy, or the like. This type of compound has enhanced metabolic stability and potent inhibitory activity against TRPC4 and TRPC5. After oral administration, the compound has good drug distribution in the kidney and liver, and also shows significant therapeutic effects in hypertensive nephropathy models and liver fibrosis models. Therefore, it has good potential for development as a drug for the treatment of kidney disease or liver disease. On this basis, the present invention is completed.

**Term**

In the present invention, unless otherwise specified, the terms used have the general meanings commonly known to those skilled in the art.

In the present invention, the term "C1-C6" refers to having 1, 2, 3, 4, 5 or 6 carbon atoms, and so on.

In the present invention, the "alkyl" is a branched or straight-chain hydrocarbon group having a specific number of carbon atoms, and representative examples include but are not limited to methyl, ethyl, n-propyl, and isopropyl.

In the present invention, the "alkoxy" refers to an -O-alkyl. For example, the term "C1-C6 alkoxy" refers to a straight-chain or branched alkoxy having 1 to 6 carbon atoms. Representative examples include but are not limited to methoxy, ethoxy, n-propoxy, isopropoxy and butoxy.

In the present invention, the "haloalkyl" and "haloalkoxy" refer to groups in which the hydrogen atoms in an "alkyl" or "alkoxy" having a specific number of carbon atoms are partially or completely substituted by "halogen atoms".

In the present invention, the "cycloalkyl" refers to a non-aromatic cyclic aliphatic hydrocarbon group with a specific number of ring carbon atoms, and a "C3-C8 cycloalkyl" refers to a cyclic aliphatic hydrocarbon group consisting of 3 to 8 ring carbon atoms. The "cycloalkyl" described in the present invention includes not only aliphatic hydrocarbon groups with fully saturated carbon atoms, but also aliphatic hydrocarbon groups with unsaturated bonds in some of the carbon atoms. Examples of the "cycloalkyl" described in the present invention include, but are not limited to:

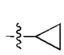, 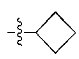, 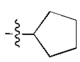, 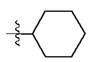, 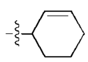.

In the present invention, "aryl" is defined as a monocyclic or bicyclic ring system consisting of a specific number of carbon atoms and obeying Hückel's rule, including phenyl and naphthyl.

In the present invention, "heteroaryl" is defined as a monocyclic or bicyclic ring system having a specific number of ring atoms and containing 1, 2, 3 or 4 heteroatoms (selected from N, O, S) and obeying Hückel's rule; examples of

"heteroaryl" include but are not limited to pyridine, pyrrole, imidazole, thiophene, benzimidazole, benzothiophene, benzofuran, etc.

In the present invention, "heterocyclyl" is defined as a saturated or partially unsaturated non-aromatic monocyclic and bicyclic ring system having a specific number of ring atoms and containing 1, 2, 3 or 4 heteroatoms (selected from N, O, S); examples of "heterocyclyl" include but are not limited to oxetanyl, dioxolanyl, morpholinyl, piperazinyl, pyrrolidinyl, piperidinyl, decahydroquinolinyl, benzotetrahydrofuranyl, etc.

In the present invention, the "halogen" includes fluorine, chlorine, bromine and iodine.

The term "substituted" as used herein means being replaced by one or more groups. Unless specified to occur on a particular atom, it indicates that it may occur on any atom where the number of substituents has not yet reached saturation. When multiple substituents are selected from the same series, they may be identical or different.

The term "optionally" used in the present invention means that the defined group may be selected from a series of candidate groups or may not be selected.

The "pharmaceutically acceptable salt" of the present invention may be a salt formed by an anion and a positively charged group on the compound of formula (I) or (I-a). Suitable anions include chloride ion, bromide ion, iodide ion, sulphate, nitrate, phosphate, citrate, methylsulfonate, trifluoroacetate, acetate, malate, toluenesulfonate, tartrate, fumarate, glutamate, glucuronoate, lactate, pentanedioate, or maleate ion. Similarly, salts can be formed from a cation and a negatively charged group on a compound. Suitable cations include sodium, potassium, magnesium, calcium and ammonium ion, such as tetramethylammonium ion.

In another preferred embodiment, "pharmaceutically acceptable salt" refers to a salt formed by a compound of formula (I) or (I-a) with an acid selected from the group consisting of: hydrofluoric acid, hydrochloric acid, hydrobromic acid, phosphoric acid, acetic acid, oxalic acid, sulfuric acid, nitric acid, methanesulfonic acid, aminosulfonic acid, salicylic acid, trifluoromethanesulfonic acid, naphthalenesulfonic acid, maleic acid, citric acid, acetic acid, lactic acid, tartaric acid, succinic acid, oxalic acid, pyruvic acid, malic acid, glutamic acid, p-toluenesulfonic acid, naphthalenesulfonic acid, ethanesulfonic acid, naphthalenedisulfonic acid, malonic acid, fumaric acid, propionic acid, oxalic acid, trifluoroacetic acid, stearic acid, pamoic acid, hydroxymaleic acid, phenylacetic acid, benzoic acid, glutamic acid, ascorbic acid, p-aminobenzenesulfonic acid, 2-acetoxybenzoic acid and isethionic acid; or sodium salt, potassium salt, calcium salt, aluminum salt or ammonium salt formed by the compound of formula (I) or (I-a) with an inorganic base; or methylamine salt, ethylamine salt or ethanolamine salt formed by the compound of formula (I) or (I-a) with an organic base.

The compound of formula (I) or (I-a) or pharmaceutically acceptable salt thereof is obtained by distillation, crystallization or recrystallization from water or an organic solvent. The compound may contain the solvent molecules used. In addition, different crystallization conditions may lead to different crystalline forms of the compound. Therefore, compounds of formula (I) or (I-a) or pharmaceutically acceptable salts thereof containing different stoichiometric amounts of crystallization solvents and all crystalline forms are within the scope of the present invention.

In the present invention, the "effective therapeutic amount" refers to a dosage that results in the cure, improvement, effective prevention, or significant reduction in the incidence of lesions or side effects in subjects receiving the treatment, compared to those not receiving such treatment. Additionally, it also includes an effective dose for enhancing normal physiological function.

In the present invention, the term "inhibitor" may also be expressed as "antagonist" or "blocker", which have equivalent meanings in the present invention and refer to a compound or mixture that can be used to reduce or inhibit biological activity.

TRPC family proteins exist not only as homotetramers, but also often as heterotetramers. Taking TRPC5 as an example, it not only forms TRPC5:C5 homotetramers, but also forms heterotetramers such as TRPC4:C5, TRPC1:C5, and TRPC1:C4:C5. Therefore, the TRPC5 inhibitor described in the present invention not only inhibits TRPC5 homotetramer channels, but also inhibits TRPC5 heterotetramer channels. Similarly, this also applies to TPRC4.

The compound of the general formula (I) or (I-a) of the present invention has chiral center, potential chiral center or unsaturated bond, and can form various forms of stereoisomer, such as racemate, enantiomer, diastereomer, E/Z isomers, cis-trans isomers, tautomer, etc. Unless otherwise indicated, in the specification and appended claims, a given chemical formula or name is intended to encompass all forms of stereoisomers, as well as mixtures consisting of individual isomers in varying proportions, and pharmaceutically acceptable salts thereof. Those skilled in the art can use commonly used laboratory separation methods to separate the compounds containing asymmetric center in the present invention to obtain single isomer, but this does not destroy the novelty of the compounds of the present invention.

Replacing hydrogen atom with deuterium atom to change the physical and chemical properties of compounds has become a structural modification method well known to those skilled in the art. Unless otherwise stated, the present invention intends to include deuterated forms of the compounds of formula (I) or (I-a).

The present invention will be further described below with reference to examples. It should be particularly pointed out that these examples are only used to illustrate the present invention, and are not intended to limit the present invention in any way. All parameters and other descriptions in the examples are based on quality unless otherwise stated. Unless otherwise specified, the fillers used for column chromatography separation are all silica gel. The experimental methods in

the following examples where specific conditions are not specified are generally carried out under conventional conditions or conditions recommended by the manufacturers.

Unless defined otherwise, all technical and scientific terms used herein have the same meanings as those commonly understood by one skilled in the art. In addition, any methods and materials similar or equivalent to those described herein can be applied to the present invention. The preferred implementation methods and materials described herein are for exemplary purposes only.

Abbreviations: DCM: dichloromethane; DIPEA: N,N-diisopropylethylamine; DMF: N,N-dimethylformamide; DMSO: dimethyl sulfoxide; TFA: trifluoroacetic acid; THF: tetrahydrofuran; EA: ethyl acetate; PE: petroleum ether; PPTS: pyridinium p-toluenesulfonate; DPPA: diphenylphosphoryl azide; DBU: 1,8-diazabicyclo[5.4.0]undec-7-ene.

## I. Preparation Example

### Example E1: 4-chloro-5-(3-((4-fluoro-2-(trifluoromethyl)phenyl)(methoxy)methyl)-5,6-dihydroimidazo[1,2-a]pyrazin-7(8H)-yl)pyridazin-3(2H)-one

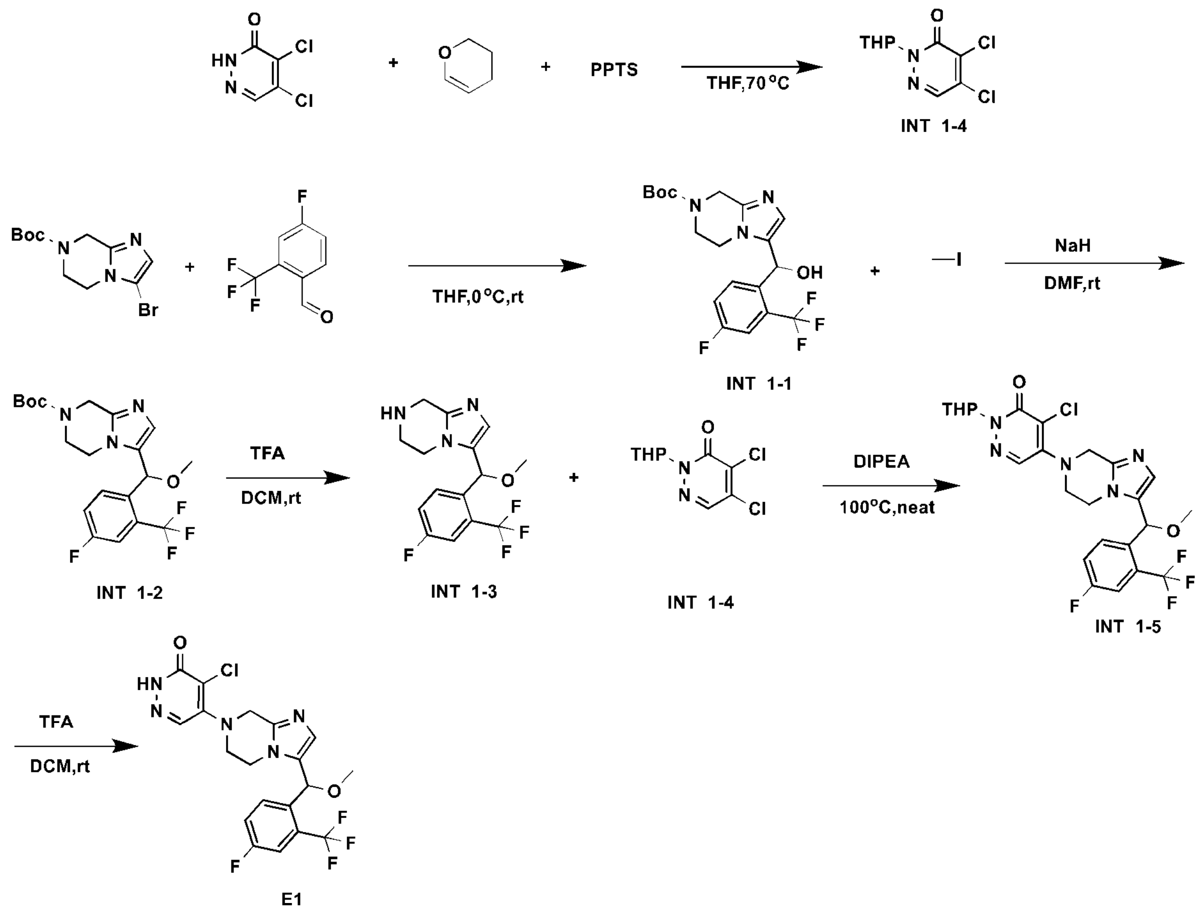

INT 1-4, INT 1-1, INT 1-2, INT 1-3, INT 1-4, INT 1-5, E1

### 1.1 Preparation of intermediate INT 1-1

7-Boc-3-bromo-5,6,7,8-tetrahydroimidazo[1,2-a]pyrazine (500 mg, 1.66 mmol) was added to a three-necked flask, and anhydrous THF was added. After nitrogen protection, the mixture was placed at 0 °C. Isopropylmagnesium chloride-lithium chloride complex (1.3 M/L in THF, 1.6 ml, 1.99 mmol) was slowly added dropwise, and the mixture was allowed to react at room temperature for 30 minutes. 4-fluoro-2-(trifluoromethyl)benzaldehyde (636.13 mg, 3.31 mmol) was dissolved in 1 ml of anhydrous THF and slowly added dropwise to the reaction mixture at 0 °C. Then, the mixture was reacted at room temperature for 3 h. After completion of the reaction monitored by TLC, the reaction mixture was separated by a flash silica gel column using MeOH/DCM as the mobile phase for elution to collect the target product, namely, the intermediate INT 1-1, which was about 500 mg.

### 1.2 Preparation of intermediate INT 1-2

The intermediate INT 1-1 (300 mg, 0.72 mmol) was dissolved in anhydrous DMF and added to a three-necked flask. Under nitrogen protection, NaH (60% dispersion in mineral oil, 58 mg, 1.45 mmol) and iodomethane (112.9 mg, 0.80 mmol) were added. The mixture was allowed to react at room temperature for 30 min. After completion of the reaction monitored by TLC, 2 ml of saturated ammonium chloride solution was added to quench the reaction. The reaction mixture was extracted with EA, and the organic phase was washed three times with saturated brine, dried over anhydrous magnesium sulfate, and filtered. The filtrate was concentrated and separated by flash silica gel chromatography using MeOH/DCM as the mobile phase to collect the target product, i.e., the intermediate INT 1-2, approximately 200 mg.

### 1.3 Preparation of intermediate INT 1-3

The intermediate INT 1-2 (200 mg, 0.47 mmol) was dissolved in DCM (3 ml), and TFA (1 ml) was added dropwise. The mixture was reacted at room temperature for 30 min. After completion of the reaction monitored by TLC, the solvent was removed by rotary evaporation to obtain 160 mg of a crude product.

### 1.4 Preparation of intermediate INT 1-4

4,5-Dichloro-3-hydroxypyridazine (2.5 g, 15.15 mmol) was added to a single-necked flask and dissolved in THF. 3,4-dihydro-2H-pyran (10.18 g, 121.21 mmol) and PPTS (760 mg, 3.03 mmol) were added. The reaction mixture was stirred at 70°C for 6 h and monitored by TLC. After the reaction was completed, the solvent was removed by rotary evaporation and the residue was separated by flash silica gel chromatography using EA/PE as the mobile phase for elution. The target product was collected and evaporated to dryness to obtain 2.88 g of a white solid, namely, intermediate INT 1-4.

### 1.5 Preparation of intermediate INT 1-5

The intermediate INT 1-3 (160 mg, 0.49 mmol) was added to a single-necked flask, and the intermediate INT 1-4 (133.7 mg, 0.53 mmol) and DIPEA (627.4 mg, 4.86 mmol) were added. The reaction mixture was placed at 100°C for overnight. After completion of the reaction monitored by TLC, the product was redissolved in DCM, washed three times with saturated brine, dried over anhydrous magnesium sulfate, filtered, and the filtrate was concentrated and separated by flash silica gel chromatography using MeOH/DCM as the mobile phase to elute the product. The target product, i.e., the intermediate INT 1-5, was collected, which was approximately 150 mg.

### 1.6 Preparation of Compound E1

The intermediate INT 1-5 (150 mg, 0.30 mmol) was dissolved in DCM (3 ml), and TFA (1 ml) was added dropwise. The mixture was reacted at room temperature for 30 min. After completion of the reaction monitored by TLC, the solvent was evaporated by rotary evaporation and the residue was purified by preparative liquid chromatography (C18 filler) using acetonitrile/water as the mobile phase. The target product was collected and evaporated to dryness to obtain 80 mg of a white solid, Example E1. 1H NMR (500 MHz, Methanol-d4) δ 8.00 (s, 1H), 7.93 (dd, J = 8.4, 5.5 Hz, 1H), 7.59 (d, J = 8.8 Hz, 2H), 6.14 (s, 1H), 5.73 (s, 1H), 4.80 - 4.70 (m, 2H), 4.38 (ddd, J = 12.0, 7.4, 4.2 Hz, 1H), 4.28 (ddd, J = 12.6, 7.7, 4.5 Hz, 1H), 4.10 (dt, J = 14.2, 4.9 Hz, 1H), 3.96 (ddd, J = 14.0, 7.5, 4.1 Hz, 1H), 3.37 (s, 3H).MS (ESI): m/z 458.1[M + H]$^+$.

### Example E2: 4-chloro-5-(3-(ethoxy(4-fluoro-2-(trifluoromethyl)phenyl)methyl)-5,6-dihydroimidazo[1,2-a]pyrazin-7(8H)-yl)pyridazin-3(2H)-one

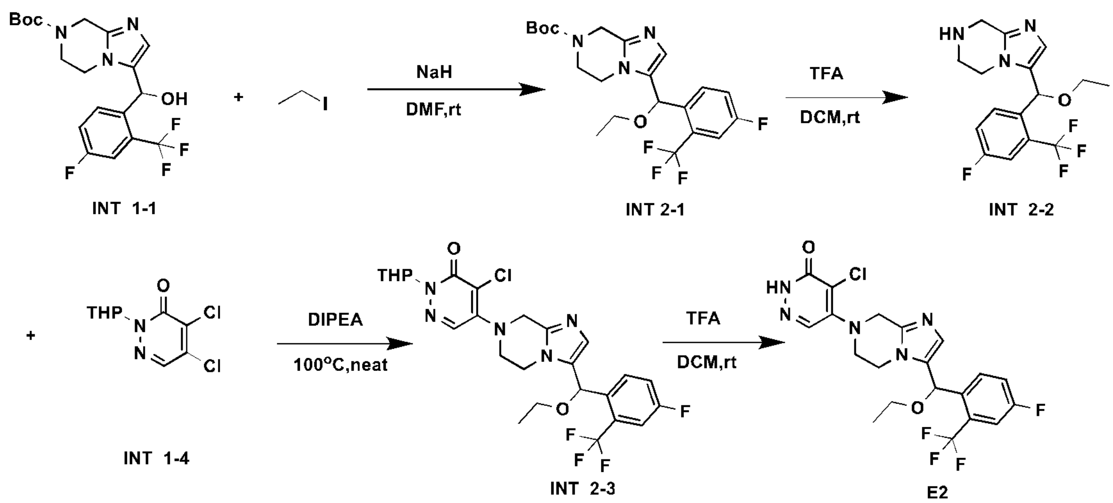


INT 1-1, INT 2-1, INT 2-2, INT 1-4, INT 2-3, E2

### 2.1 Preparation of intermediate INT 2-1

The synthesis method of intermediate INT 2-1 was the same as that of INT 1-2, except that iodoethane was used as the starting material instead of iodomethane.

### 2.2 Preparation of intermediate INT 2-2

The synthesis method of intermediate INT 2-2 was the same as that of INT 1-3, except that INT 2-1 was used as the starting material instead of INT 1-2.

### 2.3 Preparation of intermediate INT 2-3

The synthesis method of intermediate INT 2-3 was the same as that of INT 1-5, except that INT 2-2 was used as the starting material instead of INT 1-3.

### 2.4 Preparation of Compound E2

The synthesis method of Example E2 was the same as that of E1, except that the intermediate INT 2-3 was used as the starting material instead of INT 1-5. 1H NMR (500 MHz, Methanol-d4) δ 8.04 (s, 1H), 7.96 (dd, J = 8.7, 5.3 Hz, 1H), 7.69 - 7.60 (m, 2H), 6.85 (s, 1H), 5.97 (s, 1H), 5.13 - 5.00 (m, 2H), 4.63 - 4.52 (m, 2H), 4.21 (dt, J = 14.5, 4.7 Hz, 1H), 4.05 (ddd, J = 14.5, 7.3, 4.3 Hz, 1H), 3.73 - 3.66 (m, 1H), 3.55 - 3.47 (m, 1H), 1.27 (t, J = 7.4 Hz, 3H). MS (ESI): m/z 472.1[M + H]$^+$.

### Example E3: 4-chloro-5-(3-((cyclopropylmethoxy)(4-fluoro-2-(trifluoromethyl)phenyl)methyl)-5,6-dihydroimidazo[1,2-a]pyrazin-7(8H)-yl)pyridazin-3(2H)-one

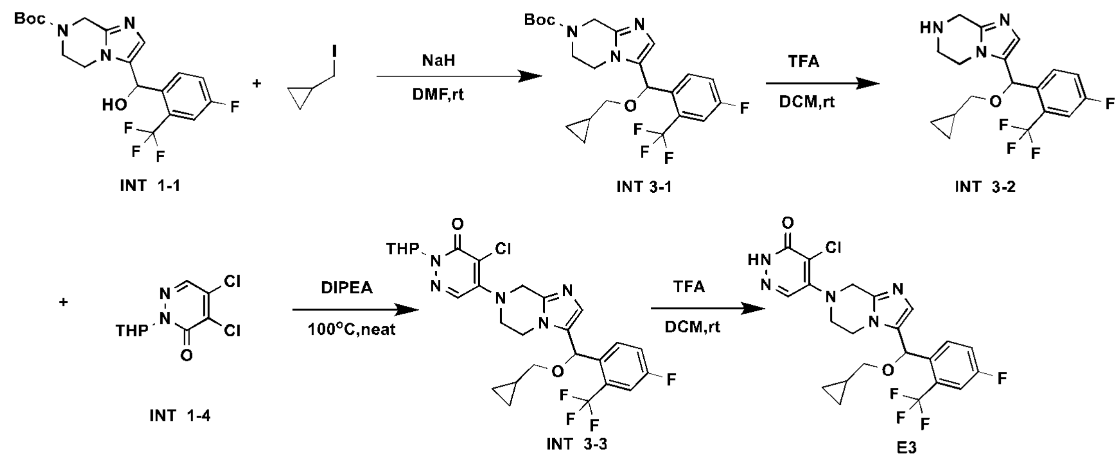


### 3.1 Preparation of intermediate INT 3-1

The synthesis method of intermediate INT 3-1 was the same as that of INT 1-2, except that (iodomethyl) cyclopropane was used as the starting material instead of iodomethane.

### 3.2 Preparation of intermediate INT 3-2

The synthesis method of intermediate INT 3-2 was the same as that of INT 1-3, except that INT 3-1 was used as the starting material instead of INT 1-2.

### 3.3 Preparation of intermediate INT 3-3

The synthesis method of intermediate INT 3-3 was the same as that of INT 1-5, except that INT 3-2 was used as the starting material instead of INT 1-3.

### 3.4 Preparation of Compound E3

The synthesis method of Example E3 was the same as that of that of E1, except that the intermediate INT 3-3 was used as the starting material instead of INT 1-5. 1H NMR (500 MHz, Methanol-d4) δ 8.04 (s, 1H), 7.98 (dd, J = 8.7, 5.3 Hz, 1H), 7.67 - 7.60 (m, 2H), 6.82 (s, 1H), 5.99 (s, 1H), 5.12 - 4.99 (m, 2H), 4.64 (ddd, J = 12.2, 7.5, 4.3 Hz, 1H), 4.57 (dt, J = 13.2, 4.6 Hz, 1H), 4.23 (dt, J = 14.6, 4.7 Hz, 1H), 4.05 (ddd, J = 14.5, 7.6, 4.1 Hz, 1H), 3.51 (dd, J = 10.1, 6.9 Hz, 1H), 3.26 (dd, J = 10.1, 7.2 Hz, 1H), 1.21 - 1.09 (m, 1H), 0.64 - 0.51 (m, 2H), 0.31 - 0.17 (m, 2H).MS (ESI): m/z 498.1[M + H]$^+$.

### Example E4: 5-(3-(butoxy(4-fluoro-2-(trifluoromethyl)phenyl)methyl)-5,6-dihydroimidazo[1,2-a]pyrazin-7(8H)-yl)-4-chloropyridazin-3(2H)-one

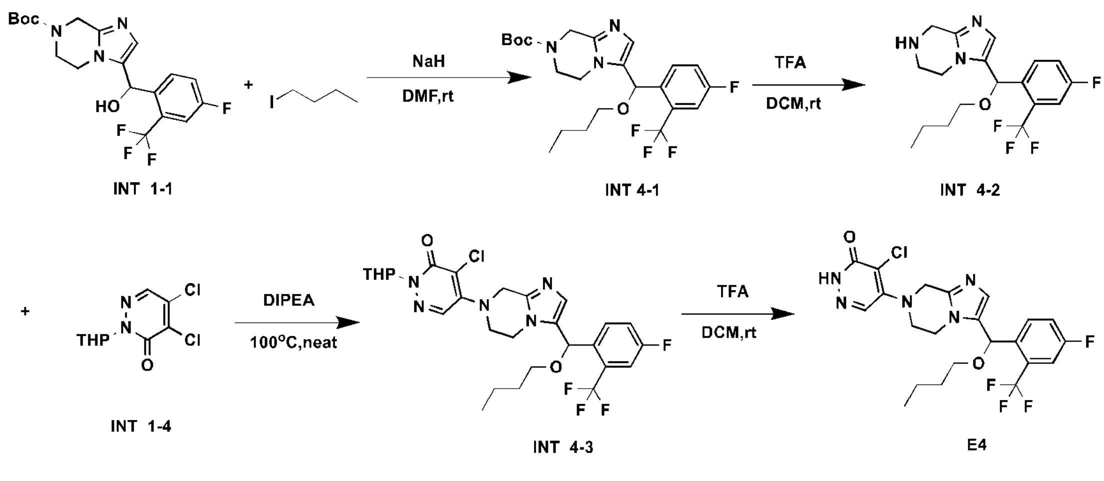

### 4.1 Preparation of intermediate INT 4-1

The synthesis method of intermediate INT 4-1 was the same as that of INT 1-2, except that iodobutane was used as the starting material instead of iodomethane.

### 4.2 Preparation of intermediate INT 4-2

The synthesis method of intermediate INT 4-2 was the same as that of INT 1-3, except that INT 4-1 was used as the starting material instead of INT 1-2.

### 4.3 Preparation of intermediate INT 4-4

The synthesis method of intermediate INT 4-3 was the same as that of INT 1-5, except that INT 4-2 was used as the starting material instead of INT 1-3.

### 4.4 Preparation of Compound E4

The synthesis method of Example E4 was the same as that of that of E1, except that the intermediate INT 4-4 was used as the starting material instead of INT 1-5. 1H NMR (500 MHz, Methanol-d4) δ 8.03 (s, 1H), 7.95 (dd, J = 8.7, 5.3 Hz, 1H), 7.69 - 7.59 (m, 2H), 6.82 (s, 1H), 5.94 (s, 1H), 5.11 - 4.97 (m, 2H), 4.61 - 4.52 (m, 2H), 4.22 (dt, J = 14.6, 4.8 Hz, 1H), 4.04 (ddd, J = 14.5, 6.7, 5.0 Hz, 1H), 3.67 (dt, J = 8.9, 6.3 Hz, 1H), 3.42 (dt, J = 9.0, 6.6 Hz, 1H), 1.71 - 1.60 (m, 2H), 1.50 - 1.39 (m, 2H), 0.94 (t, J = 7.4 Hz, 3H). MS (ESI): m/z 500.1[M + H]$^+$.

### Example E5: 5-(4-((benzyloxy)(4-fluoro-2-(trifluoromethyl)phenyl)methyl)-5,6-dihydroimidazo[1,2-a]pyrazin-7(8H)-yl)-4-chloropyridazin-4(2H)-one

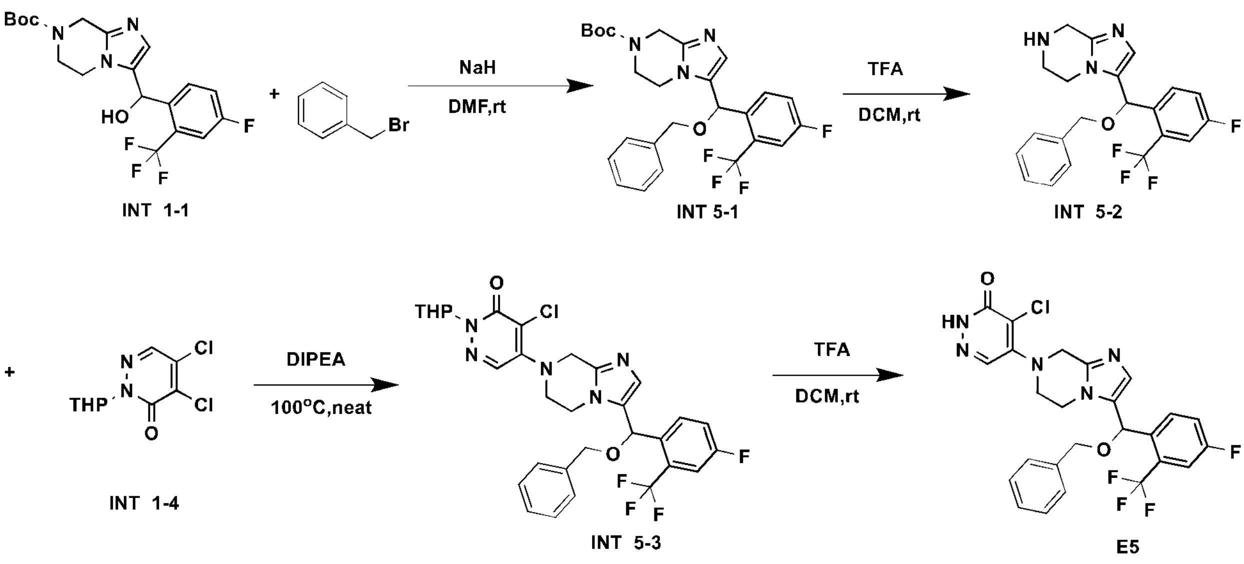


INT 1-1, INT 5-1, INT 5-2, INT 1-4, INT 5-3, E5

### 5.1 Preparation of Intermediate INT 5-1

The synthesis method of intermediate INT 5-1 was the same as that of INT 1-2, except that benzyl bromide was used as the starting material instead of iodomethane.

### 5.2 Preparation of intermediate INT 5-2

The synthesis method of intermediate INT 5-2 was the same as that of INT 1-3, except that INT 5-1 was used as the starting material instead of INT 1-2.

### 5.3 Preparation of intermediate INT 5-3

The synthesis method of intermediate INT 5-3 was the same as that of INT 1-5, except that INT 5-2 was used as the starting material instead of INT 1-3.

### 5.4 Preparation of Compound E5

The synthesis method of Example E5 was the same as that of that of E1, except that the intermediate INT 5-3 was used as the starting material instead of INT 1-5. 1H NMR (500 MHz, Methanol-d4) δ 8.05 (dd, J = 8.7, 5.4 Hz, 1H), 7.99 (s, 1H), 7.71 - 7.60 (m, 2H), 7.43 - 7.35 (m, 5H), 6.69 (s, 1H), 6.07 (s, 1H), 4.98 (s, 2H), 4.62 (d, J = 11.0 Hz, 1H), 4.54 (d, J = 11.0 Hz, 1H), 4.42 - 4.31 (m, 2H), 4.13 (dt, J = 14.4, 4.7 Hz, 1H), 3.95 (ddd, J = 14.3, 7.4, 4.3 Hz, 1H). MS (ESI): m/z 534.1[M + H]$^+$.

### Example E6: 4-chloro-5-(3-((4-fluoro-2-(trifluoromethyl)phenyl)((4-fluorobenzyl)oxy)methyl)-5,6-dihydroimidazo[1,2-a]pyrazin-7(8H)-yl)pyridazin-3(2H)-one

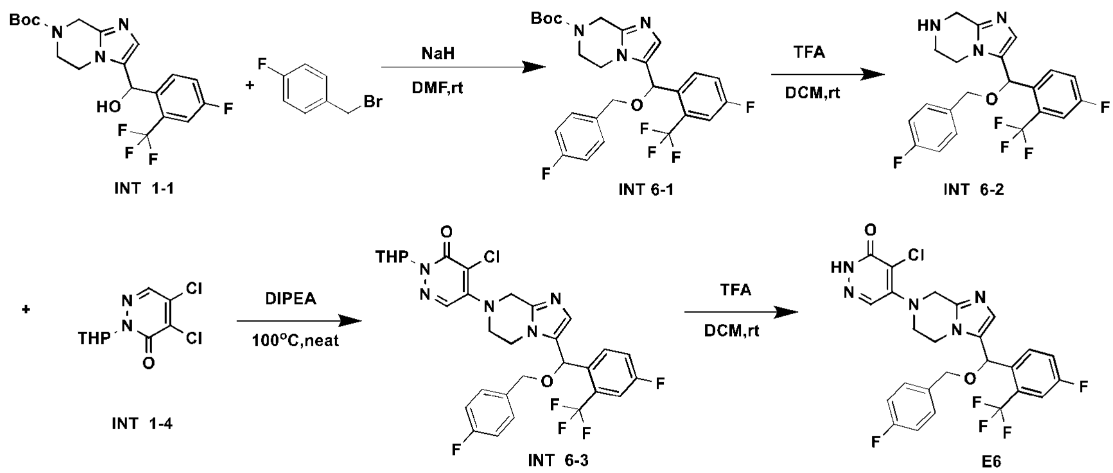


### 6.1 Preparation of Intermediate INT 6-1

The synthesis method of intermediate INT 6-1 was the same as that of INT 1-2, except that 4-fluorobenzyl bromide was used as the starting material instead of iodomethane.

### 6.2 Preparation of intermediate INT 6-2

The synthesis method of intermediate INT 6-2 was the same as that of INT 1-3, except that INT 6-1 was used as the starting material instead of INT 1-2.

### 6.3 Preparation of Intermediate INT 6-4

The synthesis method of intermediate INT 6-3 was the same as that of INT 1-5, except that INT 6-2 was used as the starting material instead of INT 1-3.

### 6.4 Preparation of Compound E6

The synthesis method of Example E6 was the same as that of that of E1, except that the intermediate INT 6-3 was used as the starting material instead of INT 1-5. $^1$H NMR (500 MHz, Chloroform-*d*) δ 7.92 (d, *J* = 5.5 Hz, 1H), 7.90 (s, 1H), 7.52 -7.43(m, 2H), 7.11-7.02 (m, 4H), 6.60 (s, 1H), 5.90 (s, 1H), 5.19 - 5.01 (m, 2H), 4.53 (t, *J* = 10.1 Hz, 1H), 4.43 (d, *J* = 11.4 Hz, 1H), 4.38 (d, *J* = 11.4 Hz, 1H), 4.17 (d, *J* = 11.5 Hz, 2H), 4.00 (d, *J* = 45.3 Hz, 1H). MS (ESI): m/z 552.1[M + H]$^+$.

**Example E7: 4-chloro-5-(3-((4-fluoro-2-(trifluoromethyl)phenyl)(hydroxy)methyl)-5,6-dihydroimidazo[1,2-a]pyrazin-7(8H)-yl)pyridazin-3(2H)-one**

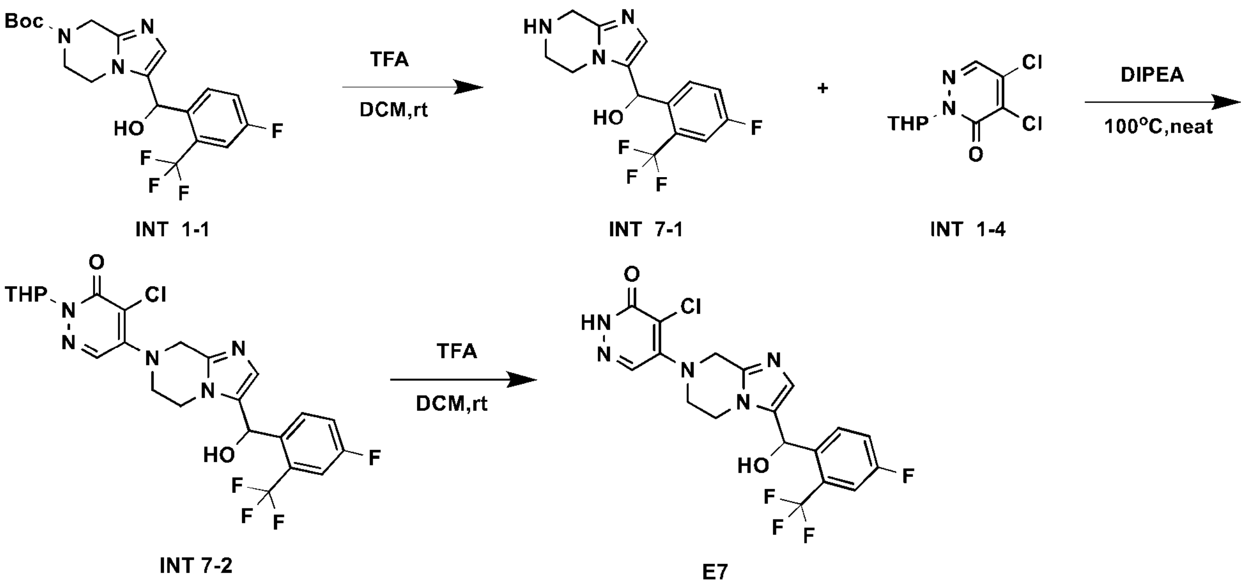


**7.1 Preparation of Intermediate INT 7-1**

The synthesis method of intermediate INT 7-1 was the same as that of INT 1-3, except that INT 1-1 was used as the starting material instead of INT 1-2.

**7.2 Preparation of Intermediate INT 7-2**

The synthesis method of intermediate INT 7-2 was the same as that of INT 1-5, except that INT 7-1 was used as the starting material instead of INT 1-3.

**7.3 Preparation of Compound E7**

The synthesis method of Example E7 was the same as that of that of E1, except that the intermediate INT 7-2 was used as the starting material instead of INT 1-5. 1H NMR (500 MHz, DMSO-d6) δ 13.12 (s, 1H), 8.02 (s, 1H), 7.93 (dd, J = 9.7, 5.4 Hz, 1H), 7.74 - 7.68 (m, 2H), 6.68 (s, 1H), 6.07 (s, 1H), 4.99 - 4.86 (m, 2H), 4.42 - 4.27 (m, 2H), 4.01 (dt, J = 14.5, 5.0 Hz, 1H), 3.93 (ddd, J = 14.4, 7.0, 4.4 Hz, 1H).MS (ESI): m/z 443.08[M + H]$^+$.

**Example E8: 4-chloro-5-(3-((2-fluoro-6-(trifluoromethyl)phenyl)(methoxy)methyl)-5,6-dihydroimidazo[1,2-a]pyrazin-7(8H)-yl)pyridazin-3(2H)-one**

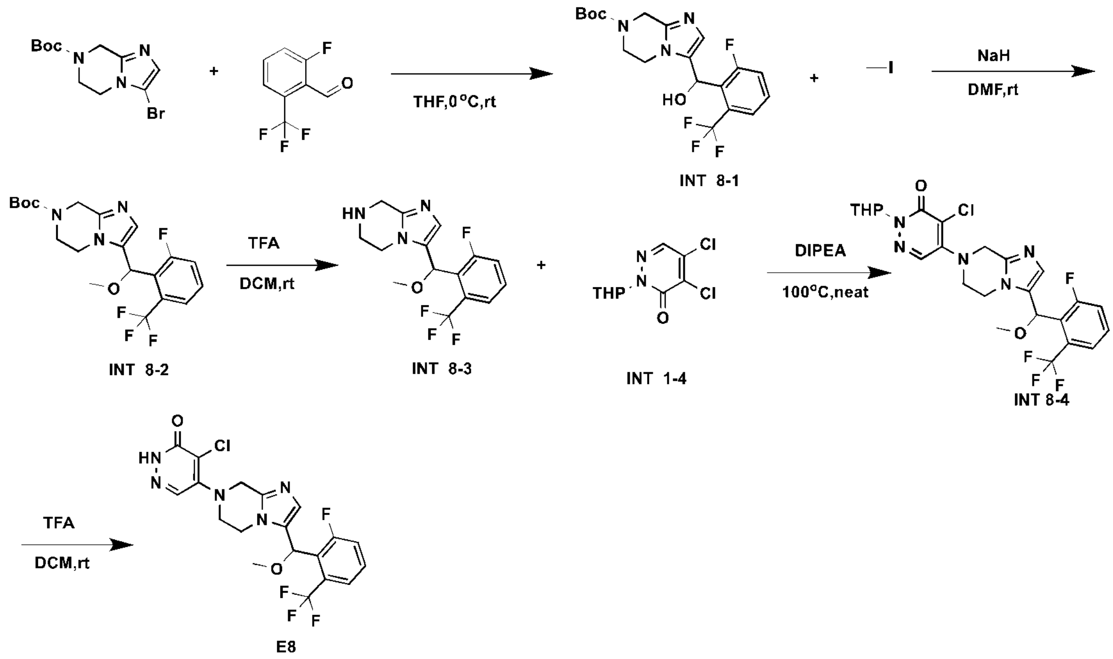


INT 8-1 INT 8-2 INT 8-3 INT 1-4 INT 8-4 E8

### 8.1 Preparation of Intermediate INT 8-1

The synthesis method of intermediate INT 8-1 was the same as that of INT 1-1, except that 2-fluoro-6-(trifluoromethyl)benzaldehyde was used as the starting material instead of 4-fluoro-2-(trifluoromethyl)benzaldehyde.

### 8.2 Preparation of Intermediate INT 8-2

The synthesis method of intermediate INT 8-2 was the same as that of INT 1-2, except that INT 8-1 was used as the starting material instead of INT 1-1.

### 8.3 Preparation of Intermediate INT 8-3

The synthesis method of intermediate INT 8-3 was the same as that of INT 1-3, except that INT 8-2 was used as the starting material instead of INT 1-2.

### 8.4 Preparation of Intermediate INT 8-4

The synthesis method of intermediate INT 8-4 was the same as that of INT 1-5, except that INT 8-3 was used as the starting material instead of INT 1-3.

### 8.5 Preparation of Compound E8

The synthesis method of Example E8 was the same as that of that of E1, except that the intermediate INT 8-4 was used as the starting material instead of INT 1-5. 1H NMR (500 MHz, DMSO-d6) δ 13.12 (s, 1H), 8.05 (s, 1H), 7.83 - 7.78 (m, 2H), 7.78 - 7.71 (m, 1H), 7.15 (s, 1H), 5.90 (s, 1H), 5.05 - 4.93 (m, 2H), 4.56 (dt, J = 13.3, 4.5 Hz, 1H), 4.37 (ddd, J = 12.8, 7.9, 4.2 Hz, 1H), 4.11 (dt, J = 14.7, 4.7 Hz, 1H), 3.91 (ddd, J = 14.5, 7.9, 4.0 Hz, 1H), 3.35 (s, 3H). MS (ESI): m/z 458.1[M + H]$^+$.

### Example E9: 5-(3-(azido(4-fluoro-2-(trifluoromethyl)phenyl)methyl)-5,6-dihydroimidazo[1,2-a]pyrazin-7(8H)-yl)-4-chloropyridazin-3(2H)-one

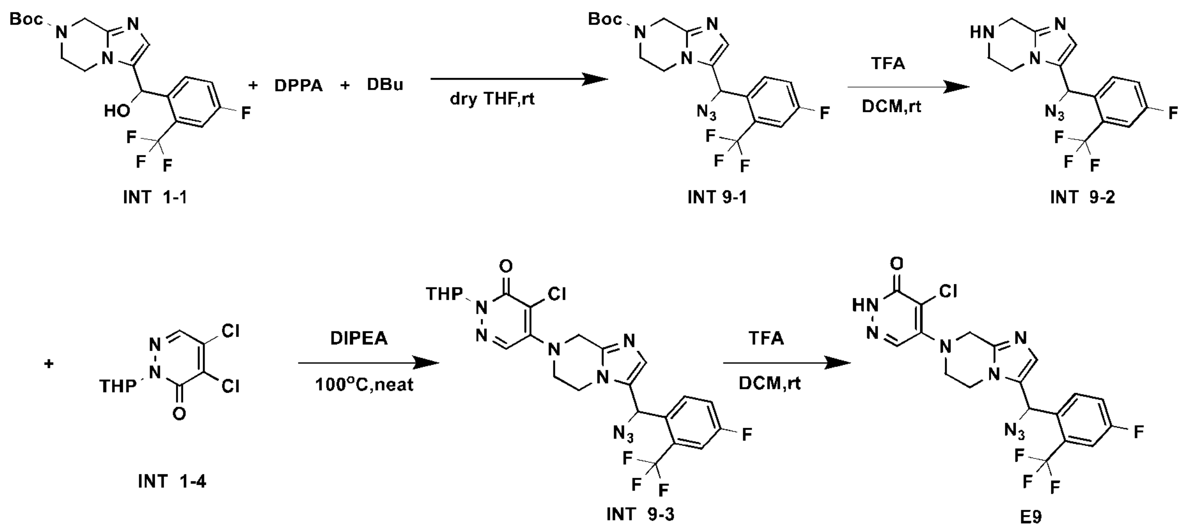


INT 1-1 INT 9-1 INT 9-2 INT 1-4 INT 9-3 E9

**9.1 Preparation of Intermediate INT 9-1**

The intermediate INT 1-1 (100 mg, 0.24 mmol) was added to a three-necked flask and dissolved in anhydrous THF. After nitrogen protection, DPPA (86 mg, 0.31 mmol) and DBu (55 mg, 0.36 mmol) were added. The reaction mixture was stirred at room temperature for 1 h and monitored by TLC. After the reaction was completed, the solvent was removed and the mixture was separated by flash silica gel chromatography using MeOH/DCM as the mobile phase to collect the target product, i.e., the intermediate INT 9-1, which was about 62 mg.

**9.2 Preparation of Intermediate INT 9-2**

The synthesis method of intermediate INT 9-2 was the same as that of INT 1-3, except that INT 9-1 was used as the starting material instead of INT 1-2.

**9.3 Preparation of Intermediate INT 9-3**

The synthesis method of intermediate INT 9-3 was the same as that of INT 1-5, except that INT 9-2 was used as the starting material instead of INT 1-3.

**9.4 Preparation of Compound E9**

The synthesis method of Example E9 was the same as that of that of E1, except that the intermediate INT 9-3 was used as the starting material instead of INT 1-5. 1H NMR (500 MHz, Methanol-d4) δ 8.00 (s, 1H), 7.80 (dd, J = 8.8, 5.2 Hz, 1H), 7.75 (dd, J = 8.9, 2.7 Hz, 1H), 7.62 (td, J = 8.3, 2.7 Hz, 1H), 7.41 (s, 1H), 6.49 (s, 1H), 5.12 - 5.01 (m, 2H), 4.47 - 4.41 (m, 1H), 4.15 - 4.05 (m, 2H), 4.04 - 3.96 (m, 1H).MS (ESI): m/z 469.1[M + H]$^+$.

**Example E10: 4-chloro-5-(3-(1-(4-fluoro-2-(trifluoromethyl)phenyl)vinyl)-5,6-dihydroimidazo[1,2-a]pyrazin-7(8H)-yl)pyridazin-3(2H)-one**

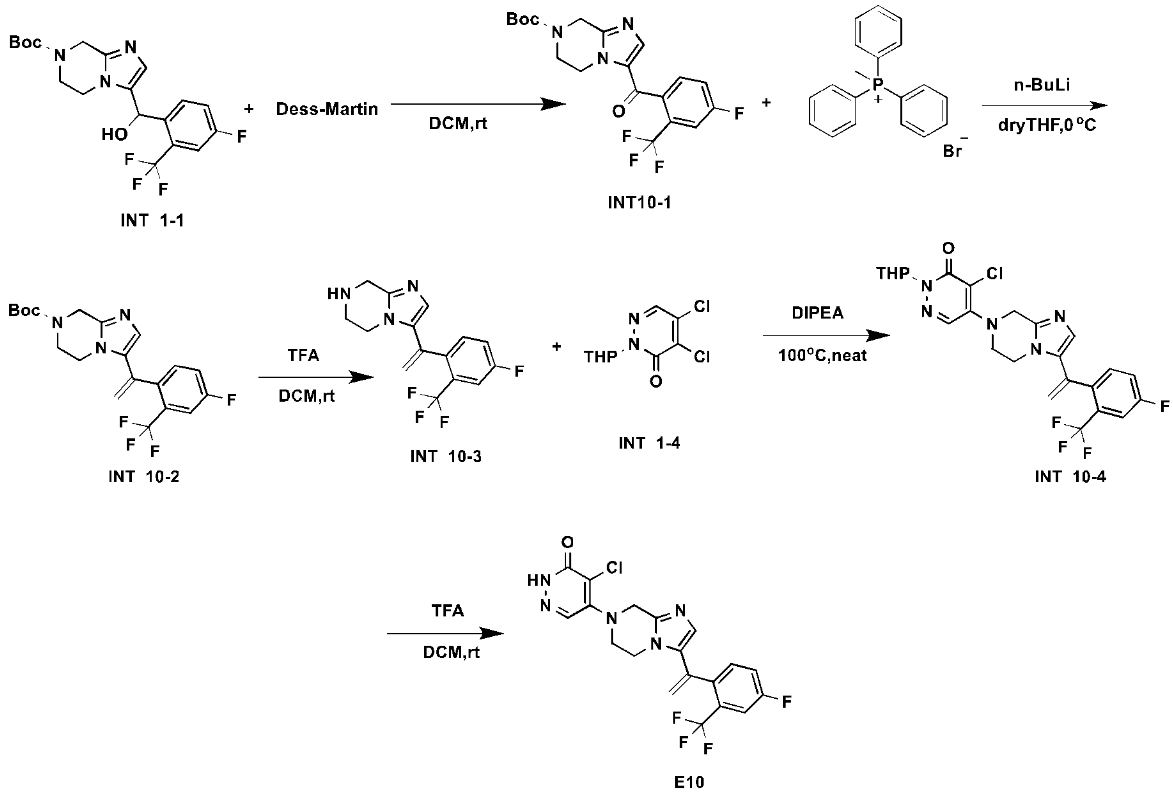


INT 1-1, INT10-1, INT 10-2, INT 10-3, INT 1-4, INT 10-4, E10

**10.1 Preparation of Intermediate INT 10-1**

The intermediate INT 1-1 (300 mg, 0.72 mmol) was added to a single-necked bottle and dissolved in DCM. Dess-Martin (612 mg, 1.44 mmol) was added and stirred at room temperature for 30 min. The reaction was monitored by TLC. The reaction mixture was quenched with a saturated sodium thiosulfate solution, extracted with EA, washed with saturated brine, dried over anhydrous magnesium sulfate, filtered, and the filtrate was spin-dried. The product was separated by flash silica gel chromatography using MeOH/DCM as the mobile phase to collect the target product, i.e., the intermediate INT 10-1, which was approximately 130 mg.

**10.2 Preparation of Intermediate INT 10-2**

Methyltriphenylphosphonium bromide (337 mg, 0.93 mmol) was added to a three-necked flask and dissolved in anhydrous THF. Under nitrogen protection, n-BuLi (2.5 M/L in THF, 150 μl, 0.37 mmol) was slowly added dropwise at 0 °C. The reaction mixture was stirred at 0 °C for 30 min. The intermediate INT 10-1 (130 mg, 0.31 mmol) was dissolved in 1 ml of anhydrous THF and slowly added dropwise to the reaction flask. The reaction mixture was stirred at 0 °C for 3 h. After completion of the reaction monitored by TLC, a solution of saturated ammonium chloride was added to quench the reaction. The reaction mixture was extracted with EA, and the organic phase was washed three times with saturated brine, dried over anhydrous magnesium sulfate, filtered, and the filtrate was concentrated and separated by flash silica gel chromatography with MeOH/DCM as the mobile phase to collect the target product, i.e., the intermediate INT 10-2, about 98 mg.

**10.3 Preparation of Intermediate INT 10-3**

The synthesis method of intermediate INT 10-3 was the same as that of INT 1-3, except that INT 10-2 was used as the starting material instead of INT 1-2.

**10.4 Preparation of Intermediate INT 10-4**

The synthesis method of intermediate INT 10-4 was the same as that of INT 1-5, except that INT 10-3 was used as the starting material instead of INT 1-3.

### 10.5 Preparation of Compound E10

The synthesis method of Example E10 was the same as that of that of E1, except that the intermediate INT 10-4 was used as the starting material instead of INT 1-5. 1H NMR (500 MHz, Methanol-d4) δ 8.02 (s, 1H), 7.66 - 7.58 (m, 2H), 7.52 (td, J = 8.3, 2.7 Hz, 1H), 7.23 (s, 1H), 6.04 (s, 1H), 5.68 (s, 1H), 5.06 (s, 2H), 4.31 (t, J = 5.2 Hz, 2H), 4.03 (t, J = 5.2 Hz, 2H). MS (ESI): m/z 440.1[M + H]+.

### Example E11: 4-chloro-5-(3-(1-(4-fluoro-2-(trifluoromethyl)phenyl)ethyl)-5,6-dihydroimidazo[1,2-a]pyrazin-7(8H)-yl)pyridazin-3(2H)-one

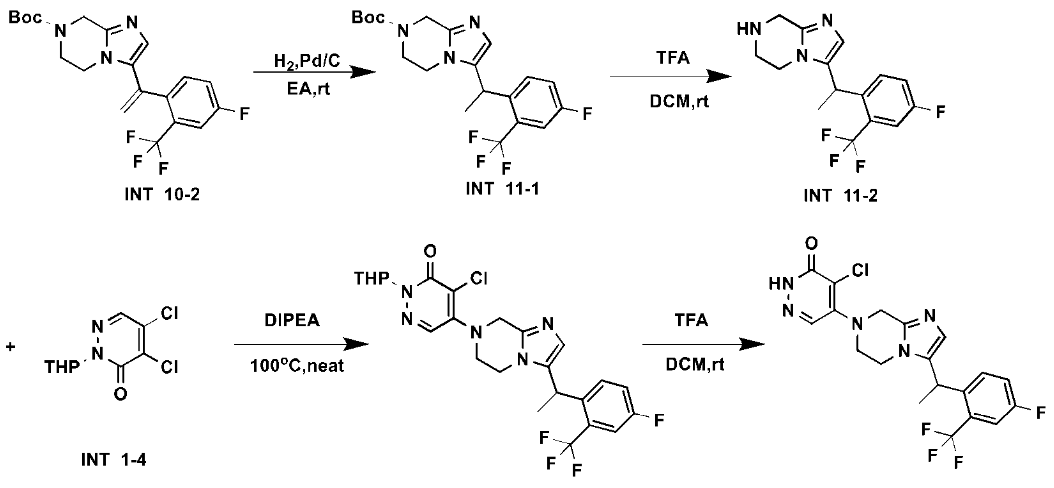


INT 10-2 INT 11-1 INT 11-2 INT 1-4 INT 11-3 E11

### 11.1 Preparation of Intermediate INT 11-1

The intermediate INT 10-2 (100 mg, 0.24 mmol) was added to a single-necked bottle and dissolved with EA. Palladium/carbon catalyst (10 mg) was added and $H_2$ was replaced three times. The reaction mixture was reacted at room temperature and monitored by LC-MS. After the reaction was completed, the mixture was filtered and the filtrate was dried by rotary evaporator to give about 68 mg of the intermediate INT 11-1.

### 11.2 Preparation of Intermediate INT 11-2

The synthesis method of intermediate INT 11-2 was the same as that of INT 1-3, except that INT 11-1 was used as the starting material instead of INT 1-2.

### 11.3 Preparation of Intermediate INT 11-3

The synthesis method of intermediate INT 11-3 was the same as that of INT 1-5, except that INT 11-2 was used as the starting material instead of INT 1-3.

### 11.4 Preparation of Compound E11

The synthesis method of Example E11 was the same as that of that of E1, except that the intermediate INT 11-3 was used as the starting material instead of INT 1-5. $^1$H NMR (500 MHz, DMSO-$d_6$) δ 13.08 (s, 1H), 7.93 (s, 1H), 7.76 - 7.69 (m, 1H), 7.53 (td, *J* = 8.5, 2.8 Hz, 1H), 7.29 (dd, *J* = 8.8, 5.3 Hz, 1H), 7.24 (s, 1H), 5.01 - 4.86 (m, 2H), 4.51 (q, *J* = 6.9 Hz, 1H), 4.11 (dt, *J* = 12.5, 4.8 Hz, 1H), 3.87 - 3.72 (m, 2H), 3.31 (ddd, *J* = 11.9, 7.0, 4.4 Hz, 1H), 1.57 (d, *J* = 6.9 Hz, 3H). MS (ESI): m/z 442.1[M + H]+.

### Example E12: 4-chloro-5-(3-((4-fluoro-2-(trifluoromethyl)phenyl)(methoxy-d3)methyl)-5,6-dihydroimidazo[1,2-a]pyrazin-7(8H)-yl)pyridazin-3(2H)-one

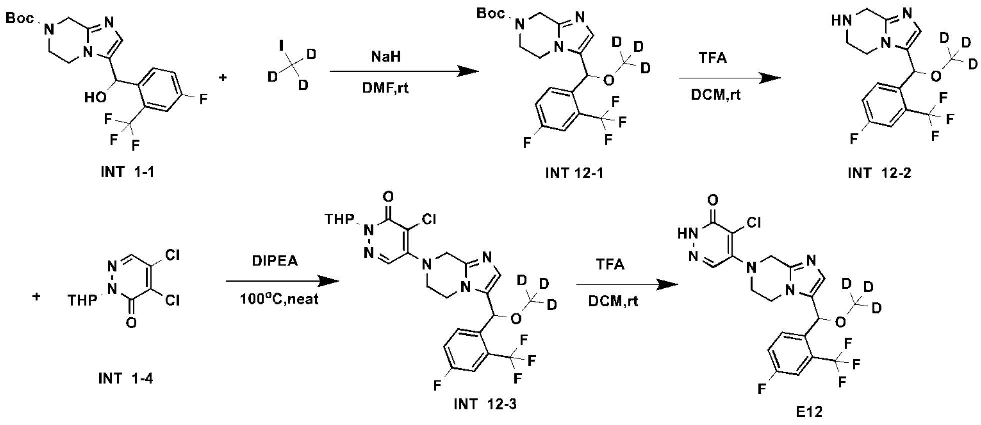


INT 1-1 INT 12-1 INT 12-2 INT 1-4 INT 12-3 E12

## 12.1 Preparation of Intermediate INT 12-1

The synthesis method of intermediate INT 12-1 was the same as that of INT 1-2, except that deuterated iodomethane was used as the starting material instead of iodomethane.

## 12.2 Preparation of Intermediate INT 12-2

The synthesis method of intermediate INT 12-2 was the same as that of INT 1-3, except that INT 12-1 was used as the starting material instead of INT 1-2.

## 12.3 Preparation of Intermediate INT12-3

The synthesis method of intermediate INT 12-3 was the same as that of INT 1-5, except that INT 12-2 was used as the starting material instead of INT 1-3.

## 12.4 Preparation of Compound E12

The synthesis method of Example E12 was the same as that of that of E1, except that the intermediate INT 12-3 was used as the starting material instead of INT 1-5. $^1$H NMR (500 MHz, DMSO-$d_6$) δ 13.11 (s, 1H), 8.02 (s, 1H), 7.83 - 7.76 (m, 2H), 7.73 (td, *J* = 8.5, 2.7 Hz, 1H), 6.77 (s, 1H), 5.76 (s, 1H), 4.96 - 4.85 (m, 2H), 4.46 (dt, *J* = 13.4, 4.5 Hz, 1H), 4.31 (ddd, *J*= 12.7, 7.4, 4.1 Hz, 1H), 4.05 (dt, *J* = 14.7, 4.7 Hz, 1H), 3.91 (ddd, *J*= 14.3, 7.7, 4.1 Hz, 1H). MS (ESI): m/z 461.1[M + H]$^+$.

**Example E13: 5-(2-bromo-3-((4-fluoro-2-(trifluoromethyl)phenyl)(methoxy)methyl)-5,6-dihydroimidazo[1,2-a] pyrazin-7(8H)-yl)-4-chloropyridazin-3(2H)-one**

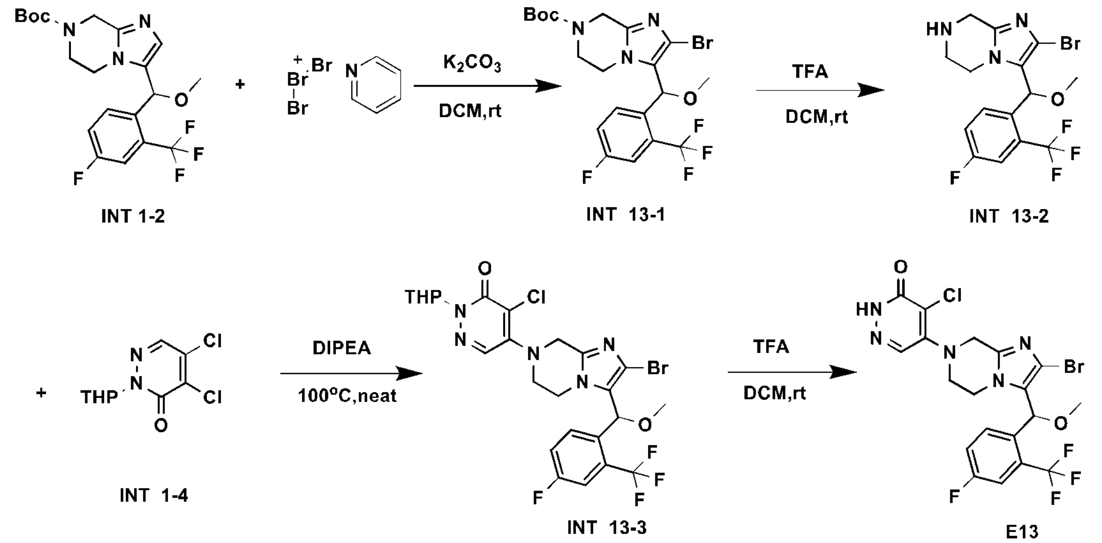


INT 1-2
INT 13-1
INT 13-2
INT 1-4
INT 13-3
E13

### 13.1 Preparation of Intermediate INT 13-1

The intermediate INT 2-1 (220 mg, 0.51 mmol) was added to a single-necked flask and dissolved in DCM. Pyridinium tribromide (328 mg, 1.02 mmol) and $K_2CO_3$ (142 mg, 1.02 mmol) were added and stirred at room temperature for 30 min. After completion of the reaction monitored by TLC, the reaction was quenched with saturated sodium bicarbonate aqueous solution and extracted with EA. The organic phase was washed three times with saturated brine, dried over anhydrous magnesium sulfate, and filtered. The filtrate was concentrated and separated by flash silica gel chromatography using MeOH/DCM as the mobile phase to collect the target product, i.e., the intermediate INT 13-1, approximately 140 mg.

### 13.2 Preparation of Intermediate INT 13-2

The synthesis method of intermediate INT 13-2 was the same as that of INT 1-3, except that INT 13-1 was used as the starting material instead of INT 1-2.

### 13.3 Preparation of Intermediate INT1 2-3

The synthesis method of intermediate INT 13-3 was the same as that of INT 1-5, except that INT 13-2 was used as the starting material instead of INT 1-3.

### 13.4 Preparation of Compound E13

The synthesis method of Example E13 was the same as that of that of E1, except that the intermediate INT 13-3 was used as the starting material instead of INT 1-5. 1H NMR (500 MHz, DMSO-d6) δ 13.07 (s, 1H), 7.95 (s, 1H), 7.75 - 7.69 (m, 2H), 7.62 (td, J = 8.4, 2.7 Hz, 1H), 5.71 (s, 1H), 4.67 (s, 2H), 4.08 (dt, J = 13.5, 5.6 Hz, 1H), 3.98 - 3.83 (m, 2H), 3.78 (ddd, J = 13.9, 7.5, 4.0 Hz, 1H), 3.32 (s, 3H). MS (ESI): m/z 536.0[M + H]$^+$.

### Example E14: 4-chloro-5-(3-methoxy(2-(trifluoromethyl)phenyl)methyl)-5,6-dihydroimidazo[1,2-a]pyrazin-7(8H)-yl)pyridazin-3(2H)-one

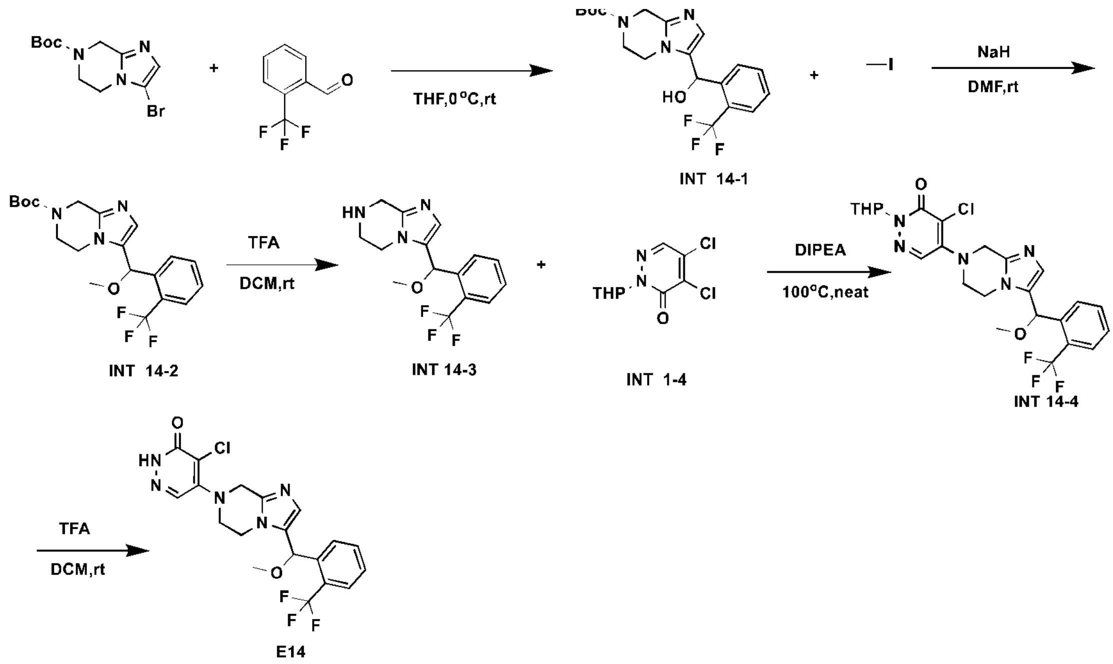


INT 14-1 INT 14-2 INT 14-3 INT 1-4 INT 14-4 E14

**14.1 Preparation of Intermediate INT 14-1**

The synthesis method of intermediate INT14-1 was the same as that of INT 1-1, except that o-trifluoromethylbenzaldehyde was used as the starting material instead of 4-fluoro-2-(trifluoromethyl)benzaldehyde.

**14.2 Preparation of Intermediate INT 14-2**

The synthesis method of intermediate INT 14-2 was the same as that of INT 1-2, except that INT 14-1 was used as the starting material instead of INT 1-1.

**14.3 Preparation of Intermediate INT14-3**

The synthesis method of intermediate INT 14-3 was the same as that of INT 1-3, except that INT 14-2 was used as the starting material instead of INT 1-2.

**14.4 Preparation of Intermediate INT 14-4**

The synthesis method of intermediate INT 14-4 was the same as that of INT 1-5, except that INT 14-3 was used as the starting material instead of INT 1-3.

**14.5 Preparation of Compound E14**

The synthesis method of Example E14 was the same as that of that of E1, except that the intermediate INT 14-4 was used as the starting material instead of INT 1-5. $^1$H NMR (500 MHz, DMSO-$d_6$) δ 13.11 (s, 1H), 8.02 (s, 1H), 7.85 (d, *J* = 7.5 Hz, 2H), 7.77 (d, *J* = 7.9 Hz, 1H), 7.70 (t, *J* = 7.7 Hz, 1H), 6.72 (s, 1H), 5.77 (s, 1H), 4.97 - 4.86 (m, 2H), 4.44 (dt, *J* = 12.9, 4.6 Hz, 1H), 4.31 (ddd, *J* = 12.8, 7.1, 4.1 Hz, 1H), 4.04 (dt, *J* = 14.8, 4.7 Hz, 1H), 3.92 (ddd, *J* = 14.1, 7.2, 3.9 Hz, 1H), 3.30 (s, 3H).MS (ESI): m/z 440.1[M + H]$^+$.

**Example E15: 4-chloro-5-(3-((5-fluoro-2-(trifluoromethyl)phenyl)(methoxy)methyl)-5,6-dihydroimidazo[1,2-a]pyrazin-7(8H)-yl)pyridazin-3(2H)-one**

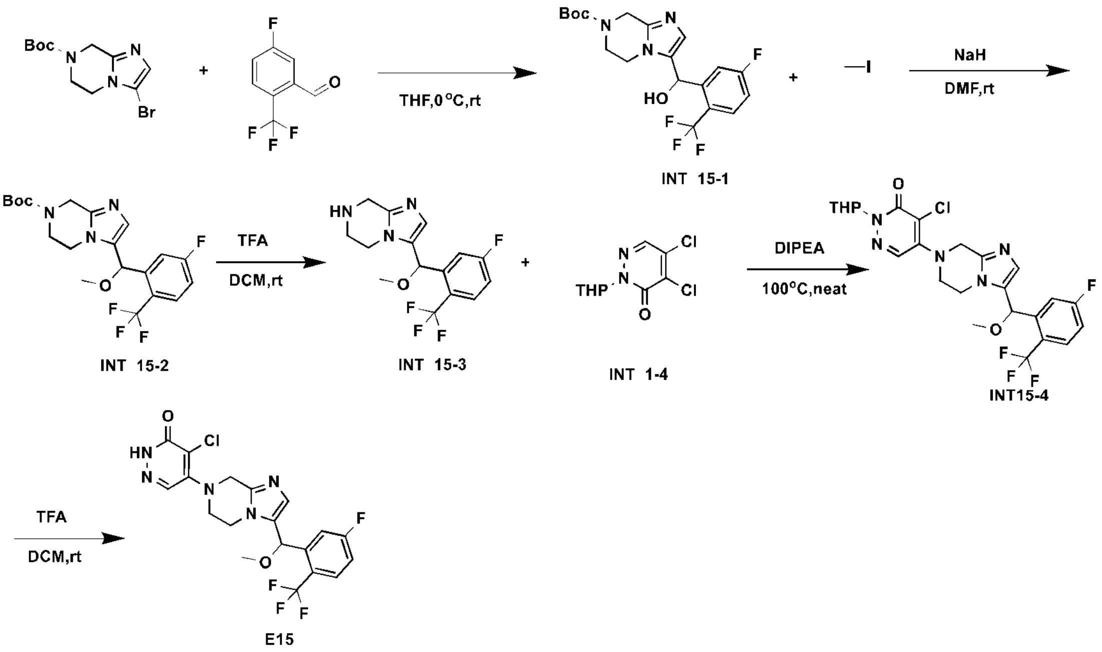


**15.1 Preparation of Intermediate INT 15-1**

The synthesis method of intermediate INT15-1 was the same as that of INT 1-1, except that 5-fluoro-2-trifluoromethylbenzaldehyde was used as the starting material instead of 4-fluoro-2-(trifluoromethyl)benzaldehyde.

**15.2 Preparation of Intermediate INT 15-2**

The synthesis method of intermediate INT 15-2 was the same as that of INT 1-2, except that INT 15-1 was used as the starting material instead of INT 1-1.

**15.3 Preparation of Intermediate INT15-3**

The synthesis method of intermediate INT 15-3 was the same as that of INT 1-3, except that INT 15-2 was used as the starting material instead of INT 1-2.

**15.4 Preparation of Intermediate INT 15-4**

The synthesis method of intermediate INT 15-4 was the same as that of INT 1-5, except that INT 15-3 was used as the starting material instead of INT 1-3.

**15.5 Preparation of Compound E15**

The synthesis method of Example E15 was the same as that of that of E1, except that the intermediate INT 15-4 was used as the starting material instead of INT 1-5. 1H NMR (500 MHz, DMSO-d6) $\delta$ 13.10 (s, 1H), 8.01 (s, 1H), 7.94 (dd, J = 8.7, 5.2 Hz, 1H), 7.57 - 7.53 (m, 1H), 7.53 - 7.50 (m, 1H), 6.82 (s, 1H), 5.77 (s, 1H), 4.95 - 4.84 (m, 2H), 4.44 (dt, J = 13.1, 4.7 Hz, 1H), 4.28 (ddd, J = 12.5, 7.4, 4.2 Hz, 1H), 4.03 (dt, J = 14.4, 4.8 Hz, 1H), 3.91 (ddd, J = 14.4, 7.5, 4.1 Hz, 1H), 3.32 (s, 3H). MS (ESI): m/z 458.1[M + H]$^+$.

**Example E16: 4-chloro-5-(3-((4-fluoro-2-(trifluoromethoxy)phenyl)(methoxymethyl)-5,6-dihydroimidazo[1,2-a] pyrazin-7(8H)-yl)pyridazin-3(2H)-one**

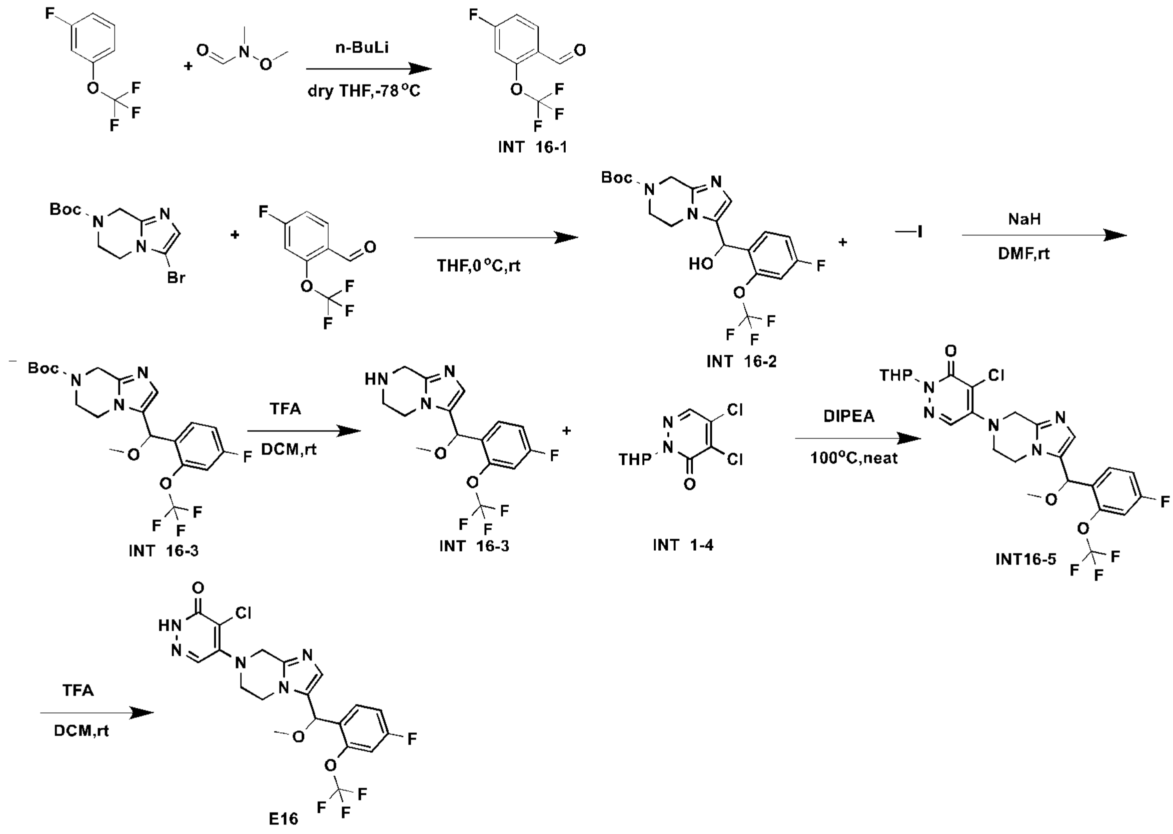


### 16.1 Preparation of Intermediate INT 16-1

m-Fluorotrifluoromethoxybenzene (500 mg, 2.78 mmol) was added to a three-necked flask and dissolved with anhydrous THF. Under nitrogen protection, the reaction mixture was placed at -78 °C. n-Butyl lithium (2.5 M/L, 1.1 ml, 3.1 mmol) was slowly added dropwise to the reaction system. The reaction mixture was stirred at -78 °C for 90 min. TLC showed that the starting material was exhausted. N-methoxy-n-methylformamide (250 mg, 2.78 mmol) was slowly added dropwise to the reaction. TLC monitored the completion of the reaction. The reaction was quenched with saturated ammonium chloride aqueous solution and extracted with EA. The organic phase was washed three times with saturated brine, dried over anhydrous magnesium sulfate, filtered, and the filtrate was concentrated and separated by flash silica gel chromatography with EA/PE as the mobile phase to collect the target product, i.e., the intermediate INT 16-1, about 250 mg.

### 16.2 Preparation of Intermediate INT 16-2

The synthesis method of intermediate INT16-2 was the same as that of INT1-1, except that INT16-1 was used as the starting material instead of 4-fluoro-2-(trifluoromethyl)benzaldehyde.

### 16.3 Preparation of Intermediate INT 16-3

The synthesis method of intermediate INT 16-3 was the same as that of INT 1-2, except that INT 16-2 was used as the starting material instead of INT 1-1.

### 16.4 Preparation of Intermediate INT16-4

The synthesis method of intermediate INT 16-4 was the same as that of INT 1-3, except that INT 16-3 was used as the starting material instead of INT 1-2.

### 16.5 Preparation of Intermediate INT 16-5

The synthesis method of intermediate INT 16-5 was the same as that of INT 1-5, except that INT 16-4 was used as the starting material instead of INT 1-3.

### 16.6 Preparation of Compound E16

The synthesis method of Example E16 was the same as that of that of E1, except that the intermediate INT 16-5 was used as the starting material instead of INT 1-5. $^1$H NMR (500 MHz, DMSO-$d_6$) δ 13.10 (s, 1H), 8.02 (s, 1H), 7.70 (td, *J* = 8.4, 6.2 Hz, 1H), 7.45 (dd, *J* = 10.0, 8.6 Hz, 1H), 7.40 (d, *J* = 8.5 Hz, 1H), 7.01 (s, 1H), 5.96 (s, 1H), 4.99 - 4.87 (m, 2H), 4.39 - 4.28 (m, 2H), 4.04 (dt, *J* = 14.3, 4.7 Hz, 1H), 3.89 (ddd, *J* = 14.4, 7.1, 4.4 Hz, 1H), 3.33 (s, 3H).MS (ESI): m/z 474.1[M + H]$^+$.

### Example E17: 4-chloro-5-(3-((4-fluoro-3-(trifluoromethyl)phenyl)(methoxy)methyl)-5,6-dihydroimidazo[1,2-a]pyrazin-7(8H)-yl)pyridazin-3(2H)-one

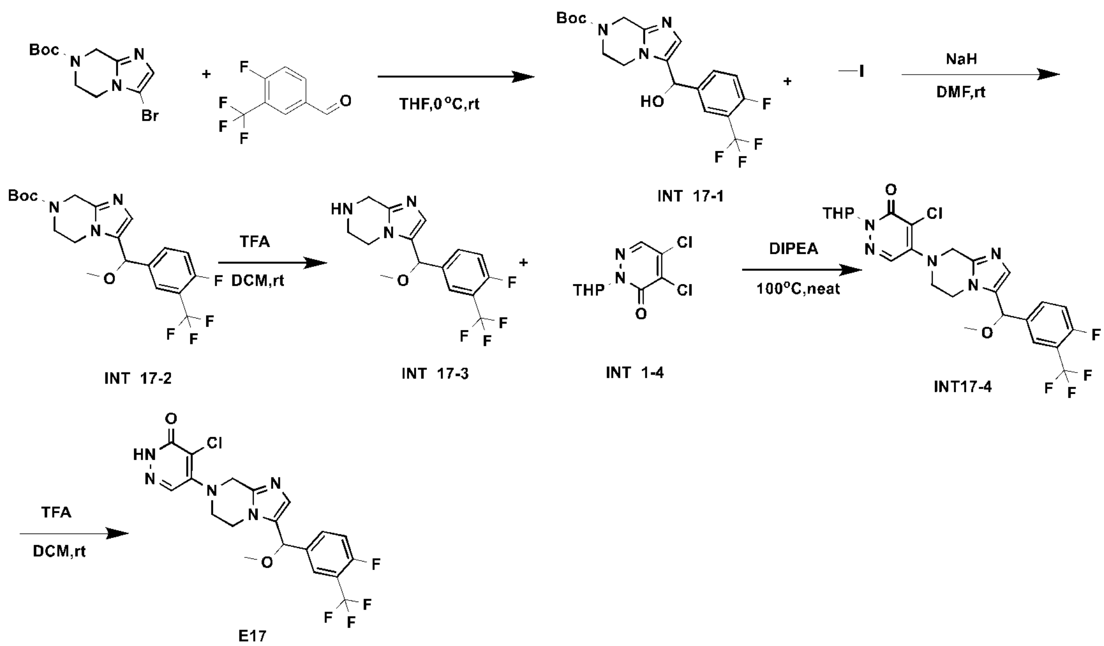


INT 17-1

INT 17-2

INT 17-3

INT 1-4

INT17-4

E17

### 17.1 Preparation of Intermediate INT 17-1

The synthesis method of intermediate INT17-1 was the same as that of INT 1-1, except that 4-fluoro-3-trifluoromethylbenzaldehyde was used as the starting material instead of 4-fluoro-2-(trifluoromethyl)benzaldehyde.

### 17.2 Preparation of Intermediate INT 17-2

The synthesis method of intermediate INT 17-2 was the same as that of INT 1-2, except that INT 17-1 was used as the starting material instead of INT 1-1.

### 17.3 Preparation of Intermediate INT17-3

The synthesis method of intermediate INT 17-3 was the same as that of INT 1-3, except that INT 17-2 was used as the starting material instead of INT 1-2.

### 17.4 Preparation of Intermediate INT 17-4

The synthesis method of intermediate INT 17-4 was the same as that of INT 1-5, except that INT 17-3 was used as the starting material instead of INT 1-3.

### 17.5 Preparation of Compound E17

The synthesis method of Example E17 was the same as that of that of E1, except that the intermediate INT 17-4 was used as the starting material instead of INT 1-5. 1H NMR (500 MHz, DMSO-d6) δ 13.10 (s, 1H), 7.99 (s, 1H), 7.82 - 7.76 (m, 2H), 7.63 (dd, J = 10.6, 8.4 Hz, 1H), 6.99 (s, 1H), 5.74 (s, 1H), 4.86 (s, 2H), 4.24 (dt, J = 13.0, 4.9 Hz, 1H), 4.11 (ddd, J = 12.4, 6.9, 4.1 Hz, 1H), 3.96 (dt, J = 14.4, 4.9 Hz, 1H), 3.87 (ddd, J = 14.4, 7.0, 4.3 Hz, 1H), 3.32 (s, 3H).MS (ESI): m/z 458.1 $[M + H]^+$.

### Example E18: 5-(3-((2-bromo-4-fluorophenyl)(methoxy)methyl)-5,6-dihydroimidazo[1,2-a]pyrazin-7(8H)-yl)-4-chloropyridazin-3(2H)-one

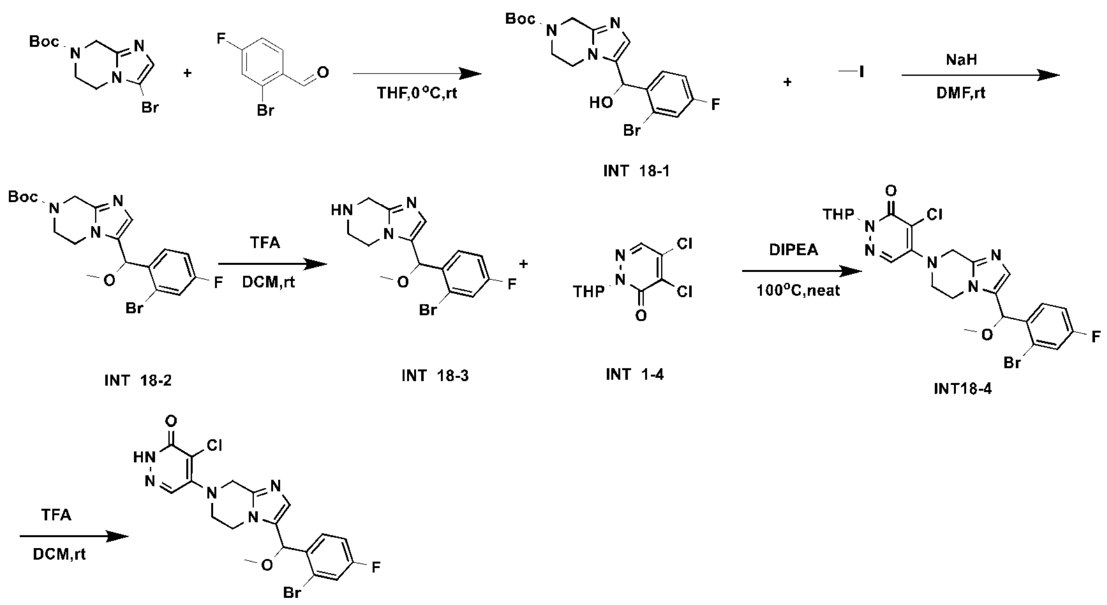


INT 18-1

INT 18-2

INT 18-3

INT 1-4

INT18-4

E18

### 18.1 Preparation of Intermediate INT 18-1

The synthesis method of intermediate INT18-1 was the same as that of INT 1-1, except that 2-bromo-4-fluorobenzaldehyde was used as the starting material instead of 4-fluoro-2-(trifluoromethyl)benzaldehyde.

### 18.2 Preparation of Intermediate INT 18-2

The synthesis method of intermediate INT 18-2 was the same as that of INT 1-2, except that INT 18-1 was used as the starting material instead of INT 1-1.

### 18.3 Preparation of Intermediate INT18-3

The synthesis method of intermediate INT 18-3 was the same as that of INT 1-3, except that INT 18-2 was used as the starting material instead of INT 1-2.

### 18.4 Preparation of Intermediate INT 18-4

The synthesis method of intermediate INT 18-4 was the same as that of INT 1-5, except that INT 18-3 was used as the starting material instead of INT 1-3.

### 18.5 Preparation of Compound E18

The synthesis method of Example E18 was the same as that of that of E1, except that the intermediate INT 18-4 was used as the starting material instead of INT 1-5. $^1H$ NMR (500 MHz, DMSO-$d_6$) δ 13.10 (s, 1H), 8.01 (s, 1H), 7.72 (dd, *J*

= 8.5, 2.5 Hz, 1H), 7.58 (dd, $J$ = 8.6, 6.2 Hz, 1H), 7.43 (td, $J$ = 8.5, 2.5 Hz, 1H), 6.94 (s, 1H), 5.75 (s, 1H), 4.91 (s, 2H), 4.43 - 4.34 (m, 1H), 4.27 (dt, $J$ = 12.7, 4.9 Hz, 1H), 4.02 (dt, $J$ = 14.6, 4.8 Hz, 1H), 3.92 (ddd, $J$ = 14.1, 7.3, 4.2 Hz, 1H), 3.36 (s, $J$ = 1.7 Hz, 3H). MS (ESI): m/z 467.0[M + H]$^+$.

**Example E19: 4-chloro-5-(3-((4-fluoro-2-methylphenyl)(methoxy)methyl)-5,6-dihydroimidazo[1,2-a]pyrazin-7(8H)-yl)pyridazin-3(2H)-one**

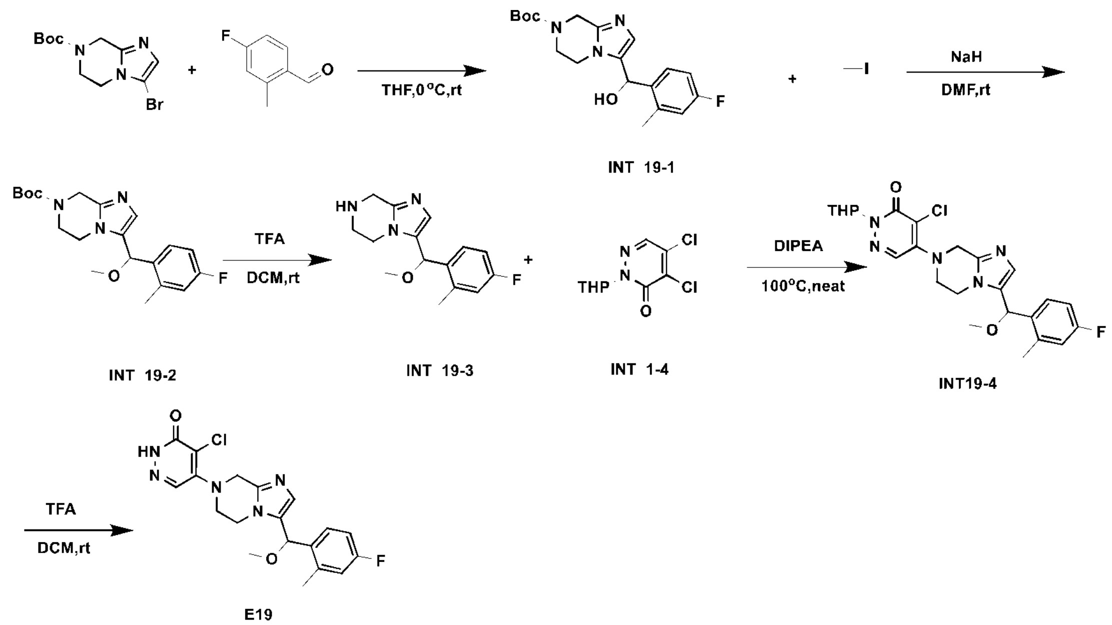


**19.1 Preparation of Intermediate INT 19-1**

The synthesis method of intermediate INT19-1 was the same as that of INT 1-1, except that 4-fluoro-2-methylbenzaldehyde was used as the starting material instead of 4-fluoro-2-(trifluoromethyl)benzaldehyde.

**19.2 Preparation of Intermediate INT 19-2**

The synthesis method of intermediate INT 19-2 was the same as that of INT 1-2, except that INT 19-1 was used as the starting material instead of INT 1-1.

**19.3 Preparation of Intermediate INT19-3**

The synthesis method of intermediate INT 19-3 was the same as that of INT 1-3, except that INT 19-2 was used as the starting material instead of INT 1-2.

**19.4 Preparation of Intermediate INT 19-4**

The synthesis method of intermediate INT 19-4 was the same as that of INT 1-5, except that INT 19-3 was used as the starting material instead of INT 1-3.

**19.5 Preparation of Compound E19**

The synthesis method of Example E19 was the same as that of that of E1, except that the intermediate INT 19-4 was used as the starting material instead of INT 1-5. $^1$H NMR (500 MHz, DMSO-$d_6$) δ 13.11 (s, 1H), 8.01 (s, 1H), 7.43 - 7.38 (m, 1H), 7.18 - 7.11 (m, 2H), 6.88 (s, 1H), 5.72 (s, 1H), 4.97 - 4.87 (m, 2H), 4.39 - 4.32 (m, 1H), 4.24 (dt, $J$ = 13.0, 5.0 Hz, 1H), 4.03 (dt, $J$ = 14.6, 4.6 Hz, 1H), 3.89 (ddd, $J$ = 14.3, 7.1, 4.1 Hz, 1H), 3.32 (s, 3H), 2.28 (s, 3H).MS (ESI): m/z 404.1[M + H]$^+$.

**Example E20: 4-chloro-5-(3-((2,4-dichlorophenyl)(methoxy)methyl)-5,6-dihydroimidazo[1,2-a]pyrazin-7(8H)-yl) pyridazin-3(2H)-one**

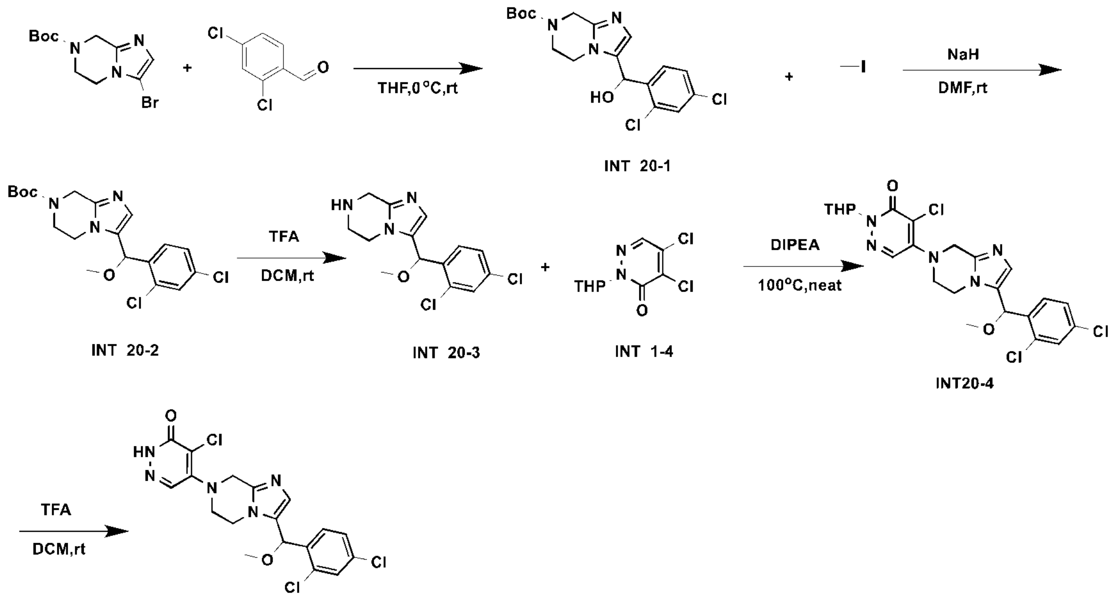

E20

**20.1 Preparation of Intermediate INT 20-1**

The synthesis method of intermediate INT20-1 was the same as that of INT 1-1, except that 2,4-dichlorobenzaldehyde was used as the starting material instead of 4-fluoro-2-(trifluoromethyl)benzaldehyde.

**20.2 Preparation of Intermediate INT 20-2**

The synthesis method of intermediate INT 20-2 was the same as that of INT 1-2, except that INT 20-1 was used as the starting material instead of INT 1-1.

**20.3 Preparation of Intermediate INT20-3**

The synthesis method of intermediate INT 20-3 was the same as that of INT 1-3, except that INT 20-2 was used as the starting material instead of INT 1-2.

**20.4 Preparation of Intermediate INT 20-4**

The synthesis method of intermediate INT 20-4 was the same as that of INT 1-5, except that INT 20-3 was used as the starting material instead of INT 1-3.

**20.5 Preparation of Compound E20**

The synthesis method of Example E20 was the same as that of that of E1, except that the intermediate INT 20-4 was used as the starting material instead of INT 1-5. 1H NMR (500 MHz, DMSO-d6) δ 13.11 (s, 1H), 8.02 (s, 1H), 7.76 (d, J = 1.9 Hz, 1H), 7.61 (d, J = 2.0 Hz, 1H), 7.59 (s, 1H), 6.99 (s, 1H), 5.84 (s, 1H), 4.97 - 4.83 (m, 2H), 4.41 (dt, J = 13.1, 4.7 Hz, 1H), 4.25 (ddd, J = 12.6, 7.4, 4.3 Hz, 1H), 4.03 (dt, J = 14.4, 4.8 Hz, 1H), 3.91 (ddd, J = 14.4, 7.4, 4.1 Hz, 1H), 3.37 (s, 3H). MS (ESI): m/z 439.04[M + H]$^+$.

**Example E21: 4-chloro-5-(3-((2-chloro-4-fluorophenyl)(methoxy)methyl)-5,6-dihydroimidazo[1,2-a]pyrazin-7(8H)-yl)pyridazin-3(2H)-one**

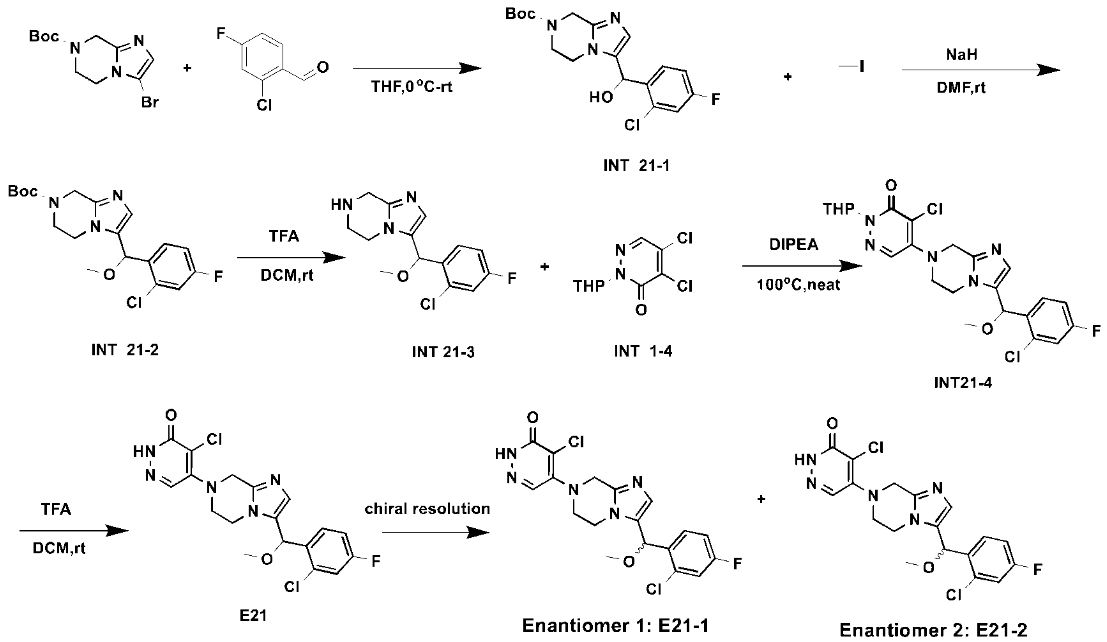


**21.1 Preparation of Intermediate INT 21-1**

The synthesis method of intermediate INT21-1 was the same as that of INT 1-1, except that 2-chloro-4-fluorobenzaldehyde was used as the starting material instead of 4-fluoro-2-(trifluoromethyl)benzaldehyde.

**21.2 Preparation of Intermediate INT 21-2**

The synthesis method of intermediate INT 21-2 was the same as that of INT 1-2, except that INT 21-1 was used as the starting material instead of INT 1-1.

**21.3 Preparation of Intermediate INT21-3**

The synthesis method of intermediate INT 21-3 was the same as that of INT 1-3, except that INT 21-2 was used as the starting material instead of INT 1-2.

**21.4 Preparation of Intermediate INT 21-4**

The synthesis method of intermediate INT 21-4 was the same as that of INT 1-5, except that INT 21-3 was used as the starting material instead of INT 1-3.

**21.5 Preparation of Compound E21**

The synthesis method of Example E21 was the same as that of that of E1, except that the intermediate INT 21-4 was used as the starting material instead of INT 1-5. $^1$H NMR (500 MHz, DMSO-$d_6$) δ 13.12 (s, 1H), 8.03 (s, 1H), 7.64 - 7.56 (m, 2H), 7.41 (td, *J* = 8.5, 2.7 Hz, 1H), 7.07 (s, 1H), 5.86 (s, 1H), 5.01 - 4.91 (m, 2H), 4.42 (dt, *J* = 13.2, 4.7 Hz, 1H), 4.28 (ddd, *J* = 12.4, 7.3, 4.2 Hz, 1H), 4.05 (dt, *J* = 14.5, 4.8 Hz, 1H), 3.94 (ddd, *J* = 14.4, 7.5, 4.1 Hz, 1H), 3.38 (s, 3H). MS (ESI): m/z 423.07[M + H]$^+$.

Compound E21 was resolved using a preparative liquid chromatography (Shimadzu, model: LC-20AT) and a CHIRALPAK AD-H chiral chromatographic column (Shanghai Daicel, column size: 0.46 cm ID × 15 cm L). Elution was performed using a mobile phase of MeOH/diethylamine (100/0.1 (v/v) (flow rate: 1.0 ml/min; detection wavelength: 214 nm). The corresponding fractions were collected and the solvent was removed by rotary evaporation to obtain two enantiomers, E21-1 and E21-2, with retention times of 3.03 and 5.17 min, respectively. The ee values of both were greater than 98%.

**Example E22: 4-chloro-5-(3-((2-chloro-5-fluorophenyl)(methoxy)methyl)-5,6-dihydroimidazo[1,2-a]pyrazin-7(8H)-yl)pyridazin-3(2H)-one**

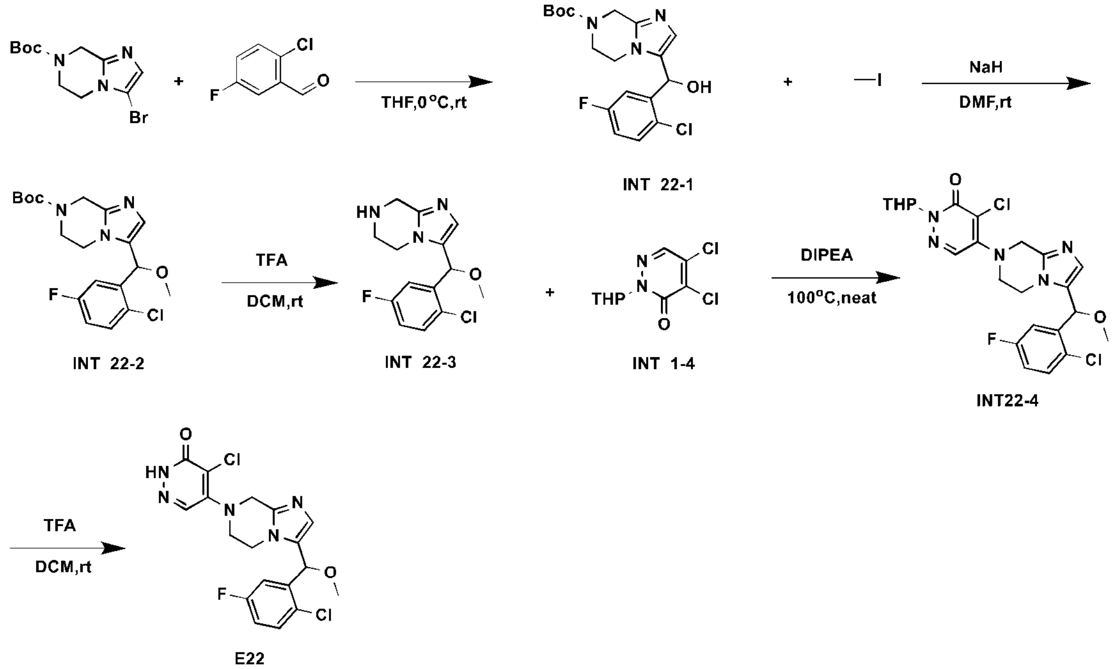


INT 22-1, INT 22-2, INT 22-3, INT 1-4, INT22-4, E22

**22.1 Preparation of Intermediate INT 22-1**

The synthesis method of intermediate INT22-1 was the same as that of INT 1-1, except that 2-chloro-5-fluorobenzaldehyde was used as the starting material instead of 4-fluoro-2-(trifluoromethyl)benzaldehyde.

**22.2 Preparation of Intermediate INT 22-2**

The synthesis method of intermediate INT 22-2 was the same as that of INT 1-2, except that INT 22-1 was used as the starting material instead of INT 1-1.

**22.3 Preparation of Intermediate INT22-3**

The synthesis method of intermediate INT 22-3 was the same as that of INT 1-3, except that INT 22-2 was used as the starting material instead of INT 1-2.

**22.4 Preparation of Intermediate INT 22-4**

The synthesis method of intermediate INT 22-4 was the same as that of INT 1-5, except that INT 22-3 was used as the starting material instead of INT 1-3.

**22.5 Preparation of Compound E22**

The synthesis method of Example E22 was the same as that of that of E1, except that the intermediate INT 22-4 was used as the starting material instead of INT 1-5. $^1$H NMR (500 MHz, DMSO-$d_6$) δ 13.12 (s, 1H), 8.02 (s, 1H), 7.63 (dd, *J* = 9.7, 4.9 Hz, 1H), 7.42 - 7.35 (m, 2H), 7.05 (s, 1H), 5.84 (s, 1H), 4.98 - 4.85 (m, 2H), 4.43 (dt, *J* = 13.1, 4.8 Hz, 1H), 4.26 (ddd, *J* = 12.6, 7.3, 4.2 Hz, 1H), 4.04 (dt, *J* = 14.6, 4.8 Hz, 1H), 3.93 (ddd, *J* = 14.4, 7.4, 4.1 Hz, 1H), 3.40 (s, 3H). MS (ESI): m/z 424.1[M + H]$^+$.

**Example E23: 4-chloro-5-(3-(methoxy(6-methoxypyridin-2-yl)methyl)-5,6-dihydroimidazo[1,2-a]pyrazin-7(8H)-yl)pyridazin-3(2H)-one**

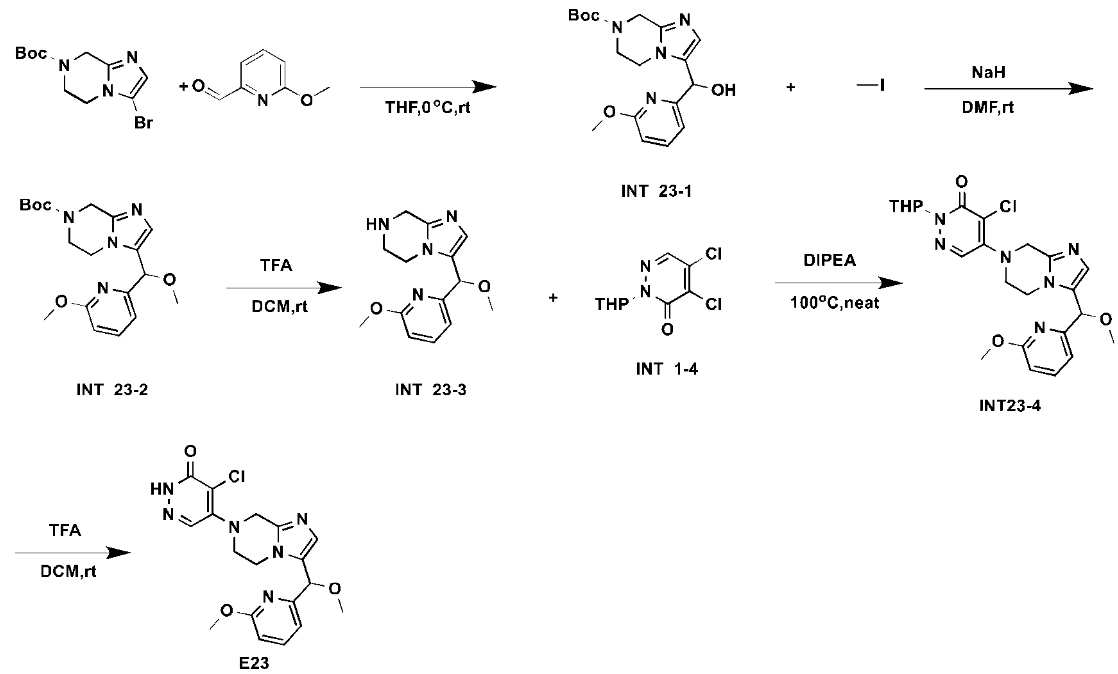


**23.1 Preparation of Intermediate INT 23-1**

The synthesis method of intermediate INT23-1 was the same as that of INT 1-1, except that 2-formyl-6-methoxypyridine was used as the starting material instead of 4-fluoro-2-(trifluoromethyl)benzaldehyde.

**23.2 Preparation of Intermediate INT 23-2**

The synthesis method of intermediate INT 23-2 was the same as that of INT 1-2, except that INT 23-1 was used as the starting material instead of INT 1-1.

**23.3 Preparation of Intermediate INT23-3**

The synthesis method of intermediate INT 23-3 was the same as that of INT 1-3, except that INT 23-2 was used as the starting material instead of INT 1-2.

**23.4 Preparation of Intermediate INT 23-4**

The synthesis method of intermediate INT 23-4 was the same as that of INT 1-5, except that INT 23-3 was used as the starting material instead of INT 1-3.

**23.5 Preparation of Compound E23**

The synthesis method of Example E23 was the same as that of that of E1, except that the intermediate INT 23-4 was used as the starting material instead of INT 1-5. 1H NMR (500 MHz, DMSO-d6) δ 13.11 (s, 1H), 8.00 (s, 1H), 7.86 - 7.80 (m, 1H), 7.33 (s, 1H), 7.19 (d, J = 7.3 Hz, 1H), 6.84 (d, J = 8.3 Hz, 1H), 5.61 (s, 1H), 5.02 - 4.91 (m, 2H), 4.41 (dt, J = 13.1, 5.0 Hz, 1H), 4.34 (dt, J = 12.8, 5.4 Hz, 1H), 4.02 - 3.90 (m, 2H), 3.78 (s, 3H), 3.39 (s, 3H). MS (ESI): m/z 403.1[M + H]$^+$.

**Example E24: 4-chloro-5-(3-((4-fluoro-2-methylphenyl)(methoxy-d3)methyl)-5,6-dihydroimidazo[1,2-a]pyrazin-7(8H)-yl)pyridazin-3(2H)-one**

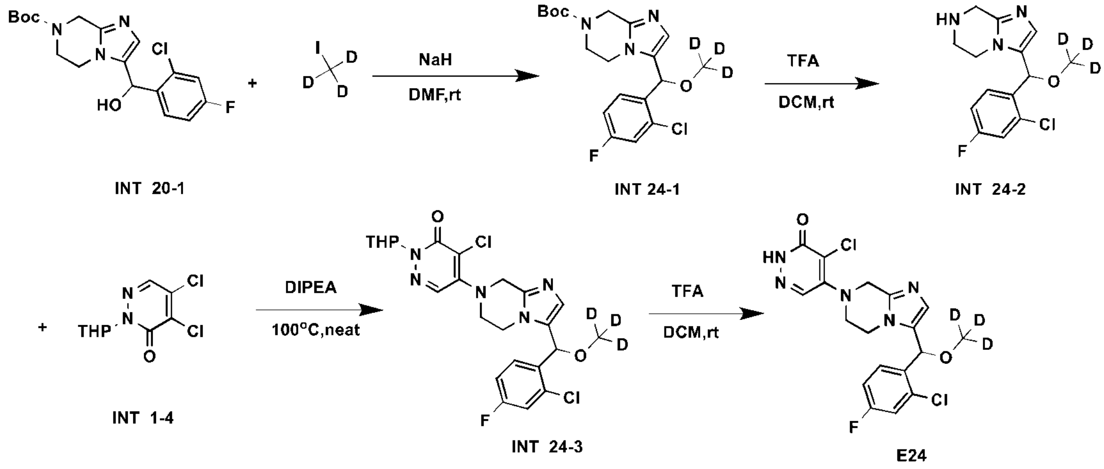


INT 20-1, INT 24-1, INT 24-2, INT 1-4, INT 24-3, E24

**24.1 Preparation of Intermediate INT 24-1**

The synthesis method of intermediate INT24-1 was the same as that of INT 12-1, except that INT 20-1 was used as the starting material instead of INT 1-1.

**24.2 Preparation of Intermediate INT 24-2**

The synthesis method of intermediate INT 24-2 was the same as that of INT 1-3, except that INT 24-1 was used as the starting material instead of INT 1-2.

**24.3 Preparation of Intermediate INT24-3**

The synthesis method of intermediate INT 24-3 was the same as that of INT 1-5, except that INT 24-2 was used as the starting material instead of INT 1-3.

**24.4 Preparation of Compound E24**

The synthesis method of Example E24 was the same as that of that of E1, except that the intermediate INT 24-3 was used as the starting material instead of INT 1-5. $^1$H NMR (500 MHz, DMSO-$d_6$) δ 13.11 (s, 1H), 8.01 (s, 1H), 7.64 - 7.55 (m, 2H), 7.40 (td, *J* = 8.5, 2.6 Hz, 1H), 7.02 (s, 1H), 5.84 (s, 1H), 4.98 - 4.89 (m, 2H), 4.40 (dt, *J* = 13.1, 4.7 Hz, 1H), 4.26 (ddd, *J* = 12.7, 7.3, 4.1 Hz, 1H), 4.03 (dt, *J* = 14.5, 4.8 Hz, 1H), 3.92 (ddd, *J* = 14.4, 7.4, 4.1 Hz, 1H). MS (ESI): m/z 407.1[M + H]$^+$.

**Example E25: 5-(2-bromo-3-((2-chloro-4-fluorophenyl)(methoxy)methyl)-5,6-dihydroimidazo[1,2-a]pyrazin-7(8H)-yl)-4-chloropyridazin-3(2H)-one**

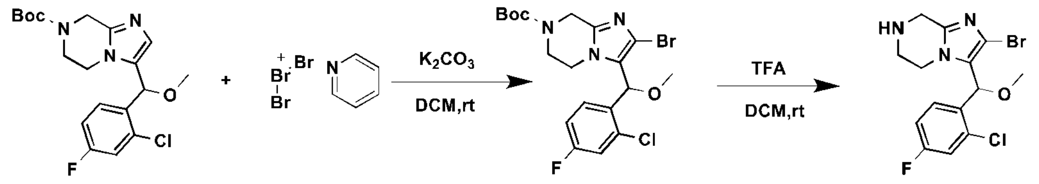


INT 21-2 INT 25-1 INT 25-2

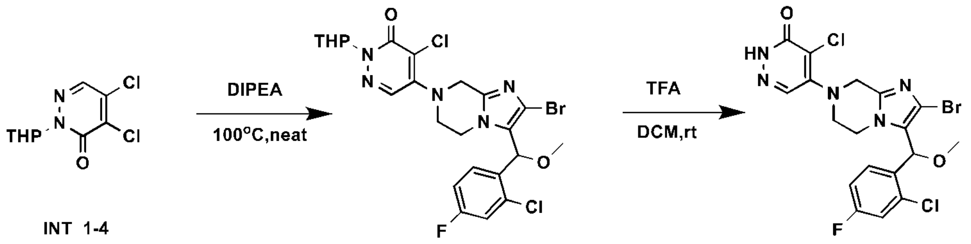


INT 1-4 INT 25-3 E25

### 25.1 Preparation of Intermediate INT 25-1

The synthesis method of intermediate INT25-1 was the same as that of INT 13-1, except that INT 21-2 was used as the starting material instead of INT 2-1.

### 25.2 Preparation of Intermediate INT 25-2

The synthesis method of intermediate INT 25-2 was the same as that of INT 1-3, except that INT 25-1 was used as the starting material instead of INT 1-2.

### 25.3 Preparation of Intermediate INT25-3

The synthesis method of intermediate INT 25-3 was the same as that of INT 1-5, except that INT 25-2 was used as the starting material instead of INT 1-3.

### 25.4 Preparation of Compound E25

The synthesis method of Example E25 was the same as that of that of E1, except that the intermediate INT 25-3 was used as the starting material instead of INT 1-5. $^1$H NMR (500 MHz, DMSO-$d_6$) δ 13.06 (s, 1H), 7.93 (s, 1H), 7.72 (dd, *J* = 8.8, 6.2 Hz, 1H), 7.49 (dd, *J* = 8.7, 2.6 Hz, 1H), 7.33 (td, *J* = 8.5, 2.6 Hz, 1H), 5.57 (s, 1H), 4.64 (s, 2H), 4.11 (ddd, *J* = 12.3, 5.8, 3.5 Hz, 1H), 3.84 (dt, *J* = 12.8, 4.1 Hz, 1H), 3.78 - 3.67 (m, 2H), 3.35 (s, 3H). MS (ESI): m/z 501.1[M + H]$^+$.

### Example E26: 5-(2-bromo-3-((4-fluoro-2-(trifluoromethyl)phenyl)(methoxy)methyl)-5,6-dihydroimidazo[1,2-a] pyrazin-7(8H)-yl)-4-chloropyridazin-3(2H)-one

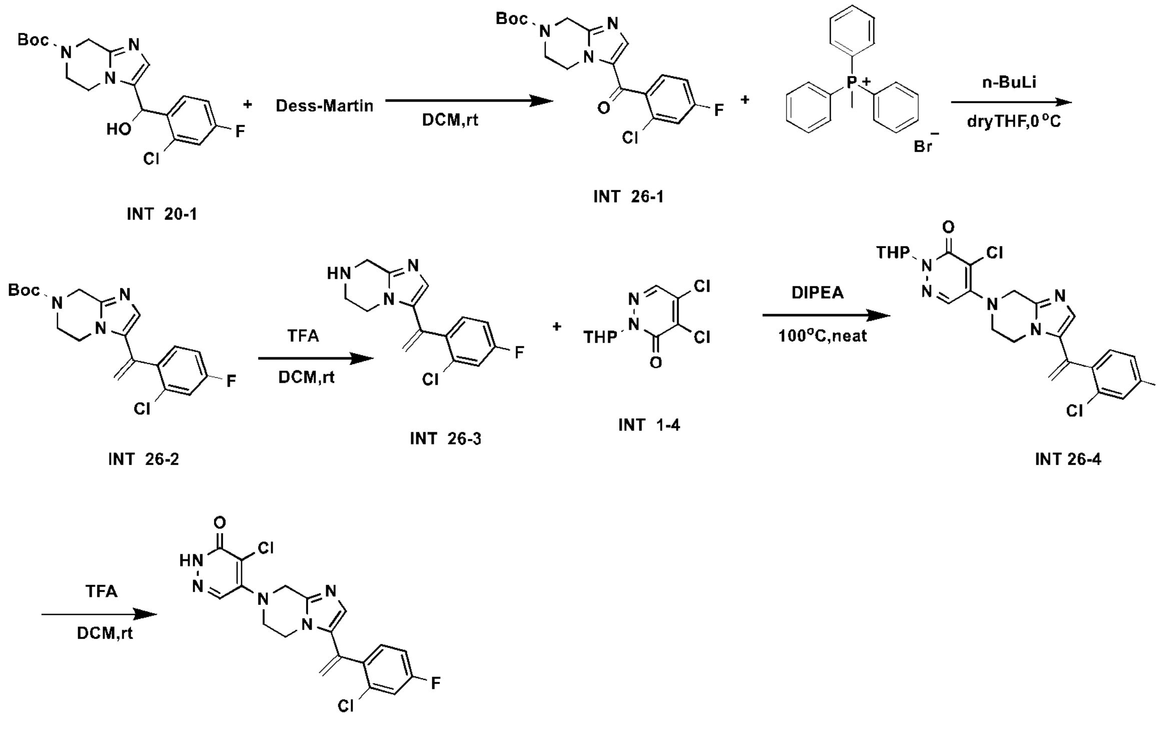


INT 20-1
INT 26-1
INT 26-2
INT 26-3
INT 1-4
INT 26-4
E26

**26.1 Preparation of Intermediate INT 26-1**

The synthesis method of intermediate INT26-1 was the same as that of INT 10-1, except that INT 20-2 was used as the starting material instead of INT 1-1.

**26.2 Preparation of Intermediate INT 26-2**

The synthesis method of intermediate INT 26-2 was the same as that of INT 10-2, except that INT 26-1 was used as the starting material instead of INT 10-1.

**26.3 Preparation of Intermediate INT26-3**

The synthesis method of intermediate INT 26-3 was the same as that of INT 1-3, except that INT 26-2 was used as the starting material instead of INT 1-2.

**26.4 Preparation of Intermediate INT26-4**

The synthesis method of intermediate INT 26-4 was the same as that of INT 1-5, except that INT 26-3 was used as the starting material instead of INT 1-5.

**26.4 Preparation of Compound E26**

The synthesis method of Example E26 was the same as that of that of E1, except that the intermediate INT 26-4 was used as the starting material instead of INT 1-5. 1H NMR (500 MHz, Methanol-d4) δ 8.01 (s, 1H), 7.56 (dd, J = 8.3, 6.3 Hz, 1H), 7.38 - 7.34 (m, 1H), 7.31 (s, 1H), 7.24 (td, J = 7.8, 2.3 Hz, 1H), 5.97 (s, 1H), 5.74 (s, 1H), 5.03 (s, 2H), 4.19 (t, J = 5.3 Hz, 2H), 4.00 (t, J = 5.3 Hz, 2H).MS (ESI): m/z 406.1[M + H]$^+$.

**Example E27: 4-chloro-5-(3-(1-(2-chloro-5-fluorophenyl)vinyl)-5,6-dihydroimidazo[1,2-a]pyrazin-7(8H)-yl)pyridazin-3(2H)-one**

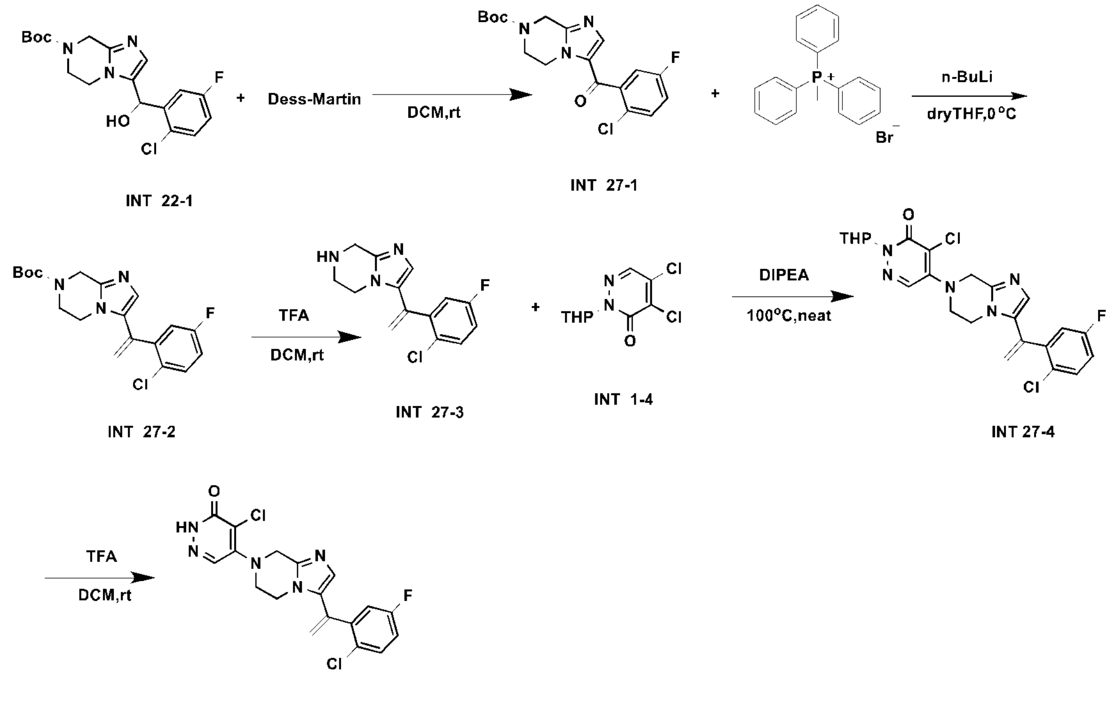


INT 22-1 INT 27-1 INT 27-2 INT 27-3 INT 1-4 INT 27-4

E27

### 27.1 Preparation of Intermediate INT 27-1

The synthesis method of intermediate INT 27-1 was the same as that of INT 10-1, except that INT 22-1 was used as the starting material instead of INT 1-1.

### 27.2 Preparation of Intermediate INT 27-2

The synthesis method of intermediate INT 27-2 was the same as that of INT 10-2, except that INT 27-1 was used as the starting material instead of INT 10-1.

### 27.3 Preparation of Intermediate INT27-3

The synthesis method of intermediate INT 27-3 was the same as that of INT 1-3, except that INT 27-2 was used as the starting material instead of INT 1-2.

### 27.4 Preparation of Intermediate INT27-4

The synthesis method of intermediate INT 27-4 was the same as that of INT 1-5, except that INT 27-3 was used as the starting material instead of INT 1-5.

### 27.4 Preparation of Compound E27

The synthesis method of Example E27 was the same as that of that of E1, except that the intermediate INT 27-4 was used as the starting material instead of INT 1-5. $^1$H NMR (500 MHz, Methanol-*d*$_4$) δ 7.99 (s, 1H), 7.49 (dd, *J* = 8.9, 5.2 Hz, 1H), 7.28 (dd, *J* = 8.8, 3.0 Hz, 1H), 7.21 (td, *J* = 8.3, 2.8 Hz, 1H), 6.95 (s, 1H), 5.86 (s, 1H), 5.58 (s, 1H), 5.37 (s, 2H), 4.14 (t, *J* = 5.3 Hz, 2H), 3.97 (t, *J* = 5.2 Hz, 2H). MS (ESI): m/z 406.1[M + H]$^+$.

**Example E28: 4-chloro-5-(3-(1-(2-chloro-4-fluorophenyl)ethyl)-5,6-dihydroimidazo[1,2-a]pyrazin-7(8H)-yl)pyridazin-3(2H)-one**

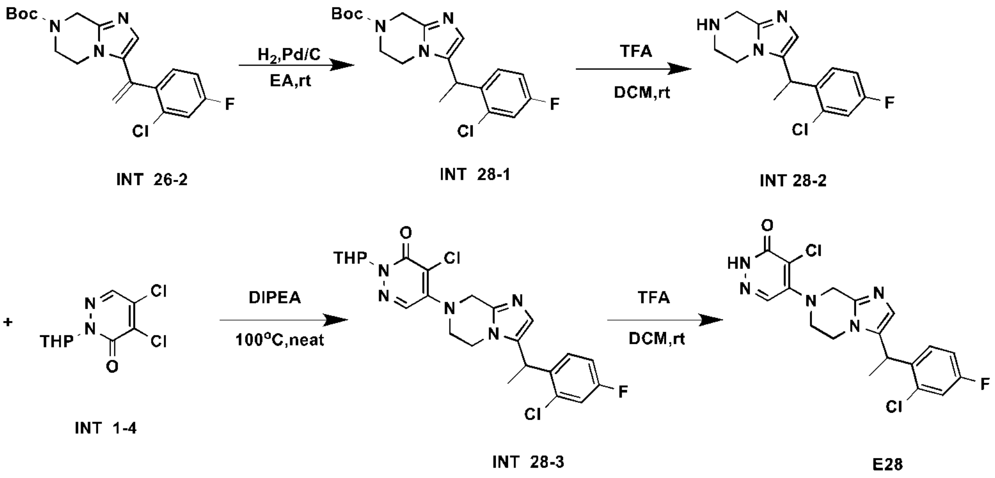


INT 26-2 INT 28-1 INT 28-2

INT 1-4 INT 28-3 E28

**28.1 Preparation of Intermediate INT 28-1**

The synthesis method of intermediate INT28-1 was the same as that of INT 11-1, except that INT 26-2 was used as the starting material instead of INT 10-2.

**28.2 Preparation of Intermediate INT 28-2**

The synthesis method of intermediate INT 28-2 was the same as that of INT 1-3, except that INT 28-1 was used as the starting material instead of INT 1-2.

**28.3 Preparation of Intermediate INT28-3**

The synthesis method of intermediate INT 28-3 was the same as that of INT 1-5, except that INT 28-2 was used as the starting material instead of INT 1-3.

**28.4 Preparation of Compound E28**

The synthesis method of Example E28 was the same as that of that of E1, except that the intermediate INT 28-3 was used as the starting material instead of INT 1-5. $^1$H NMR (500 MHz, Methanol-$d_4$) δ 7.94 (s, 1H), 7.33 (dd, $J$ = 8.7, 2.2 Hz, 1H), 7.20 (s, 1H), 7.11 - 7.05 (m, 2H), 5.04 - 4.86 (m, 2H), 4.68 (q, $J$ = 7.0 Hz, 1H), 4.15 - 4.07 (m, 1H), 3.96 - 3.81 (m, 2H), 3.53 - 3.44 (m, 1H), 1.63 (d, $J$ = 7.0 Hz, 3H). MS (ESI): m/z 408.1[M + H]$^+$.

**Example E29: 4-chloro-5-(3-(1-(2-chloro-5-fluorophenyl)ethyl)-5,6-dihydroimidazo[1,2-a]pyrazin-7(8H)-yl)pyridazin-3(2H)-one**

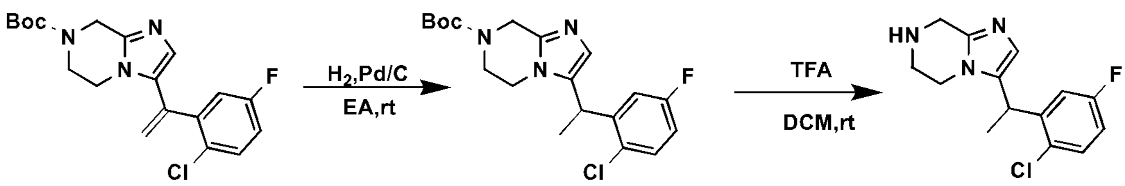


INT 27-2 INT 29-1 INT 29-2

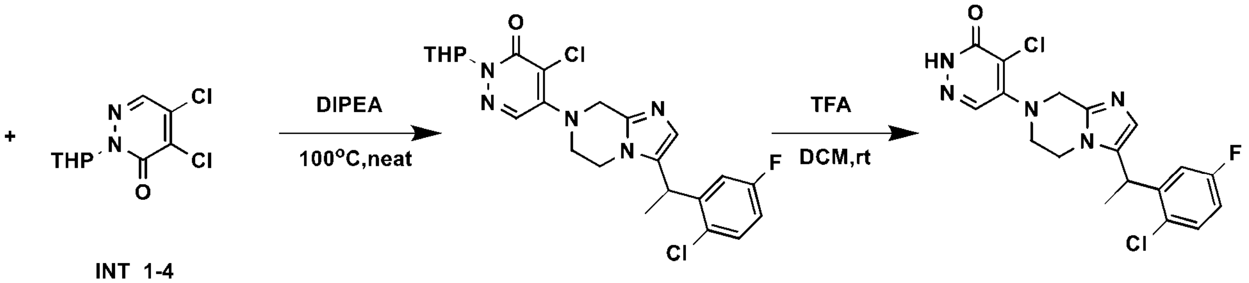


INT 1-4

INT 29-3

E29

**29.1 Preparation of Intermediate INT 29-1**

The synthesis method of intermediate INT29-1 was the same as that of INT 11-1, except that INT 27-2 was used as the starting material instead of INT 10-2.

**29.2 Preparation of Intermediate INT 29-2**

The synthesis method of intermediate INT 29-2 was the same as that of INT 1-3, except that INT 29-1 was used as the starting material instead of INT 1-2.

**29.3 Preparation of Intermediate INT 29-3**

The synthesis method of intermediate INT 29-3 was the same as that of INT 1-5, except that INT 29-2 was used as the starting material instead of INT 1-3.

**29.4 Preparation of Compound E29**

The synthesis method of Example E29 was the same as that of that of E1, except that the intermediate INT 29-3 was used as the starting material instead of INT 1-5. 1H NMR (500 MHz, Methanol-d4) δ 7.97 (s, 1H), 7.43 (s, 1H), 7.37 - 7.32 (m, 1H), 7.16 - 7.06 (m, 2H), 4.81-4.77(m, 1H), 4.74 -4.61 (m, 2H), 4.34 - 4.28 (m, 1H), 3.98 - 3.82 (m, 2H), 3.54 - 3.46 (m, 1H), 1.68 (d, J = 7.1 Hz, 3H). MS (ESI): m/z 408.1[M + H]$^+$.

**Example E30: 5-(3-(azido(2-chloro-5-fluorophenyl)methyl)-5,6-dihydroimidazo[1,2-a]pyrazin-7(8H)-yl)-4-chloropyridazin-3(2H)-one**

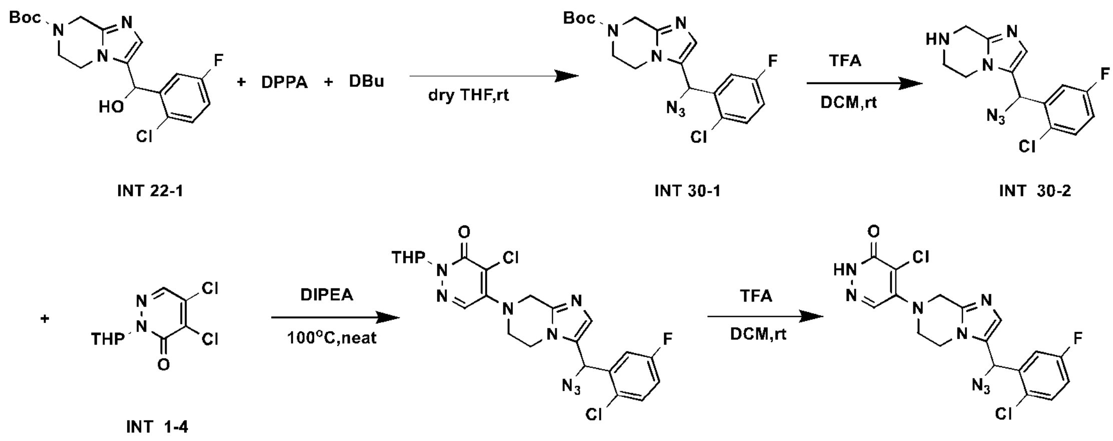


INT 22-1

INT 30-1

INT 30-2

INT 1-4

INT 30-3

E30

**30.1 Preparation of Intermediate INT 30-1**

The synthesis method of intermediate INT30-1 was the same as that of INT 9-1, except that INT 22-1 was used as

the starting material instead of INT 1-1.

**30.2 Preparation of Intermediate INT 30-2**

The synthesis method of intermediate INT 30-2 was the same as that of INT 1-3, except that INT 30-1 was used as the starting material instead of INT 1-2.

**30.3 Preparation of Intermediate INT 30-3**

The synthesis method of intermediate INT 30-3 was the same as that of INT 1-5, except that INT 30-2 was used as the starting material instead of INT 1-3.

**30.4 Preparation of Compound E30**

The synthesis method of Example E30 was the same as that of that of E1, except that the intermediate INT 30-3 was used as the starting material instead of INT 1-5. 1H NMR (500 MHz, DMSO-d6) δ 13.09 (s, 1H), 7.99 (s, 1H), 7.66 (dd, J = 8.7, 5.1 Hz, 1H), 7.45 - 7.36 (m, 2H), 6.68 (s, 1H), 6.58 (s, 1H), 4.88 - 4.77 (m, 2H), 4.23 - 4.16 (m, 1H), 4.11 (dd, J = 12.6, 5.1 Hz, 1H), 3.98 - 3.91 (m, 1H), 3.91 - 3.84 (m, 1H). MS (ESI): m/z 434.06 $[M + H]^+$.

**Example E31: (E)-3-(2-chloro-5-fluorophenyl)-3-(7-(5-chloro-6-oxo-1,6-dihydropyridazin-4-yl)-5,6,7,8-tetrahydroimidazo[1,2-a]pyrazin-3-yl)acrylonitrile**

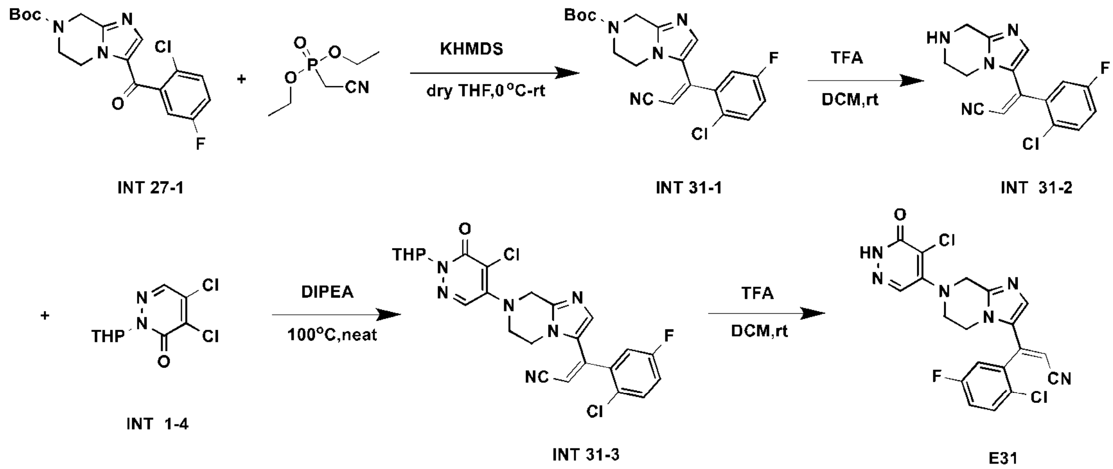


INT 27-1 INT 31-1 INT 31-2 INT 1-4 INT 31-3 E31

**31.1 Preparation of Intermediate INT 31-1**

Diethyl cyanomethylphosphonate (186.5 mg, 1.05 mmol) was added to a three-necked flask, dissolved in anhydrous THF, protected with nitrogen, and placed at 0 °C. KHMDS (0.5 M/L, 2.1 ml, 1.05 mmol) was slowly added. Then the mixture was stirred at room temperature for 40 minutes. Then the mixture was placed at 0 °C. The intermediate INT 27-1 (200 mg, 0.52 mmol) was dissolved in 1 ml of anhydrous THF and slowly added dropwise to the reaction mixture. The mixture was refluxed at room temperature overnight. After completion of the reaction monitored by TLC, the reaction was quenched with saturated ammonium chloride aqueous solution and extracted with EA. The organic phase was washed three times with saturated brine, dried over anhydrous magnesium sulfate, and filtered. The filtrate was concentrated and separated by flash silica gel chromatography using MeOH/DCM as the mobile phase to collect the target product, i.e., the intermediate INT 31-1, approximately 140 mg.

**31.2 Preparation of Intermediate INT 31-2**

The synthesis method of intermediate INT 31-2 was the same as that of INT 1-3, except that INT 31-1 was used as the starting material instead of INT 1-2.

### 31.3 Preparation of Intermediate INT 31-3

The synthesis method of intermediate INT 31-3 was the same as that of INT 1-5, except that INT 31-2 was used as the starting material instead of INT 1-3.

### 31.4 Preparation of Compound E31

The synthesis method of Example E31 was the same as that of that of E1, except that the intermediate INT 31-3 was used as the starting material instead of INT 1-5. 1H NMR (500 MHz, DMSO-d6) δ 13.04(s, 1H), δ 7.92 (s, 1H), 7.61 (dd, J = 8.9, 5.0 Hz, 1H), 7.52 (s, 1H), 7.48 (dd, J = 9.0, 2.9 Hz, 2H), 6.04 (s, 1H), 5.52 (s, 1H), 4.75 (s, 2H), 4.23 (t, J = 5.4 Hz, 1H), 3.86 (t, J = 5.3 Hz, 1H), 3.70 (t, J = 5.3 Hz, 2H). MS (ESI): m/z 430.05 [M + H]$^+$.

### Example E32: 4-chloro-5-(3-((2-chloro-4-fluorophenyl)(methoxy)methyl)-2-(trifluoromethyl)-5,6-dihydroimidazo[1,2-a]pyrazin-7(8H)-yl)pyridazin-3(2H)-one

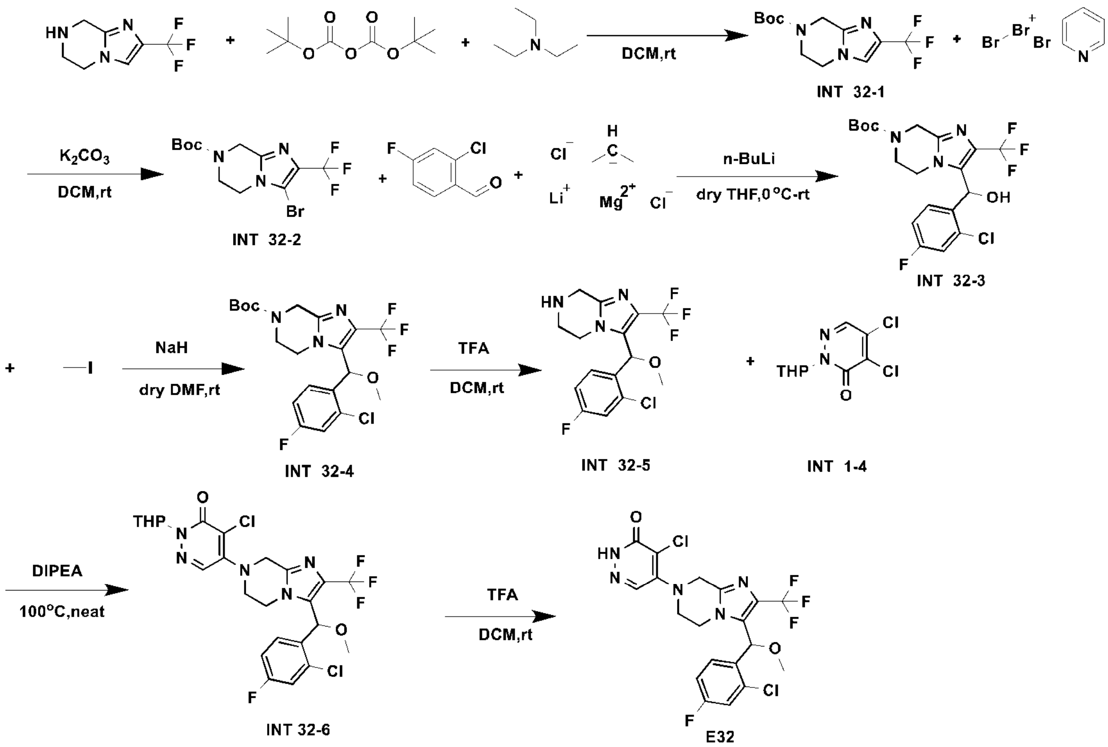


### 32.1 Preparation of Intermediate INT 32-1

2-Trifluoromethyl-5,6,7,8-tetrahydroimidazo[1,2-a]pyrazine (500 mg, 2.6 mmol) was added to a single-necked bottle and dissolved in DCM. Di-tert-butyl dicarbonate (685.6 mg, 3.1 mmol) and triethylamine (317.3 mg, 3.1 mmol) were added and stirred at room temperature for 48 hours. The solvent was removed and the residue was separated by flash silica gel chromatography using MeOH/DCM as the mobile phase to collect the target product, i.e., the intermediate INT 32-1, which was approximately 500 mg.

### 32.2 Preparation of Intermediate INT 32-2

The synthesis method of intermediate INT 32-2 was the same as that of INT 13-1, except that INT 32-1 was used as the starting material instead of INT 2-1.

### 32.3 Preparation of Intermediate INT 32-3

The synthesis method of intermediate INT 32-3 was the same as that of INT 1-1, except that INT 32-2 was used

instead of 7-Boc-3-bromo-5,6,7,8-tetrahydroimidazo[1,2-a]pyrazine; and 2-chloro-4-fluorobenzaldehyde was used as the starting material instead of 4-fluoro-2-(trifluoromethyl)benzaldehyde.

### 32.4 Preparation of Intermediate INT 32-4

The synthesis method of intermediate INT 32-4 was the same as that of INT 1-2, except that INT 32-3 was used as the starting material instead of INT 1-1.

### 32.5 Preparation of intermediate INT 32-5

The synthesis method of intermediate INT 31-5 was the same as that of INT 1-3, except that INT 32-4 was used as the starting material instead of INT 1-2.

### 32.6 Preparation of intermediate INT 32-6

The synthesis method of intermediate INT 32-6 was the same as that of INT 1-5, except that INT 32-5 was used as the starting material instead of INT 1-3.

### 32.7 Preparation of Compound E32

The synthesis method of Example E32 was the same as that of that of E1, except that the intermediate INT 32-6 was used as the starting material instead of INT 1-5. 1H NMR (500 MHz, DMSO-d6) δ 13.06 (s, 1H), 7.94 (s, 1H), 7.59 (dd, J = 8.8, 6.1 Hz, 1H), 7.51 (dd, J = 8.7, 2.6 Hz, 1H), 7.30 (td, J = 8.5, 2.7 Hz, 1H), 5.81 (s, 1H), 4.73 (s, 2H), 4.16-4.12 (m, 1H), 3.84 (td, J = 12.3, 11.2, 5.5 Hz, 1H), 3.80 - 3.72 (m, 2H), 3.38 (s, 3H). MS (ESI): m/z 492.1[M + H]$^+$.

## Example E33: 4-chloro-5-(3-((4-fluoro-2-nitrophenyl)(methoxy)methyl)-5,6-dihydroimidazo[1,2-a]pyrazin-7(8H)-yl)pyridazin-3(2H)-one

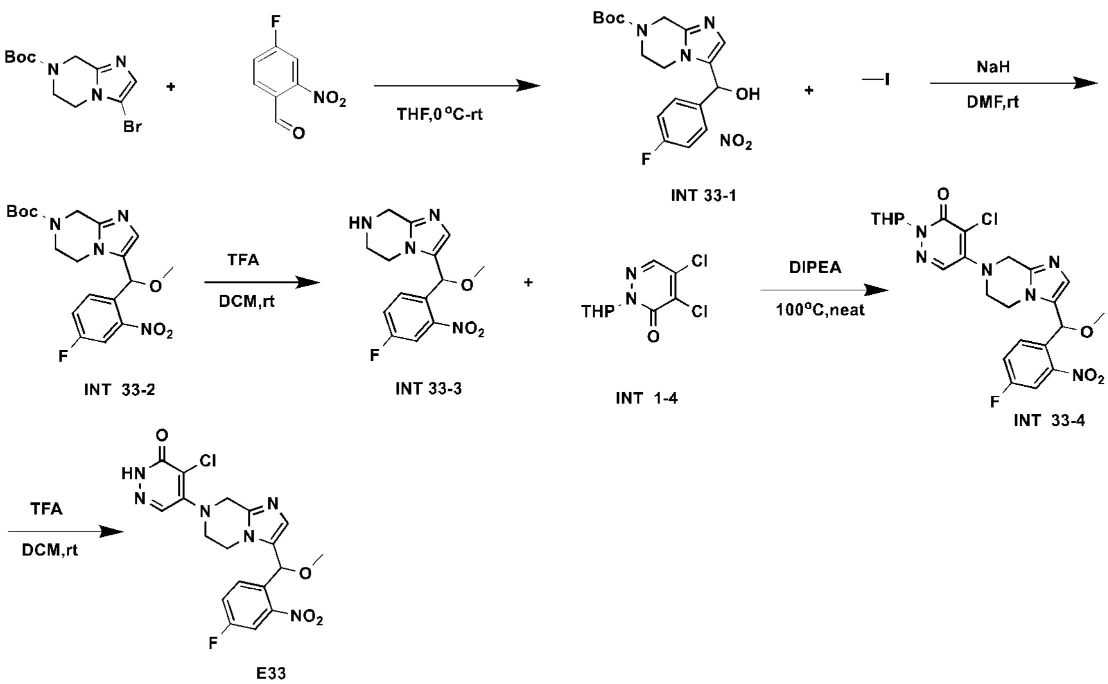


### 33.1 Preparation of Intermediate INT 33-1

The synthesis method of intermediate INT33-1 was the same as that of INT 1-1, except that 4-fluoro-2-nitrobenzaldehyde was used instead of 4-fluoro-2-(trifluoromethyl)benzaldehyde as the starting material.

### 33.2 Preparation of Intermediate INT 33-2

The synthesis method of intermediate INT 33-2 was the same as that of INT 1-2, except that INT 33-1 was used as the starting material instead of INT 1-1.

### 33.3 Preparation of Intermediate INT33-3

The synthesis method of intermediate INT 33-3 was the same as that of INT 1-3, except that INT 33-2 was used as the starting material instead of INT 1-2.

### 33.4 Preparation of Intermediate INT 33-4

The synthesis method of intermediate INT 33-4 was the same as that of INT 1-5, except that INT 33-3 was used as the starting material instead of INT 1-3.

### 33.5 Preparation of Compound E33

The synthesis method of Example E33 was the same as that of that of E1, except that the intermediate INT 33-4 was used as the starting material instead of INT 1-5. $^1$H NMR (500 MHz, DMSO-$d_6$) δ 13.12 (s, 1H), 8.16 - 8.11 (m, 1H), 8.03 (s, 1H), 7.88 - 7.80 (m 2H), 7.07 (s, 1H), 6.09 (s, 1H), 4.96 (s, 2H), 4.40 (dt, *J* = 13.2, 4.9 Hz, 1H), 4.24 (ddd, *J* = 12.7, 7.1, 4.2 Hz, 1H), 4.04 (dt, *J* = 14.6, 4.9 Hz, 1H), 3.96 (ddd, *J* = 14.4, 7.1, 4.2 Hz, 1H), 3.34 (s, 3H). MS (ESI): m/z 435.1[M + H]$^+$.

### Example E34: 4-chloro-5-(3-(1-(2-chloro-4-fluorophenyl)-1-methoxyethyl)-5,6-dihydroimidazo[1,2-a]pyrazin-7(8H)-yl)pyridazin-3(2H)-one

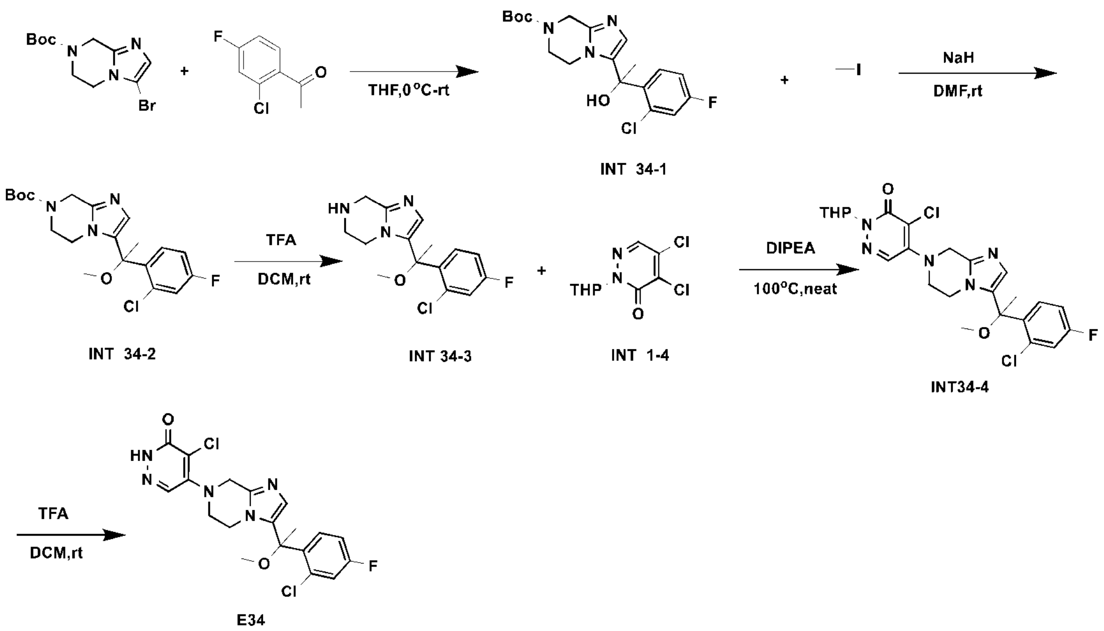


INT 34-1 INT 34-2 INT 34-3 INT 1-4 INT34-4 E34

### 34.1 Preparation of Intermediate INT 34-1

The synthesis method of intermediate INT34-1 was the same as that of INT 1-1, except that 2-chloro-4-fluoroacetophenone was used instead of 4-fluoro-2-(trifluoromethyl)benzaldehyde as the starting material.

### 34.2 Preparation of Intermediate INT 34-2

The synthesis method of intermediate INT 34-2 was the same as that of INT 1-2, except that INT 34-1 was used as the starting material instead of INT 1-1.

### 34.3 Preparation of Intermediate INT34-3

The synthesis method of intermediate INT 34-3 was the same as that of INT 1-3, except that INT 34-2 was used as the starting material instead of INT 1-2.

### 34.4 Preparation of Intermediate INT 34-4

The synthesis method of intermediate INT 34-4 was the same as that of INT 1-5, except that INT 34-3 was used as the starting material instead of INT 1-3.

### 34.5 Preparation of Compound E34

The synthesis method of Example E34 was the same as that of that of E1, except that the intermediate INT 34-4 was used as the starting material instead of INT 1-5. $^1$H NMR (500 MHz, DMSO-$d_6$) δ 13.08 (s, 1H), 7.92 (s, 1H), 7.90 (dd, *J* = 9.1, 6.4 Hz, 1H), 7.79 (s, 1H), 7.44 (dd, *J* = 8.7, 2.8 Hz, 1H), 7.34 (ddd, *J* = 9.0, 7.9, 2.7 Hz, 1H), 5.02 - 4.84 (m, 2H), 3.88 (dd, *J* = 13.1, 4.9 Hz, 1H), 3.75 (td, *J* = 9.0, 8.0, 4.2 Hz, 1H), 3.70 (dt, *J* = 14.3, 3.5 Hz, 1H), 3.23 - 3.17 (m, 1H), 3.16 (s, 3H), 1.90 (s, 3H). MS (ESI): m/z 438.1[M + H]$^+$.

### Example E35: 4-chloro-5-(3-(1-(4-fluoro-2-(trifluoromethyl)phenyl)-1-methoxyethyl)-5,6-dihydroimidazo[1,2-a]pyrazin-7(8H)-yl)pyridazin-3(2H)-one

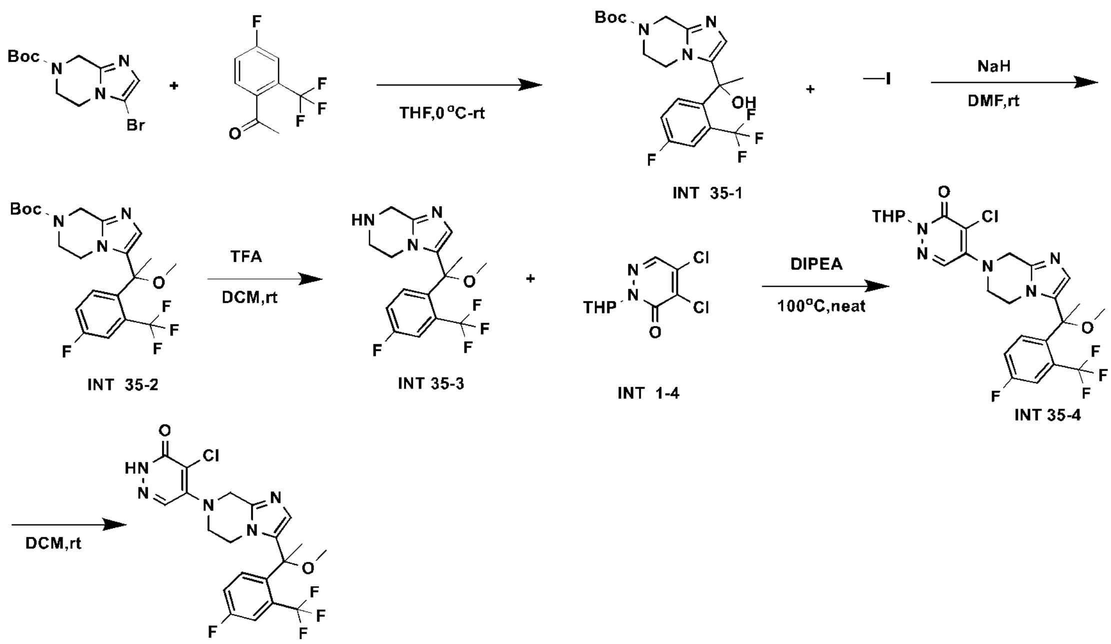


E35

### 35.1 Preparation of Intermediate INT 35-1

The synthesis method of intermediate INT35-1 was the same as that of INT 1-1, except that 4-fluoro-2-trifluoromethylacetophenone was used as the starting material instead of 4-fluoro-2-(trifluoromethyl)benzaldehyde.

### 35.2 Preparation of Intermediate INT 35-2

The synthesis method of intermediate INT 35-2 was the same as that of INT 1-2, except that INT 35-1 was used as the starting material instead of INT 1-1.

### 35.3 Preparation of Intermediate INT35-3

The synthesis method of intermediate INT 35-3 was the same as that of INT 1-3, except that INT 35-2 was used as the starting material instead of INT 1-2.

### 35.4 Preparation of Intermediate INT 35-4

The synthesis method of intermediate INT 35-4 was the same as that of INT 1-5, except that INT 35-3 was used as the starting material instead of INT 1-3.

### 35.5 Preparation of Compound E35

The synthesis method of Example E35 was the same as that of that of E1, except that the intermediate INT 35-4 was used as the starting material instead of INT 1-5. $^1$H NMR (500 MHz, DMSO-$d_6$) δ 13.06 (s, 1H), 7.92 (s, 1H), 7.75 - 7.70 (m, 1H), 7.48 (dd, *J* = 20.3, 11.0 Hz, 2H), 6.68 (s, 1H), 4.80 (s, 2H), 3.92 - 3.84 (m, 1H), 3.70 (ddd, *J* = 14.3, 7.0, 4.0 Hz, 1H), 3.66 - 3.57 (m, 1H), 3.23 - 3.17 (m, 1H), 3.10 (s, 3H), 1.82 (s, 3H). MS (ESI): m/z 472.1[M + H]$^+$.

### Example E36: 4-chloro-5-(3-((4-fluoro-2-(trifluoromethyl)phenyl)(methoxy)methyl)-5,6-dihydro-[1,2,4]triazolo[4,3-a]pyrazin-7(8H)-yl)pyridazin-3(2H)-one

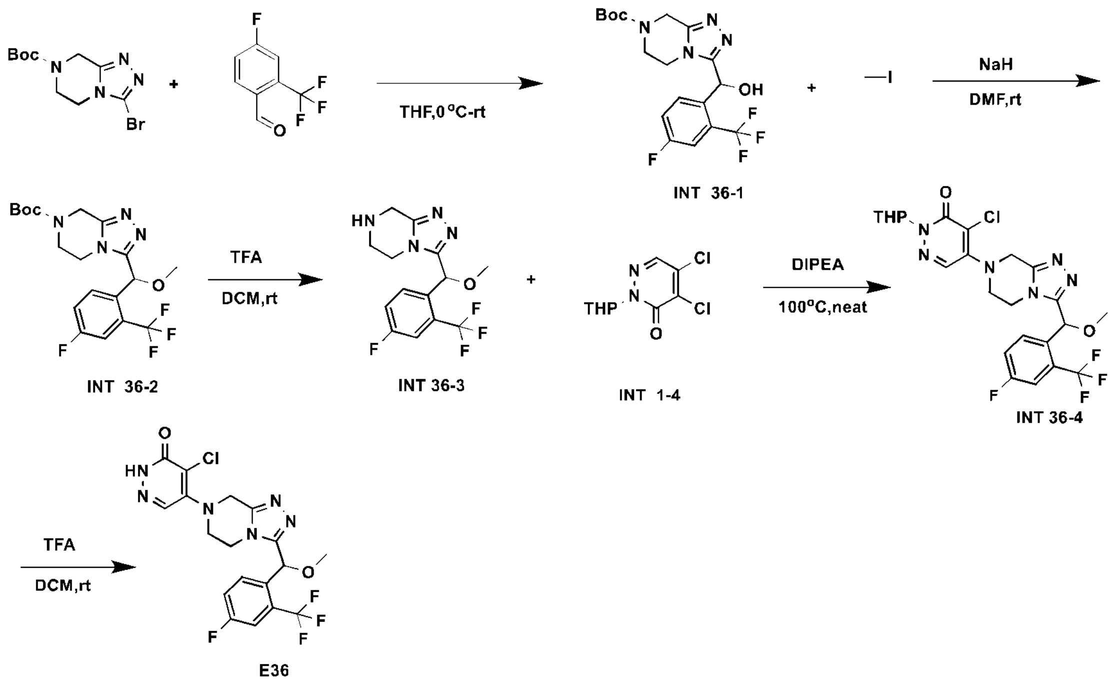

INT 36-1
INT 36-2
INT 36-3
INT 1-4
INT 36-4
E36

### 36.1 Preparation of Intermediate INT 36-1

The synthesis method of intermediate INT36-1 was the same as that of INT 1-1, except that 7-Boc-3-bromo-5,6,7,8-tetrahydro-1,2,4-triazolo[4,3-a]pyrazine was used as the starting material instead of 7-Boc-3-bromo-5,6,7,8-tetrahydroimidazo[1,2-a]pyrazine.

### 36.2 Preparation of Intermediate INT 36-2

The synthesis method of intermediate INT 36-2 was the same as that of INT 1-2, except that INT 36-1 was used as the starting material instead of INT 1-1.

### 36.3 Preparation of Intermediate INT36-3

The synthesis method of intermediate INT 36-3 was the same as that of INT 1-3, except that INT 36-2 was used as the starting material instead of INT 1-2.

### 36.4 Preparation of Intermediate INT 36-4

The synthesis method of intermediate INT 36-4 was the same as that of INT 1-5, except that INT 36-3 was used as the starting material instead of INT 1-3.

### 36.5 Preparation of Compound E36

The synthesis method of Example E36 was the same as that of that of E1, except that the intermediate INT 36-4 was used as the starting material instead of INT 1-5. 1H NMR (500 MHz, Chloroform-d) δ 7.81 (dd, J = 8.7, 5.4 Hz, 1H), 7.77 (s, 1H), 7.43 - 7.39 (m, 1H), 7.37 (dd, J = 8.2, 2.7 Hz, 1H), 5.74 (s, 1H), 4.81 - 4.71 (m, 2H), 4.36 (ddd, J = 11.9, 7.0, 4.2 Hz, 1H), 4.20 (ddd, J = 12.2, 6.0, 4.1 Hz, 1H), 3.95 (ddd, J = 14.1, 6.0, 4.3 Hz, 1H), 3.87 (ddd, J = 14.0, 7.1, 4.1 Hz, 1H), 3.20 (s,3H).MS (ESI): m/z 459.1[M + H]$^+$.

### Example E37: 4-chloro-5-(3-(ethoxy(4-fluoro-2-(trifluoromethyl)phenyl)methyl)-5,6-dihydro-[1,2,4]triazolo[4,3-a]pyrazin-7(8H)-yl)pyridazin-3(2H)-one

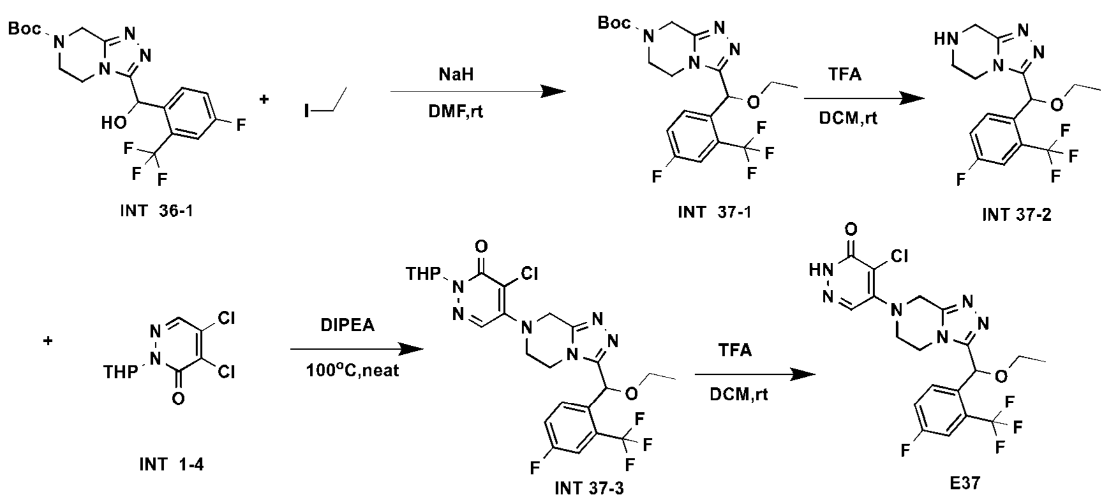


INT 36-1 INT 37-1 INT 37-2 INT 1-4 INT 37-3 E37

### 37.1 Preparation of Intermediate INT 37-1

The synthesis method of intermediate INT 37-1 was the same as that of INT 1-2, except that INT 36-1 was used as the starting material instead of INT 1-1, and iodoethane was used as the starting material instead of iodomethane.

### 37.2 Preparation of Intermediate INT 37-2

The synthesis method of intermediate INT 37-2 was the same as that of INT 1-3, except that INT 37-1 was used as the starting material instead of INT 1-2.

### 37.3 Preparation of Intermediate INT 37-3

The synthesis method of intermediate INT 37-3 was the same as that of INT 1-5, except that INT 37-2 was used as the starting material instead of INT 1-3.

### 37.4 Preparation of Compound E37

The synthesis method of Example E37 was the same as that of that of E1, except that the intermediate INT 37-3

was used as the starting material instead of INT 1-5. 1H NMR (500 MHz, ) δ 8.03 (s, 1H), 7.91 (dd, J = 8.6, 5.4 Hz, 1H), 7.62 - 7.51 (m, 2H), 6.03 (s, 1H), 5.16 - 5.02 (m, 2H), 4.41 (d, J = 4.6 Hz, 2H), 4.15 - 4.05 (m, 1H), 3.95 (dd, J = 13.4, 7.0 Hz, 1H), 3.72 - 3.65 (m, 1H), 3.57 (p, J = 7.2 Hz, 1H), 1.27 (t, J = 7.4 Hz, 3H). MS (ESI): m/z 472.10[M + H]$^+$.

**Example E38: 4-chloro-5-(3-((cyclobutylmethoxy)(4-fluoro-2-(trifluoromethyl)phenyl)methyl)-5,6-dihydro-[1,2,4]triazolo[4,3-a]pyrazin-7(8H)-yl)pyridazin-3(2H)-one**

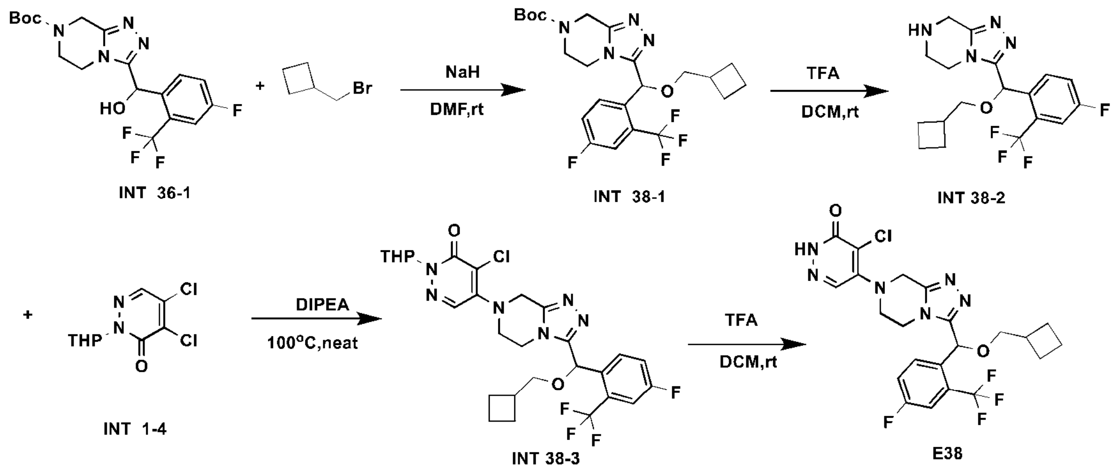


**38.1 Preparation of Intermediate INT 38-1**

The synthesis method of intermediate INT 38-1 was the same as that of INT 1-2, except that INT 36-1 was used instead of INT 1-1 as the starting material, and bromomethylcyclobutane was used instead of iodomethane as the starting material.

**38.2 Preparation of Intermediate INT 38-2**

The synthesis method of intermediate INT 38-2 was the same as that of INT 1-3, except that INT 38-1 was used as the starting material instead of INT 1-2.

**38.3 Preparation of Intermediate INT 38-3**

The synthesis method of intermediate INT 38-3 was the same as that of INT 1-5, except that INT 38-2 was used as the starting material instead of INT 1-3.

**38.4 Preparation of Compound E38**

The synthesis method of Example E38 was the same as that of that of E1, except that the intermediate INT 38-3 was used as the starting material instead of INT 1-5. 1H NMR (500 MHz, Chloroform-d) δ 11.46 (s, 1H), 7.90 (dd, J = 8.7, 5.4 Hz, 1H), 7.76 (s, 1H), 7.45 (dd, J = 9.0, 2.5 Hz, 1H), 7.41 (dd, J = 8.2, 2.8 Hz, 1H), 5.87 (s, 1H), 4.90 - 4.79 (m, 2H), 4.41 (ddd, J = 11.8, 7.2, 4.2 Hz, 1H), 4.26 (ddd, J = 12.3, 5.9, 4.1 Hz, 1H), 4.06 - 4.00 (m, 1H), 3.94 (ddd, J = 14.1, 7.1, 4.1 Hz, 1H), 3.50 (dd, J = 9.2, 6.6 Hz, 1H), 3.44 (dd, J = 9.1, 7.0 Hz, 1H), 2.62 (dq, J = 14.8, 7.4 Hz, 1H), 2.07 (tq, J = 11.3, 3.6 Hz, 2H), 1.96 (dt, J = 11.2, 8.5 Hz, 1H), 1.86 (ddt, J = 15.4, 8.6, 4.4 Hz, 1H), 1.78 - 1.72 (m, 2H). MS (ESI): m/z 512.14[M + H]$^+$.

**Example E39: 4-chloro-5-(3-((4-fluoro-2-methylphenyl)(methoxy-d3)methyl)-5,6-dihydroimidazo[1,2-a]pyrazin-7(8H)-yl)pyridazin-3(2H)-one**

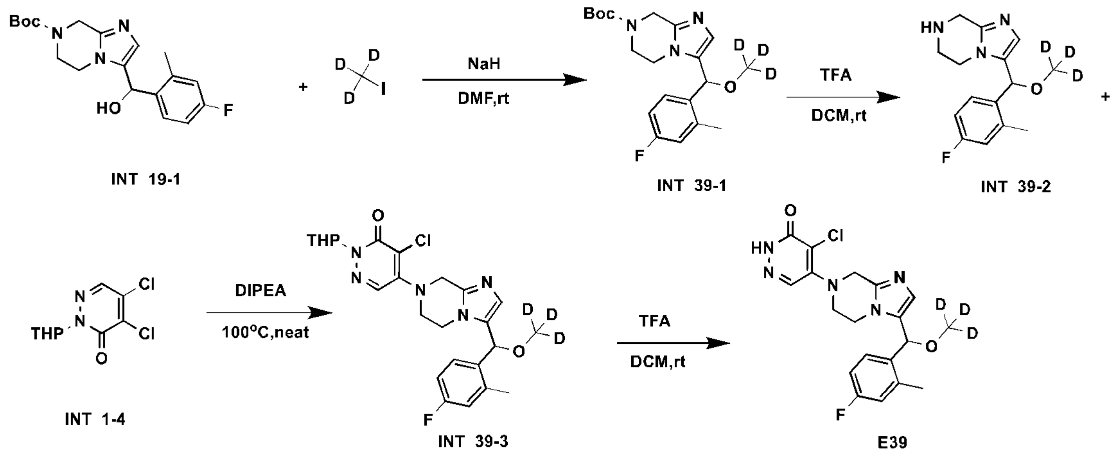


INT 19-1 INT 39-1 INT 39-2 INT 1-4 INT 39-3 E39

### 39.1 Preparation of Intermediate INT 39-1

The synthesis method of intermediate INT 39-1 was the same as that of INT 12-1, except that INT 19-1 was used as the starting material instead of INT 1-1.

### 39.2 Preparation of Intermediate INT 39-2

The synthesis method of intermediate INT 39-2 was the same as that of INT 1-3, except that INT39-1 was used as the starting material instead of INT 1-2.

### 39.3 Preparation of Intermediate INT39-3

The synthesis method of intermediate INT 39-3 was the same as that of INT 1-5, except that INT 39-2 was used as the starting material instead of INT 1-3.

### 39.4 Preparation of Compound E39

The synthesis method of Example E39 was the same as that of that of E1, except that the intermediate INT 39-3 was used as the starting material instead of INT 1-5. 1H NMR (600 MHz, DMSO-d6) δ 13.10 (s, 1H), 8.01 (s, 1H), 7.72 (dd, J = 8.4, 2.6 Hz, 1H), 7.58 (dd, J = 8.7, 6.1 Hz, 1H), 7.44 (td, J = 8.5, 2.6 Hz, 1H), 6.93 (s, 1H), 5.75 (s, 1H), 4.97 - 4.84 (m, 2H), 4.39 (dt, J = 13.5, 4.8 Hz, 1H), 4.31 - 4.23 (m, 1H), 4.02 (dt, J = 14.6, 4.9 Hz, 1H), 3.92 (ddd, J = 14.6, 7.4, 4.1 Hz, 1H). MS (ESI): m/z 460.14[M + H]$^+$.

## Example E40: 5-(3-((benzyloxy)(4-fluoro-2-(trifluoromethyl)phenyl)methyl)-5,6-dihydro-[1,2,4]triazolo[4,3-a]pyrazin-7(8H)-yl)-4-chloropyridazin-3(2H)-one

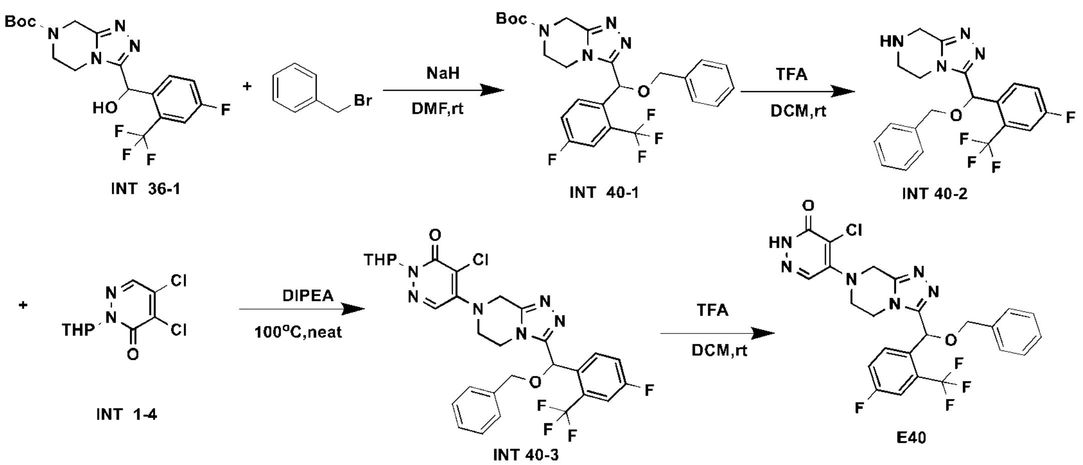


### 40.1 Preparation of Intermediate INT 40-1

The synthesis method of intermediate INT 40-1 was the same as that of INT 1-2, except that INT 36-1 was used instead of INT 1-1 as the starting material, and benzyl bromide was used instead of iodomethane as the starting material.

### 40.2 Preparation of Intermediate INT 40-2

The synthesis method of intermediate INT 40-2 was the same as that of INT 1-3, except that INT 40-1 was used as the starting material instead of INT 1-2.

### 40.3 Preparation of Intermediate INT 40-3

The synthesis method of intermediate INT 40-3 was the same as that of INT 1-5, except that INT 40-2 was used as the starting material instead of INT 1-3.

### 40.4 Preparation of Compound E40

The synthesis method of Example E40 was the same as that of that of E1, except that the intermediate INT 40-3 was used as the starting material instead of INT 1-5. 1H NMR (500 MHz, Methanol-d4 ) δ 8.07 (dd, J = 8.5, 5.4 Hz, 1H), 7.69 (s, 1H), 7.48 (t, J = 2.5 Hz, 1H), 7.47 (d, J = 3.6 Hz, 1H), 7.42 (d, J = 1.7 Hz, 1H), 7.41 - 7.39 (m, 2H), 7.34 (dd, J = 7.6, 1.9 Hz, 2H), 6.00 (s, 1H), 4.86 - 4.75 (m, 2H), 4.65 (d, J = 11.5 Hz, 1H), 4.46 (d, J = 11.4 Hz, 1H), 4.05 (ddd, J = 11.4, 7.2, 3.9 Hz, 1H), 3.99 - 3.86 (m, 2H), 3.78 (ddd, J = 13.9, 7.1, 3.8 Hz, 1H).MS (ESI): m/z 459.1[M + H]$^+$.

## Example E41: 4-chloro-5-(3-(4-fluoro-2-(trifluoromethyl)benzoyl)-5,6-dihydro-[1,2,4]triazolo[4,3-a]pyrazin-7(8H)-yl)pyridazin-3(2H)-one

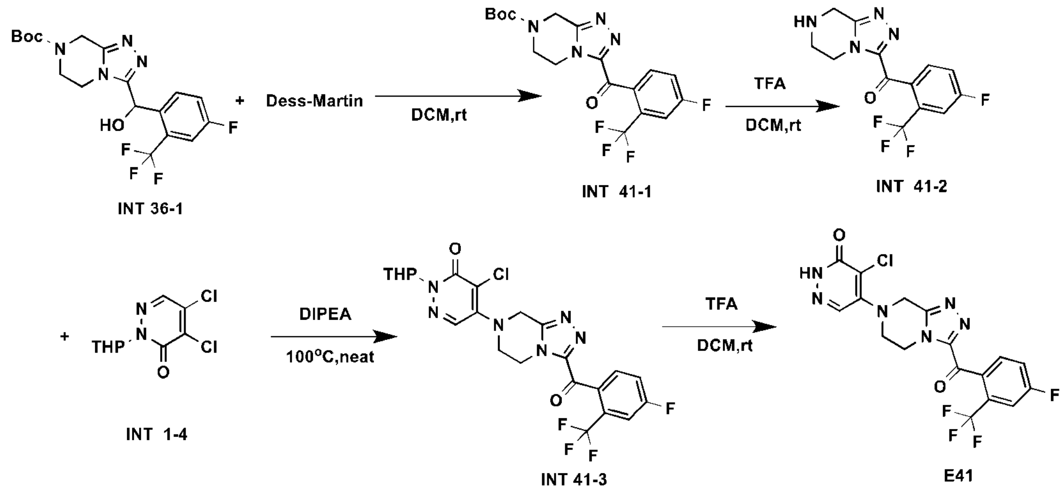


INT 36-1 INT 41-1 INT 41-2 INT 1-4 INT 41-3 E41

### 41.1 Preparation of Intermediate INT 41-1

The synthesis method of intermediate INT 41-1 was the same as that of INT 10-1, except that INT 36-1 was used as the starting material instead of INT 1-1.

### 41.2 Preparation of Intermediate INT 41-2

The synthesis method of intermediate INT 41-2 was the same as that of INT 1-3, except that INT 41-1 was used as the starting material instead of INT 1-2.

### 41.3 Preparation of Intermediate INT 41-3

The synthesis method of intermediate INT 41-3 was the same as that of INT 1-5, except that INT 41-2 was used as the starting material instead of INT 1-3.

### 41.4 Preparation of Compound E41

The synthesis method of Example E41 was the same as that of that of E1, except that the intermediate INT 41-3 was used as the starting material instead of INT 1-5. 1H NMR (500 MHz, DMSO-d6) δ 13.11 (s, 1H), 8.03 (s, 1H), 7.97 (dd, J = 8.7, 5.4 Hz, 1H), 7.90 (dd, J = 9.3, 2.6 Hz, 1H), 7.82 - 7.70 (m, 1H), 4.99 (s, 2H), 4.55 (t, J = 5.5 Hz, 2H), 3.91 (t, J = 5.4 Hz, 2H). MS (ESI): m/z 443.1[M + H]$^+$.

## Example E42: (E)-3-(7-(5-chloro-6-oxo-1,6-dihydropyridazin-4-yl)-5,6,7,8-tetrahydro-[1,2,4]triazolo[4,3-a]pyrazin-3-yl)-3-(4-fluoro-2-(trifluoromethyl)phenyl)acrylonitrile

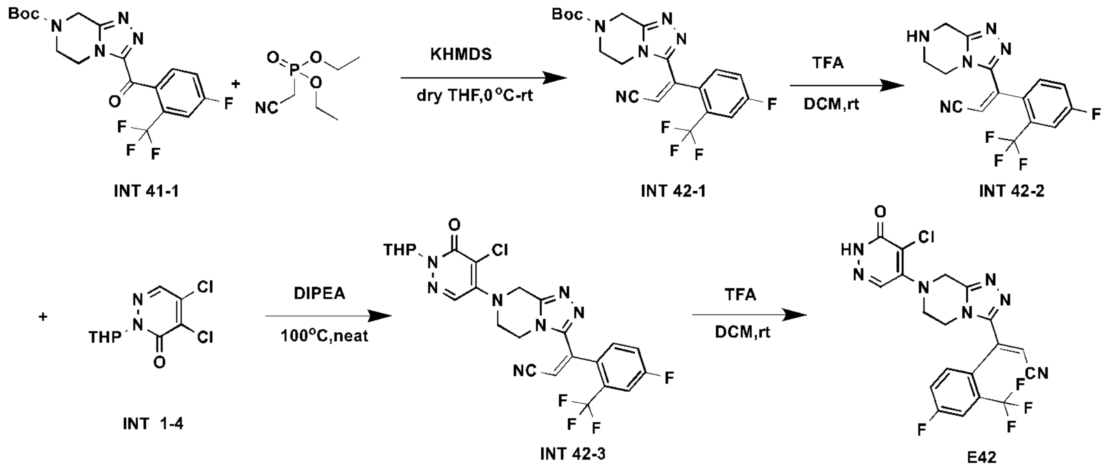


INT 41-1 INT 42-1 INT 42-2 INT 1-4 INT 42-3 E42

**42.1 Preparation of Intermediate INT 42-1**

The synthesis method of intermediate INT 42-1 was the same as that of INT 31-1, except that INT 41-1 was used as the starting material instead of INT 27-1.

**42.2 Preparation of Intermediate INT 42-2**

The synthesis method of intermediate INT 42-2 was the same as that of INT 1-3, except that INT 42-1 was used as the starting material instead of INT 1-2.

**42.3 Preparation of Intermediate INT 42-3**

The synthesis method of intermediate INT 42-3 was the same as that of INT 1-5, except that INT 42-2 was used as the starting material instead of INT 1-3.

**42.4 Preparation of Compound E42**

The synthesis method of Example E42 was the same as that of that of E1, except that the intermediate INT 42-3 was used as the starting material instead of INT 1-5. $^1$H NMR (500 MHz, DMSO-$d_6$) δ 13.13 (s, 1H), 7.96 (s, 1H), 7.92 - 7.86 (m, 1H), 7.81 - 7.72 (m, 2H), 6.82 (s, 1H), 4.96 - 4.84 (m, 2H), 4.21 - 4.12 (m, 2H), 3.92 - 3.83 (m, 1H), 3.81 - 3.73 (m, 1H).MS (ESI): m/z 465.07[M + H]$^+$.

**Example E43: 3-(7-(5-chloro-6-oxo-1,6-dihydropyridazin-4-yl)-5,6,7,8-tetrahydro-[1,2,4]triazolo[4,3-a]pyrazin-3-yl)-3-(4-fluoro-2-(trifluoromethyl)phenyl)propionitrile**

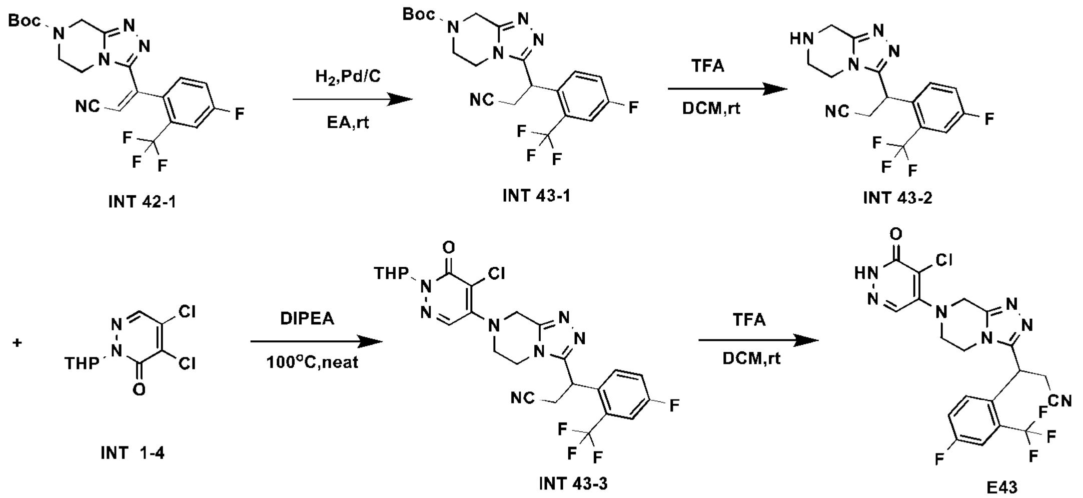


INT 42-1 INT 43-1 INT 43-2

INT 1-4 INT 43-3 E43

### 43.1 Preparation of Intermediate INT 43-1

The synthesis method of intermediate INT 43-1 was the same as that of INT 11-1, except that INT 42-1 was used as the starting material instead of INT 10-2.

### 43.2 Preparation of Intermediate INT 43-2

The synthesis method of intermediate INT 43-2 was the same as that of INT 1-3, except that INT 43-1 was used as the starting material instead of INT 1-2.

### 43.3 Preparation of Intermediate INT 43-3

The synthesis method of intermediate INT 43-3 was the same as that of INT 1-5, except that INT 43-2 was used as the starting material instead of INT 1-3.

### 43.4 Preparation of Compound E43

The synthesis method of Example E43 was the same as that of that of E1, except that the intermediate INT 43-3 was used as the starting material instead of INT 1-5. 1H NMR (500 MHz, DMSO-d6) δ 13.08 (s, 1H), 7.93 (s, 1H), 7.77 (dd, J = 9.3, 2.9 Hz, 1H), 7.61 (td, J = 8.4, 2.8 Hz, 1H), 7.45 (dd, J = 8.8, 5.3 Hz, 1H), 5.22 - 5.09 (m, 1H),4.92 (t, J = 7.0 Hz, 1H), 4.85 - 4.75 (m, 2H), 4.20 - 4.11 (m, 1H), 3.81 - 3.67 (m, 2H), 2.48 - 2.32 (m, 2H). MS (ESI): m/z 467.09[M + H]$^+$.

## Example E44: 5-(2-bromo-3-((2-chloro-5-fluorophenyl)(methoxy)methyl)-5,6-dihydroimidazo[1,2-a]pyrazin-7(8H)-yl)-4-chloropyridazin-3(2H)-one

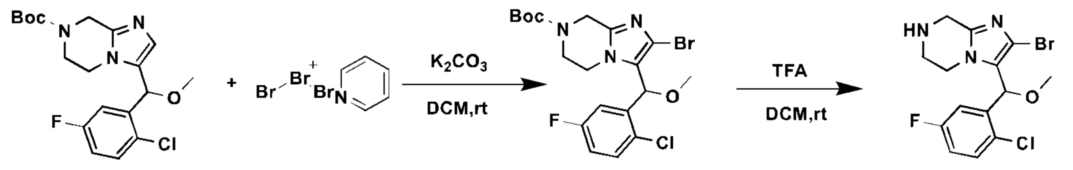


INT 22-1 INT 44-1 INT 44-2

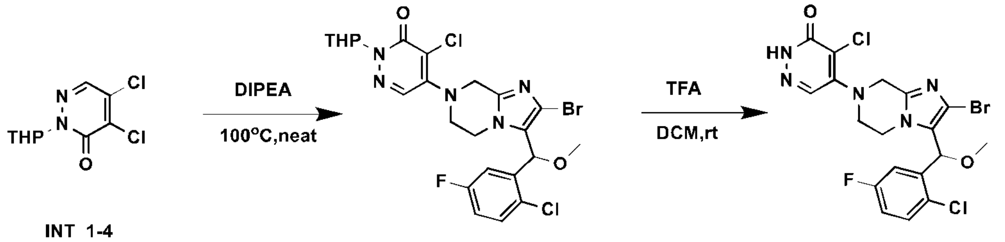


INT 1-4 INT 44-3 E44

### 44.1 Preparation of Intermediate INT 44-1

The synthesis method of intermediate INT44-1 was the same as that of INT 13-1, except that INT 20-2 was used as the starting material instead of INT 2-1.

### 44.2 Preparation of Intermediate INT 44-2

The synthesis method of intermediate INT 44-2 was the same as that of INT 1-3, except that INT 44-1 was used as the starting material instead of INT 1-2.

### 44.3 Preparation of Intermediate INT44-3

The synthesis method of intermediate INT 44-3 was the same as that of INT 1-5, except that INT 44-2 was used as the starting material instead of INT 1-3.

### 44.4 Preparation of Compound E44

The synthesis method of Example E44 was the same as that of that of E1, except that the intermediate INT 44-3 was used as the starting material instead of INT 1-5. 1H NMR (500 MHz, DMSO-d6) δ 13.06 (s, 1H), 7.93 (s, 1H), 7.57 - 7.48 (m, 2H), 7.29 (td, J = 8.4, 3.1 Hz, 1H), 5.56 (s, 1H), 4.64 (s, 2H), 4.14 (dt, J = 12.4, 4.7 Hz, 1H), 3.85 (dt, J = 13.8, 4.8 Hz, 1H), 3.75 (ddd, J = 13.7, 7.2, 3.8 Hz, 1H), 3.68 (ddd, J = 11.7, 7.2, 4.1 Hz, 1H), 3.36 (s, 3H). MS (ESI): m/z 500.98[M + H]$^+$.

### Example E45: 5-(3-((2-bromo-4-fluorophenyl)(methoxy-d3)methyl)-5,6-dihydroimidazo[1,2-a]pyrazin-7(8H)-yl)-4-chloropyridazin-3(2H)-one

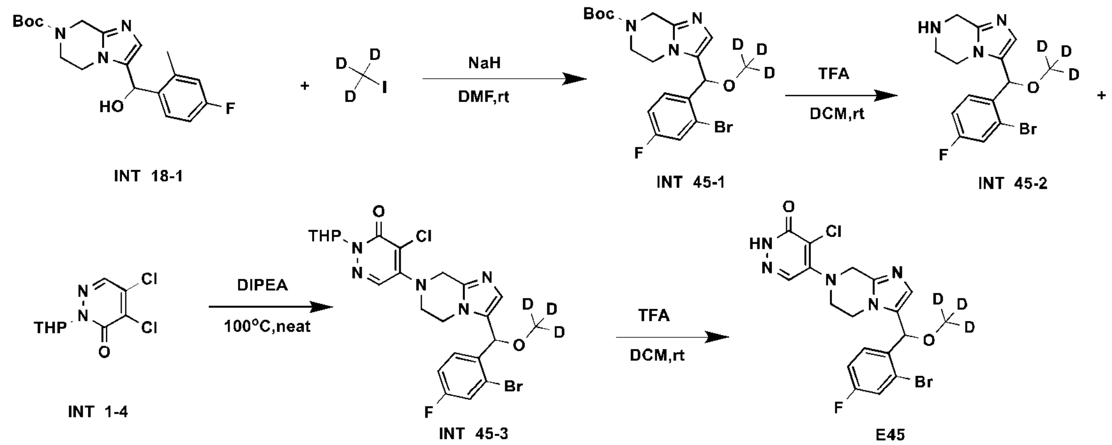


INT 18-1 INT 45-1 INT 45-2

INT 1-4 INT 45-3 E45

### 45.1 Preparation of Intermediate INT 45-1

The synthesis method of intermediate INT45-1 was the same as that of INT 12-1, except that INT 18-1 was used as the starting material instead of INT 1-1.

### 45.2 Preparation of Intermediate INT 45-2

The synthesis method of intermediate INT 45-2 was the same as that of INT 1-3, except that INT 45-1 was used as the starting material instead of INT 1-2.

### 45.3 Preparation of Intermediate INT45-3

The synthesis method of intermediate INT 45-3 was the same as that of INT 1-5, except that INT 45-2 was used as the starting material instead of INT 1-3.

### 45.4 Preparation of Compound E45

The synthesis method of Example E45 was the same as that of that of E1, except that the intermediate INT 45-3 was used as the starting material instead of INT 1-5. 1H NMR (600 MHz, DMSO-d6) δ 13.10 (s, 1H), 8.01 (s, 1H), 7.72 (dd, J = 8.4, 2.6 Hz, 1H), 7.58 (dd, J = 8.7, 6.1 Hz, 1H), 7.44 (td, J = 8.5, 2.6 Hz, 1H), 6.93 (s, 1H), 5.75 (s, 1H), 4.97 - 4.84 (m, 2H), 4.39 (dt, J = 13.5, 4.8 Hz, 1H), 4.31 - 4.23 (m, 1H), 4.02 (dt, J = 14.6, 4.9 Hz, 1H), 3.92 (ddd, J = 14.6, 7.4, 4.1 Hz, 1H).MS (ESI): m/z 470.03 $[M + H]^+$.

## II. Pharmacological Examples

### 1. TRPC4/5 inhibitory activity assay

1.1 Experimental methods

HEK-293 cells expressing hTRPC4 or hTRPC5 were seeded into polylysine (PDL)-coated, black-bottomed, transparent 96-well plates at $2 \times 10^4$ cells per well. After 8 h of culture, the culture medium was discarded and 60 μL of 4 μM Fluo-4/AM dye was added. The plates were incubated at 37°C for 60 min and then rinsed five times with calcium flux assay buffer. The plates were then placed in 30°C preheated FLIPR®TETRA (Molecular Devices, Sunnyvale, CA, USA) and excited at 488 nm. Fluorescence signals were recorded continuously in the 515-535 nm range at a sampling rate of 1 s. After recording for 60 seconds, solvent control, test compound and positive inhibitor were added, and the signal was collected for another 300 seconds. Then, the agonist Englerin A (EA) (MedChemExpress, Shanghai, China, final concentration of 0.3 nM) was added, and the fluorescence signal was collected for another 600 seconds. The fluorescence signal in the trajectory diagram was expressed as $F/F_0$, wherein F was the fluorescence signal at different time points and $F_0$ was the basic fluorescence signal, that is, the average of the fluorescence signals at the first 10 time points. The dose-effect curve is first established with $F/F_0 = 1$ as the baseline. The area under the curve of the fluorescence intensity change after the addition of EA was calculated. The $IC_{50}$ value was calculated using the log[Inhibitor] vs. response - Variable slope, combining the area under the curve and the log value of the compound concentration.

1.2 Test Results

As shown in Table 1, the compounds of the present invention can significantly inhibit the activity of TRPC5 channels, and some of the compounds have equivalent efficacy to the positive control L002 (WO2022001767) or Compound 100 (WO2020191056). As shown in Table 2, the compounds of the present invention can also effectively inhibit the activity of TRPC4 channels, and therefore are expected to be used to prepare drugs for preventing, delaying or treating diseases related to abnormal functions and expressions of TRPC4 and/or TRPC5.

**Table 1: Inhibitory activity of compounds on TRPC5 channels**

| Example | FLIPR $IC_{50}$ (nM) | Example | FLIPR $IC_{50}$ (nM) | Example | FLIPR $IC_{50}$ (nM) |
|---|---|---|---|---|---|
| E1 | 8.58 | E2 | >100 | E5 | >100 |
| E7 | >100 | E9 | 24.6 | E10 | 1.50 |
| E11 | 79.5 | E12 | 18.9 | E13 | 45.0 |
| E14 | 8.95 | E15 | 22.8 | E18 | 3.11 |
| E19 | 8.25 | E20 | 89.9 | E21 | 8.11 |
| E21-1 | 132 | E21-2 | 5.29 | E22 | 60.1 |
| E24 | 15.3 | E25 | 35.9 | E26 | 4.40 |
| E27 | 5.28 | E28 | 18.3 | E30 | 37.1 |
| E33 | 67.5 | E35 | >100 | E36 | 130 |
| E39 | 30.2 | E41 | 146 | E44 | 46.1 |
| E45 | 14.0 | L002 | 10.4 | Compound 100 | 8.74 |

**Table 2: Inhibitory activity of compounds on TRPC4 channels**

| Example | TRPC4 $IC_{50}$ (nM) |
|---|---|
| E21 | 34.7 |
| Compound 100 | 18.09 |

**2. Liver microsome stability test**

2.1 Experimental method

0.1M pH 7.4 Tris buffer was prepared and then this buffer was used to prepare a 100 mM $MgCl_2$ solution and a 10 mM coenzyme factor NADPH solution. The test compound was first prepared as a stock solution in DMSO and then diluted to a working concentration with 0.1% (w/v) BSA-PBS solution as needed.

During the assay, the liver microsome in TRIS buffer (final concentration of 0.33 mg/mL microsome protein), $MgCl_2$ solution (final concentration of 5 mM), test compound solution (final concentration of 1 μM), and NADPH solution (final concentration of 1 mM) were incubated at 37°C. The reaction was terminated by adding methanol at 0, 7, 17, 30, and 60 minutes. The residual concentration of the test compound was determined using LC/MS/MS.

Calculation of half-life:

$$T_{1/2}=0.693/k_e;$$

Calculation of intrinsic clearance:

$$Cl_{int} = \frac{1000 \times slope}{P};$$

Calculation of *in vivo* clearance:

$$Cl = \frac{Cl_{int} \times Houston \times LW}{1000};$$

Calculation of hepatic clearance:

$$Cl_{hep} = \frac{HBF \times fu \times Cl}{HBF + (fu \times Cl)};$$

Calculation of Metabolic utilization rate:

$$\%MF = 100 - \frac{Cl_{hep} \times 100}{HBF}.$$

$k_e$: Slope of the linear regression line (absolute value of slope) when plotting semi-logarithmic graph. The semi-logarithmic plot was made with the semi-logarithm of the remaining percentage of substrate versus reaction time. P: Microsome protein concentration (mg/mL); Houston: Houston factor (45 mg microsome protein/g liver); LW: Liver weight (g); HBF: Hepatic blood flow (mL/min); fu: Unbound fraction (typically fu=1).

2.2 Test Results

As shown in Table 3, compared with compound L002, the compound of the present invention has a longer metabolic half-life in liver microsomes, especially in human liver microsomes, and a higher metabolic bioavailability, indicating better metabolic stability.

**Table 3: Metabolic stability of compounds in liver microsomes**

| Example | Species | Half-life $T_{1/2}$ (min) | MF % |
|---|---|---|---|
| E18 | human | 126 | 54.0 |
| | mouse | 35.3 | 42.8 |
| E21 | human | 119 | 52.5 |
| | mouse | 46.7 | 49.7 |
| E24 | human | 189 | 63.8 |
| | mouse | 59.8 | 55.9 |
| E29 | human | 70.2 | 39.6 |
| | mouse | 56.4 | 54.4 |
| Compound L002 (WO2022001767) | human | 65.8 | 38.1 |
| | mouse | 38.7 | 45.0 |
| Note: MF% indicates metabolic bioavailability. | | | |

**3. Drug tissue distribution testing**

3.1 Experimental methods

250 mg of CMC-Na was weighted accurately, dissolved in 50 mL of mili-Q water, and sonicated for 1 h to prepare a 0.5% (m/v) CMC-Na solution for later use. 7.0 mg of compound E21 was weighted accurately and 140 μL of DMSO was added. After vortexing to dissolve, 140 μL of Tween 80 was added and mixed by vortex. Then 6.72 mL of CMC-Na solution was added and sonicated for 30 min to obtain 7 mL of a 1.0 mg/mL solution for use.

6-8 week old (18-22 g) male ICR mice (Annocon Biotech, license number: SCXK(Su)2017-0007) were housed in an SPF animal room with a light-to-dark ratio of 1:1, room temperature controlled at 23 ± 2°C, and humidity controlled at 55%. Mice were able to eat and drink freely and randomly divided into groups, with 3 mice in each group, and each mouse was gavaged with a dosage volume of 10 mL/kg. Blood was collected from the orbital venous plexus 1 and 4 hours after

administration. Approximately 500 μL of mouse blood was placed in an EDTA-K2 anticoagulant tube. At the same time points, the brain, heart, lung, liver, kidney, spleen, colon, and muscle tissues of the mice were collected. The blood samples were centrifuged at 3,000 rpm for 15 min, and the supernatant was collected to obtain plasma, which was stored at -80°C until use. Mouse brain, heart, lung, liver, kidney, spleen, colon, and muscle tissues were ground into tissue homogenate using a grinder with physiological saline equal to 5 times the weight of the tissue. The homogenate was centrifuged at 3,000 rpm for 10 min, and the supernatant was stored at -80°C for later use.

198 μL of plasma sample was placed in a centrifuge tube, 2 μl of internal standard solution was added, then 400 μL of acetonitrile was added, mixed by vortex for 1 min, and centrifuged at 13,000 rpm for 6 min. All the supernatant was taken, evaporated at room temperature for two days, re-dissolved in 200 μL of acetonitrile solvent, filtered with a 0.22 μm filter membrane and then injected for testing. 198 μL of the tissue homogenate supernatant sample was placed in a centrifuge tube, 2 μl of internal standard solution was added, then 400 μL of acetonitrile was added, mixed by vortex for 1 min, and centrifuged at 13,000 rpm for 6 min. All the supernatant was taken, evaporated at room temperature for two days, re-dissolved in 200 μL of acetonitrile solvent, filtered with a 0.22 μm filter membrane and then injected for testing.

The drug concentration was tested using an Agilent 1260 high performance liquid chromatograph/triple quadrupole tandem mass spectrometer, and the drug concentration was calculated based on the concentration standard curve of compound E21 and the molecular ion peak area of the sample. Chromatographic column: Ultimate XB-C18 3 μM 2.1*100 mm; flow rate: 0.3 mL/min; column temperature: 30 °C; injection volume: 5 μL; mobile phase: A (pure water, containing 0.1% by volume formic acid), B (acetonitrile). The mass spectrometry ion source was an electrospray ionization (ESI) ionization source with a source temperature of 300°C. The detection mode was positive and negative ion modes with a spray voltage of 1500 V. The scanning mode was multiple reaction ionization (MRM) mode, and the quantitative ion pair of compound E21 was m/z 424.1-173.1.

3.2 Test Results

As shown in Table 4, compound E21 has good drug exposure and distribution in plasma, liver, and kidney 1 and 4 hours after oral administration to mice, and is suitable for the development of a drug for liver and kidney diseases.

Table 4: Concentrations of compound E21 (10 mg/kg) in plasma and tissues after oral administration to mice

| Time | Drug concentration (Plasma: ng/mL, rests: ng/g) | | | | | | | | |
|---|---|---|---|---|---|---|---|---|---|
| | Plasma | Heart | Lung | Liver | Kidney | Spleen | Colon | Muscle | Brain |
| 1 h | 1147 | 191 | 219 | 565 | 719 | 531 | 824 | 153 | 52 |
| 4 h | 250 | 26 | 39 | 205 | 97 | 60 | 133 | 52 | 28 |

**4. Evaluation of drug efficacy in a DOCA-salt-induced hypertensive nephropathy rat model**

4.1 Experimental methods

(1) Establishment of a hypertensive nephropathy rat model

After one week of adaptive feeding, 40 healthy male SD rats (SPF grade, 6 weeks old, weighing 200 g) were randomly divided into cages and fed with normal water. Five rats were selected as the normal group. The other 35 rats were weighed and anesthetized with 6% (w/v) chloral hydrate (200 g/ml, Sinopharm Group Reagent Company) intraperitoneally. The dorsal surgical area was then trimmed and the rats were fixed in a prone position on a surgical board. After strict disinfection, a 2-cm incision was made on the left side of the spine, below the twelfth rib. The muscles were separated, the renal capsule was freed, and the left kidney was exposed. The renal artery and vein were ligated close to the renal hilum, and the left kidney was removed. After clearing congestion, the muscle layer and skin were sutured. After surgery (after waking up from anesthesia), the rats were given an intraperitoneal injection of penicillin sodium (80,000 units intraperitoneal injection, for 3 consecutive days, prepared with normal saline. If 400,000 units of powder were used with 2.5 mL of normal saline, and 0.5 mL was injected per mouse per day). Afterwards, the normal group was given a subcutaneous injection of vegetable oil (commonly corn oil) and fed with tap water; the DOCA-salt modeling group was given a subcutaneous injection of deoxycorticosterone acetate (DOCA, CAS: 56-47-3, Shanghai Aladdin Biochemical Technology) dissolved in vegetable oil once a week, 30 mg/kg each time, and 10 g/L NaCl and 2 g/L KCl were added to the drinking water. The model was established after 4 consecutive weeks of induction.

DOCA-salt hypertensive nephropathy modeling criteria: ① After 4 weeks of modeling, if the systolic blood pressure of the rat is more than 160 mmHg and there is no necrosis, atrophy or fibrosis in the left kidney after dissection, the modeling is successful; ② For 24-hour urine collected, the modeling group shows an increased urine albumin/creatinine

ratio (UACR) compared with the normal group.

(2) Drug preparation and administration

Four weeks after modeling, the DOCA-salt hypertensive nephropathy rats were divided into four groups according to UACR. The drug was dissolved in 2% (v/v) DMSO + 2% (w/v) Tween 80 + physiological saline system and administered by gavage. The groups and dosing were as follows: ① DOCA-salt model group: 10 rats, given 2% DMSO + 2% Tween + normal saline every day; ② DOCA-salt + losartan group: rats, given losartan (Hangzhou Merck Pharmaceutical) as a positive control every morning, at a dose of 30 mg/kg, once a day; ③ DOCA-salt + E21-H group: 8 rats, given compound E21, 10 mg/kg in the morning and evening; ④ DOCA-salt + E21-M group: 8 rats, given compound E21, 5 mg/kg in the morning and evening. Normal group: 5 rats, raised normally. Each group had free access to food and water.

(3) Sampling and testing

After the start of drug administration, rats were placed in metabolic cages to collect 24 hours urine per week. According to the instructions of the kit (Nanjing Jiancheng Biological), the concentrations of urine creatinine, urine albumin, and urine total protein in urine were detected, and the urine albumin/creatinine ratio (UACR) and the total 24-h urine protein were calculated. Three weeks after administration, serum was collected to check the concentrations of blood creatinine and urea nitrogen. Then, the rats were euthanized under anesthesia, and the renal tissues were obtained. The RNA levels of nephrin and podocin were detected by quantitative RT-PCR. The primer sequences were shown in Table 6:

Table 5: Primer sequences used for PCR

| Target gene | Forward primer | Reverse primer |
|---|---|---|
| rGAPDH | TGATTCTACCCACGGCAAGTT | TGATGGGTTTCCCATTGATGA |
| rNephrin | CGGAGAACAAGAACGTGACC | ATTGTCTTCTCTCCGCACCA |
| rPodocin | GTGGCTTCTTGTCCTCTCCT | TGTGATAGGTGTCCAGGCAG |

(4) Statistical analysis

GraphPad Prism (Ver 6.0) was used for statistical analysis. All data were expressed as mean ± standard error (mean ± SEM). One-way ANOVA was used to analyze the data. Dunnett's multiple test was used to analyze the significance of the differences between the groups. When $P < 0.05$, the differences between the groups were statistically significant.

4.2 Experimental Results

(1) Effects of compound E21 on UACR and 24h urine protein in rats

As shown in Figures 1 and 2, after uninephrectomy and DOCA-salt feeding for 4 weeks, the UACR and 24-h urine protein of rats increase significantly, indicating the formation of nephropathy. Oral treatment with compound E21 for 3 weeks (weeks 5-7) significantly and dose-dependently reduces UACR and 24-h urine protein, and its effect is comparable to that of the positive control losartan.

(2) Effects of compound E21 on serum creatinine (Scr) and urea nitrogen (BUN) in rats

As shown in Figure 3, the serum creatinine and urea nitrogen levels in the uninephrectomized nephrotic rats fed with DOCA-salt are significantly increased. Oral treatment with the positive control losartan and a medium dose of compound E21 for 3 weeks effectively reduce serum creatinine but have no effect on urea nitrogen.

(3) Effect of compound E21 on rat kidney slit diaphragm protein

As shown in Figure 4, the levels of nephrin and podocin (slit diaphragm protein) in the kidneys of uninephrectomized nephrotic rats fed with DOCA-salt are significantly decreased, indicating glomerular podocyte damage. Oral treatment with the positive control losartan and high-dose compound E21 for 3 weeks effectively increase the levels of nephrin and podocin, indicating a protective effect on podocytes.

In summary, uninephrectomy plus DOCA-salt feeding successfully induce a hypertensive nephropathy model. Compound E21 administration significantly reduces urine albumin and total urine protein levels, suppresses serum creatinine, and increases podocyte slit diaphragm protein gene levels, indicating that the compound of the present invention has good potential for treating kidney diseases.

**5. Evaluation of drug efficacy in a bile duct ligation (BDL) mouse model**

5.1 Experimental methods

(1) BDL modeling

Before surgery, mice were anesthetized by intraperitoneal injection of 0.2 mL/10 g tribromoethanol solution. The hair on the chest and abdomen of the mice was shaved clean, and the limbs were fixed on cardboard with tape and placed on an electric blanket. Iodine was applied to the chest and abdomen by a cotton swab, the outer and inner skin were cut in turn from the abdomen upwards to near the xiphoid process. The smaller the opening, the better, and the maximum should not exceed 2 cm. The liver and gastrointestinal tract were gently pushed upwards and downwards by using two cotton swabs moistened with saline and the parallel blood vessels were carefully separated. Two ligature threads were passed beneath the common bile duct and tied into two knots, and the common bile duct was cut between the two knots. The liver and gastrointestinal tract were returned to their original positions, the inner and outer skin were sutured in turn (a purse-string suture method, tie at least two straight knots and tie them as tightly as possible). Iodine was applied to the sutures. The mouse was placed on its back in a cage lined with clean bedding near a heating device, waiting for regaining consciousness. Povidone-iodine was applied to the wound site once daily for three days before modeling

(2) Drug preparation and administration

Compound E21 was first dissolved in DMSO to prepare a stock solution, and then physiological saline containing 2% (w/v) Tween 80 was used as the solvent. The stock solution was added to the solvent and mixed to make the DMSO concentration be 1% (v/v). The solvent control was physiological saline containing only 2% Tween 80 and 1% DMSO. Starting from the second day after surgery, the solvent control or compound E21 was administered twice a day at a dose of 2.5 mg/kg each time, for a total of 5 mg/kg/d, by oral gavage at 10 mL/kg.

(3) Sampling

After modeling, the eyeballs were removed and blood was collected (no anticoagulant was added, the blood was placed at room temperature for 30 minutes, then centrifuged at 3,000 rpm for 15 minutes, and then stored below -20°C) to detect serum biochemical indicators. After cardiac perfusion, the liver was removed and cut into pieces, fixed in 4% (w/v) paraformaldehyde, and embedded in paraffin. Another part of the tissue was quickly frozen in liquid nitrogen and transferred to a -80°C refrigerator overnight for RNA extraction and detection of other indicators.

(4) Serum ALT and AST level detection

The detection kit (Nanjing Jiancheng Biotechnology) employed the Riedel method and microplate method. The experiment was performed according to the instructions of the kit. After the reaction was completed, the absorbance value at 505 nM was detected with a microplate reader, and the enzyme activity level was converted according to the standard curve given in the instructions.

(5) Preparation and staining of pathological sections

1) Preparation of paraffin sections: Tissue blocks were placed in pre-prepared 4% (w/v) paraformaldehyde fixative and left at room temperature for 2 days. Then they were trimmed, placed in alcohol from low concentration to high concentration for dehydration, and then placed in the clearing agent xylene for clearing. The cleared tissue blocks were embedded in paraffin. The embedded wax block was fixed on a microtome and cut into 5-8 μm thin slices, which were flattened in heated water, then attached to a glass slide and dried in a 45 °C constant temperature box.
2) HE staining: Before staining, the paraffin in the slices was removed with xylene, then the slices are washed with alcohol from high concentration to low concentration, and finally immersed with distilled water before staining. Hematoxylin (H) and eosin (Eosin, E) dyes were used for HE staining. The sections that have been immersed in

distilled water were placed in the hematoxylin aqueous solution for staining for several minutes, then separated in acid water and ammonia water. After washing with tap water and distilled water, they were dehydrated in 70% (v/v) and 90% (v/v) alcohol for 10 minutes each. After they were stained in alcohol eosin staining solution for 2-3 minutes, the above dehydration and transparent steps were repeated, and finally the sections were mounted with gum.

3) Masson staining: Paraffin sections were first dewaxed and washed sequentially with tap water and then distilled water. The nuclei were stained with Regaud's hematoxylin solution for 5-10 minutes. After thorough washing, the sections were stained with Masson's Ponceau acid fuchsin solution for 5-10 minutes. The sections were then briefly immersed in 2% (v/v) glacial acetic acid aqueous solution and differentiated in 1% (w/v) phosphomolybdic acid solution for 3-5 minutes. The sections were then directly stained with aniline blue or light green solution for 5 minutes. The sections were briefly immersed in 0.2% (v/v) glacial acetic acid aqueous solution. Finally, the sections were dehydrated, cleared, and mounted.

4) Sirius red staining: Paraffin sections were first dewaxed and washed with tap water and then distilled water. The sections were treated with Sirius red staining solution for 1 hour, rinsed with running water and briefly immersed in 0.5% acetic acid, and finally dehydrated, cleared, and mounted.

(6) q-RT PCR experiments

The mRNA extracted by the Trizol method was dissolved in NEPC water, and 1 μL was taken for RNA quantification using Nano-100. 1 μg of mRNAwas taken out to construct a 20 μL reverse transcription system, and the reverse transcription program was set to 55°C for 15 min-95°C for 5 s. Finally, 20 μL of cDNA stock solution was obtained for each sample. According to the instructions of ChamQ SYBR qPCR Master Mix (Low ROX Premixed, Nanjing Novozymes), a 20 μL q-RT PCR system was constructed in a 96-well PCR plate. The plate was sealed tightly with a sealing film, and amplification was performed on a Real-Time qPCR instrument according to the program set in the instructions: full pre-denaturation at 94-95 °C for 3 min, denaturation at 94-95 °C for 15 s, annealing and extension at 60 °C for 30 s, and 40 cycles. The primer sequences used in this experiment were shown in Table 6.

Table 6: Related primer sequences

| | |
|---|---|
| *Gapdh*-F | 5'-CATCTTCCAGGAGCGAGACC-3' |
| *Gapdh*-R | 5'-GAAGGGGCGGAGATGATGAC-3' |
| *Acta2*-F | 5'-GTTCAGTGGTGCCTCTGTCA-3' |
| *Acta2*-R | 5'-ACTGGGACGACATGGAAAAG-3' |
| *Colla1*-F | 5'-AGCACGTCTGGTTTGGAGAG-3' |
| *Colla1*-R | 5'-ACATTAGGCGCAGGAAGGTC-3' |
| *Timpl-F* | 5'-ACTCGGACCTGGTCATAAGGGC-3' |
| *Timpl-R* | 5'-TTCCGTGGCAGGCAAGCAAAGT-3' |
| *Pdgfr*-F | 5'-TTGCCTTACGACTCCACCTG-3' |
| *Pdgfr*-R | 5'-TAGATGGGCCCTCCTTTGGT-3' |

(7) Data processing

Pathological sections were scanned with a slide scanner (Hamamatsu NanoZoomer 2.0 RS). Collagen areas were counted for Masson and Sirius red staining at a 10x field of view. The color threshold function of ImageJ software was used to count the blue and red areas. q-RT-PCR results were analyzed using the $2^{-\triangle\triangle}$Ct method. The Ct value of each group was used to calculate the difference between the Ct of the target primer and the internal reference. The difference between the gene and its control group was then calculated to obtain the ΔΔCt. GraphPad (Version 6.01) software was used for data processing and analysis, and histograms of enzyme activity levels, collagen area, and $2^{-\triangle\triangle}$Ct values were prepared.

5.2 Experimental Results

(1) Effect of compound E21 on serum ALT and AST in BDL mice

As shown in Figure 5, serum ALT and AST levels in the BDL modeling group soar, indicating the formation of liver

damage. E21 treatment significantly reduces ALT and AST levels, indicating a protective effect on the liver.

(2) Effect of compound E21 on liver histopathology in BDL mice

As can be seen from the HE-stained sections in Figure 6, collagen deposition increases in the liver of BDL model mice, inflammatory cell infiltration and aggregation are obvious, and more bridging necrosis occurs. However, the collagen deposition, inflammatory cell aggregation and bridging necrosis in the liver sections of mice treated with compound E21 are significantly reduced.

From the Masson-stained sections and collagen-positive area statistics in Figure 7, it can be seen that the positive area in the liver sections of BDL model mice increases significantly, suggesting the formation of fibrosis. E21 treatment can significantly reduce the collagen-positive area, indicating that it can effectively inhibit the formation of liver fibrosis.

It can also be seen from the Sirius red stained sections and collagen positive area statistics in Figure 8 that the positive area in the liver sections of BDL model mice increases significantly, while E21 treatment can significantly reduce the collagen positive area, indicating that it can effectively inhibit the formation of liver fibrosis.

(3) Effects of compound E21 on liver fibrosis markers in BDL mouse liver tissue

Liver fibrosis is a repair response to chronic liver damage. Its essence is a pathological disease caused by an imbalance in the synthesis and degradation of the extracellular matrix, mainly type I collagen, and excessive deposition of collagen in the liver. ACTA2 ($\alpha$-SMA), COL1a1, TIMP-1, and PDGFR are considered to be key cytokines driving the formation of liver fibrosis and are also commonly used biomarkers of liver fibrosis. As can be seen from Figure 9, the mRNA levels of these four markers in the liver tissue of BDL model mice are greatly or significantly increased, indicating liver fibrosis. E21 treatment can significantly reduce the mRNA levels of these four markers, indicating that it can effectively inhibit the formation of liver fibrosis.

In summary, BDL mice are a classic model of liver fibrosis, which induces cholestasis through bile duct ligation, thereby inducing liver damage and the formation of liver fibrosis. Administration of compound E21 can significantly reduce biochemical indicators of liver damage in BDL model mice, improve liver tissue pathology, and downregulate the levels of liver fibrosis markers, indicating that the compound of the present invention has good potential for treating cholestatic liver disease and liver fibrosis.

## Claims

1. A compound represented by general formula (I), a stereoisomer, deuterated derivative and pharmaceutically acceptable salt thereof;

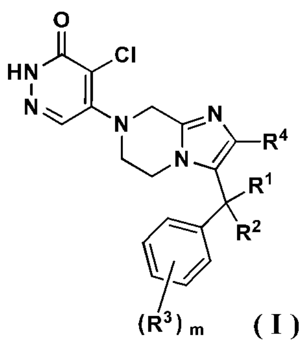

(I)

wherein,

$R^1$ and $R^2$ are each independently H, C1-C6 alkyl, C1-C6 alkoxy, azido, or hydroxyl; or $R^1$ and $R^2$ together with a carbon atom to which they are attached form

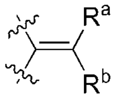,

wherein the C1-C6 alkyl or C1-C6 alkoxy is unsubstituted or further substituted with one or more substituents selected from the group consisting of C3-C8 cycloalkyl, C6-C10 aryl, C6-C10 haloaryl, deuterium atom, or halogen; $R^a$ and $R^b$ are each independently H, C1-C6 alkyl, or cyano;
the premise is that $R^1$ and $R^2$ are not H at the same time;
m is an integer from 1 to 4, and each $R^3$ is independently H, halogen, C1-C6 alkyl, C1-C6 haloalkyl, C1-C6 alkoxy, C1-C6 haloalkoxy, or nitro;
$R^4$ is H, halogen, C1-C6 alkyl, or C1-C6 haloalkyl.

**2.** The compound according to claim 1, wherein $R^1$ and $R^2$ are each independently H, C1-C3 alkyl, or C1-C3 alkoxy, wherein the C1-C3 alkoxy is unsubstituted or further substituted with a deuterium atom;

the premise is that $R^1$ and $R^2$ are not H at the same time;
or $R^1$ and $R^2$ together constitute $=CH_2$.

**3.** The compound according to claim 1, wherein each $R^3$ is independently H, halogen, C1-C3 alkyl, C1-C3 haloalkyl, or nitro.

**4.** The compound according to claim 1, wherein $R^4$ is H.

**5.** The compound according to claim 1, wherein the compound has formula (I-a):

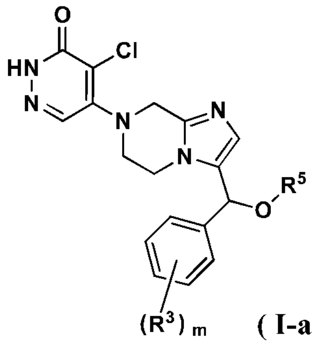

( I-a )

wherein,

m is an integer from 1 to 3;
each $R^3$ is independently H, halogen, C1-C3 alkyl, C1-C3 haloalkyl, or nitro;
$R^5$ is C1-C3 alkyl or C1-C3 deuterated alkyl.

**6.** The compound according to claim 1, wherein the compound is selected from:

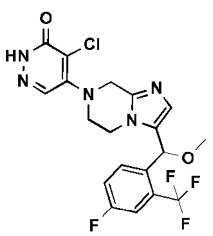
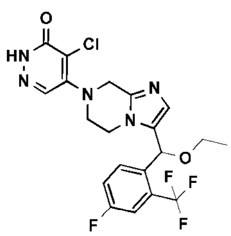
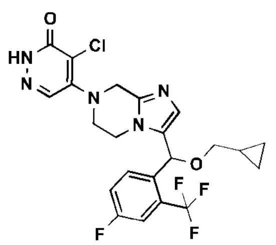
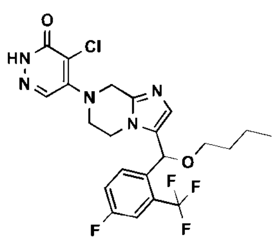
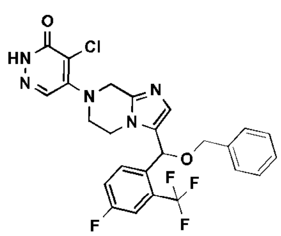
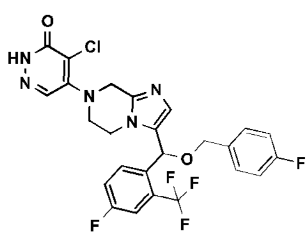
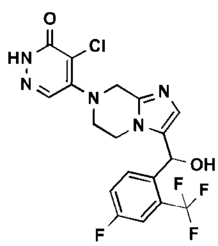
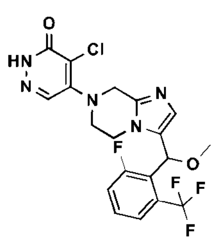

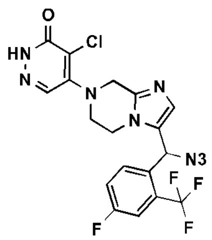 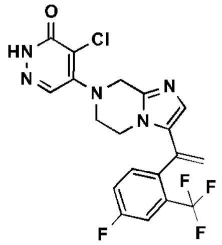 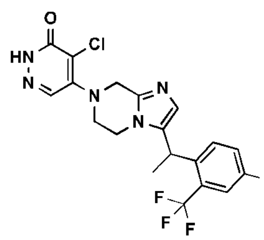 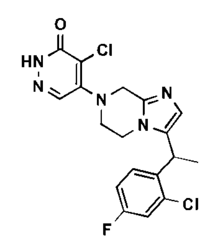 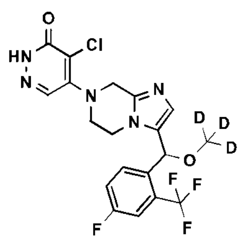

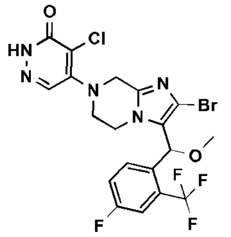 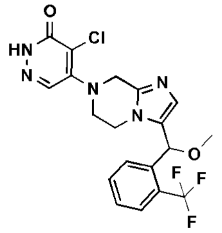 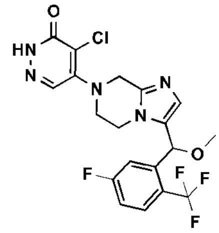 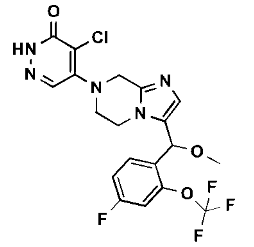 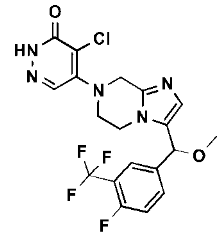

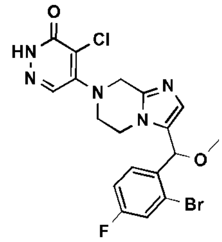 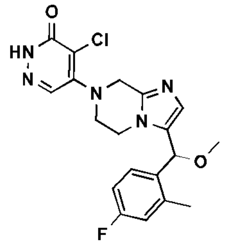 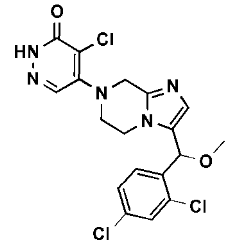 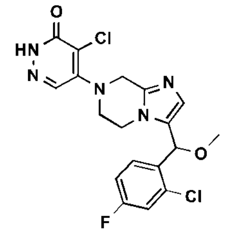 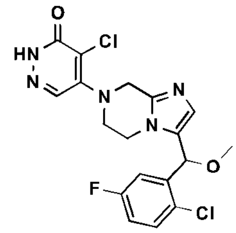

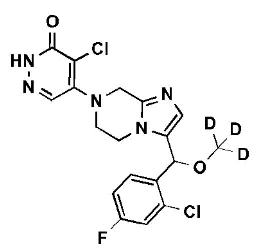 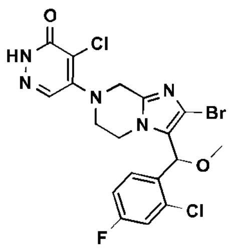 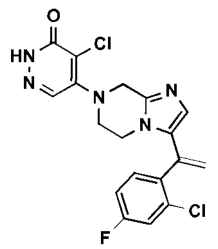 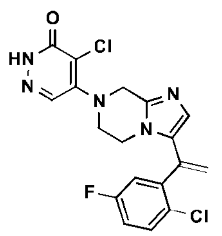

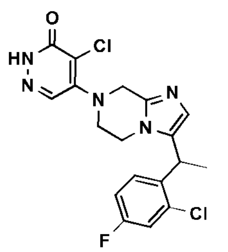 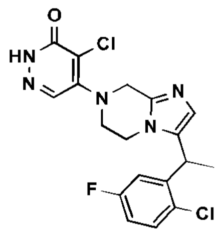 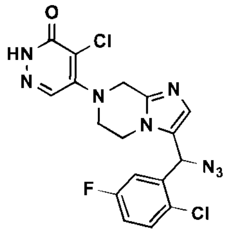 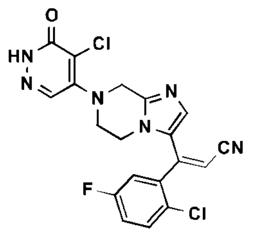

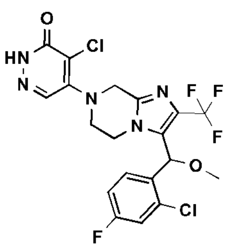 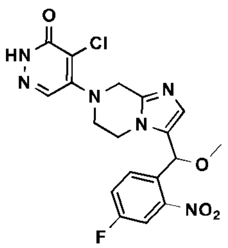 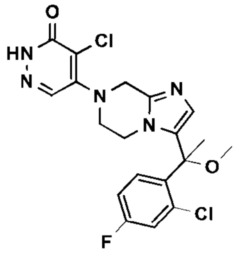 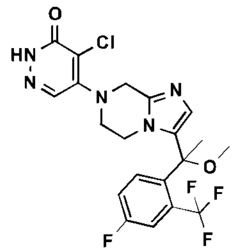

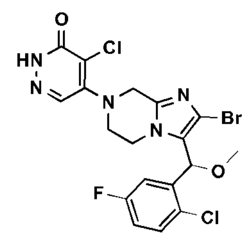 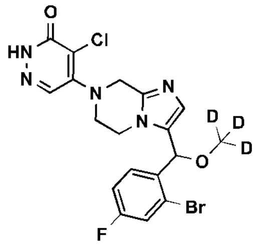 or 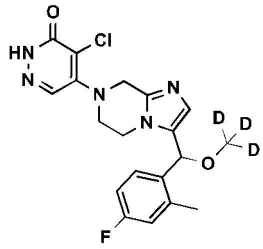 .

**7.** A pharmaceutical composition comprising:

the compound according to any one of claims 1 to 6, the stereoisomer, deuterated derivative and pharmaceutically acceptable salt thereof, and
a pharmaceutically acceptable excipient.

**8.** Use of the compound according to claims 1 to 6 and the pharmaceutical composition according to claim 7 for the manufacture of an inhibitor of TRPC4 and/or TRPC5 channels, or for the manufacture of a medicament for preventing, delaying or treating a disease related to abnormal function or expression of TRPC4 and/or TRPC5.

**9.** The use according to claim 8, wherein the disease is selected from the group consisting of epilepsy, mental illness, neurodegenerative disease, kidney disease, pain, cardiovascular disease, cancer, skin disease, and liver disease.

**10.** The use according to claim 9, wherein the mental illness is selected from the group consisting of borderline personality disorder, depression, dysthymia, postpartum depression, bipolar disorder, post-traumatic stress disorder, panic disorder, agoraphobia, social phobia, generalized anxiety disorder, social anxiety disorder, separation anxiety, schizophrenia, mania, obsessive-compulsive disorder, apathy, neurasthenia, and paranoia;

the neurodegenerative disease is selected from the group consisting of Alzheimer's disease, Parkinson's disease, Huntington's disease, amyotrophic lateral sclerosis, memory impairment, amnesia, aphasia, chronic fatigue syndrome, Creutzfeldt-Jakob disease, dissociative amnesia, fugue amnesia, learning disorder, sleep disorder, and other brain disorders caused by trauma or aging;
the kidney disease is selected from the group consisting of kidney damage caused by toxic substance, kidney damage caused by viral or bacterial infection, hypertensive nephropathy, diabetic nephropathy, glomerulonephritis, lupus erythematosus nephritis, IgA nephropathy, nephrotic syndrome, membranous nephropathy, minimal change nephropathy, polycystic kidney disease, focal segmental glomerulosclerosis; preferably, the kidney disease is focal segmental glomerulosclerosis.
the pain is selected from the group consisting of nociceptive pain, mechanical pain, inflammatory pain, cancer pain and neuropathic pain;
the cardiovascular disease is selected from the group consisting of hypertension, atherosclerosis, coronary heart disease, angina pectoris, myocardial infarction, arrhythmia, stroke, and pulmonary hypertension;
the cancer is selected from the group consisting of renal cell carcinoma, breast cancer, colorectal cancer, and hepatocellular carcinoma;
the skin disease is selected from the group consisting of psoriasis, ichthyosis, palmoplantar keratoderma, Olmsted's disease, toad skin disease, and menopausal keratoderma;
the liver disease is selected from the group consisting of cholestatic liver disease, alcoholic steatohepatitis, nonalcoholic steatohepatitis, viral hepatitis, autoimmune liver disease, chemical liver injury, liver fibrosis, and cirrhosis; preferably, the liver disease is cholestatic liver disease.

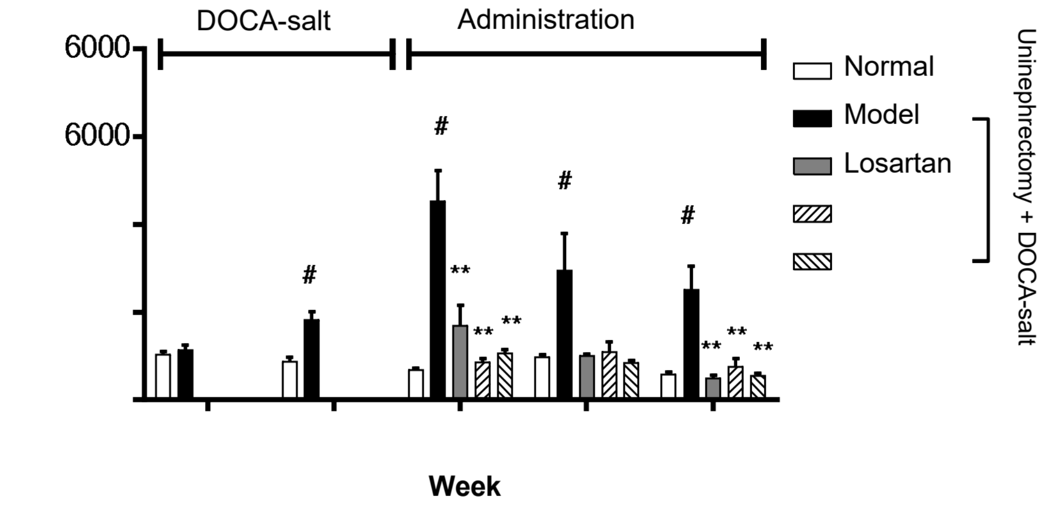


**Figure 1**

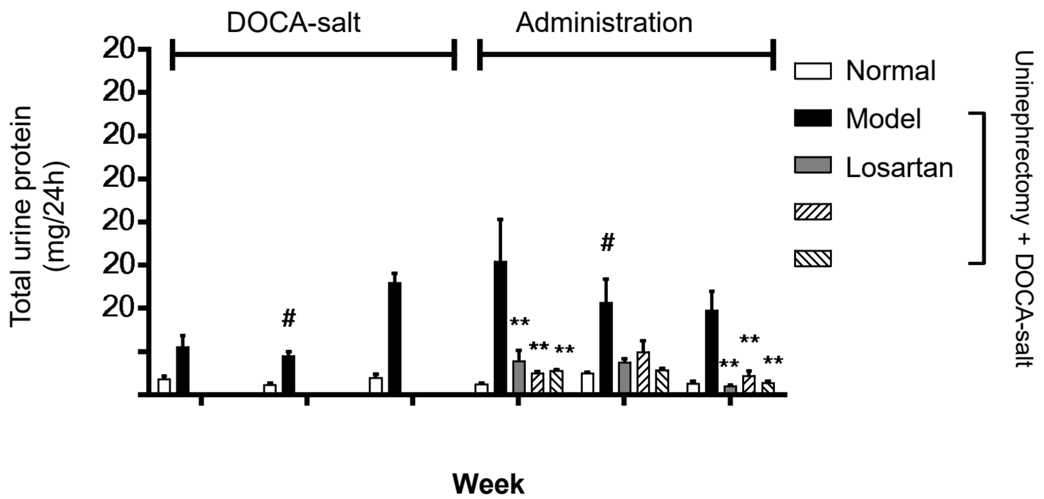


**Figure 2**

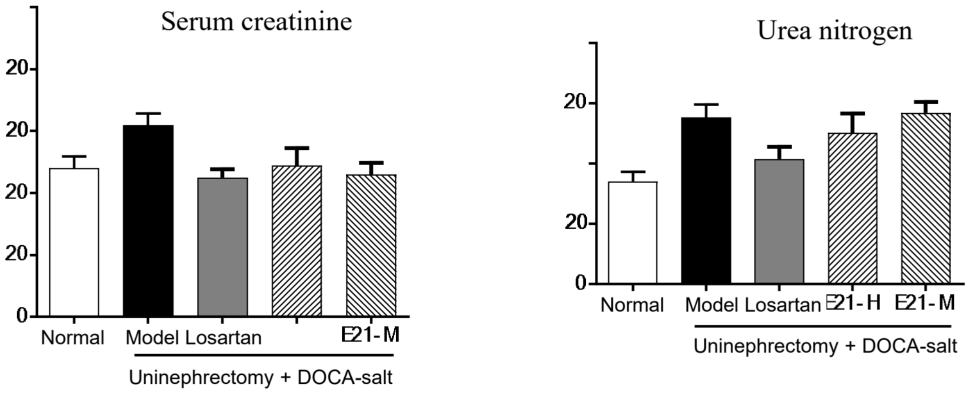


**Figure 3**

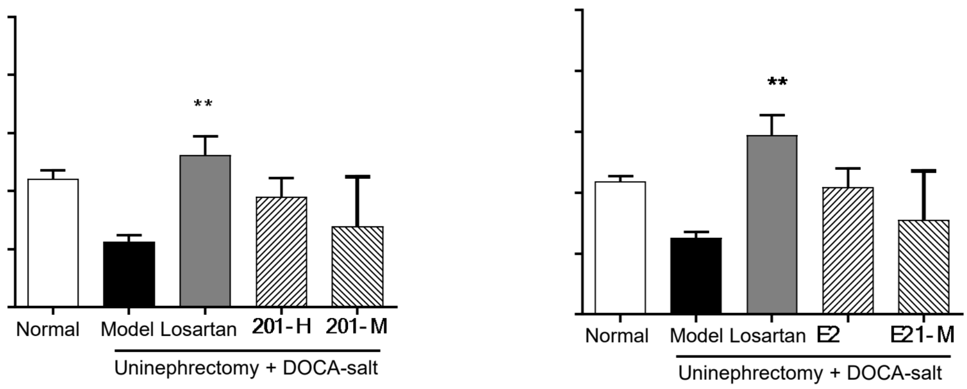


**Figure 4**

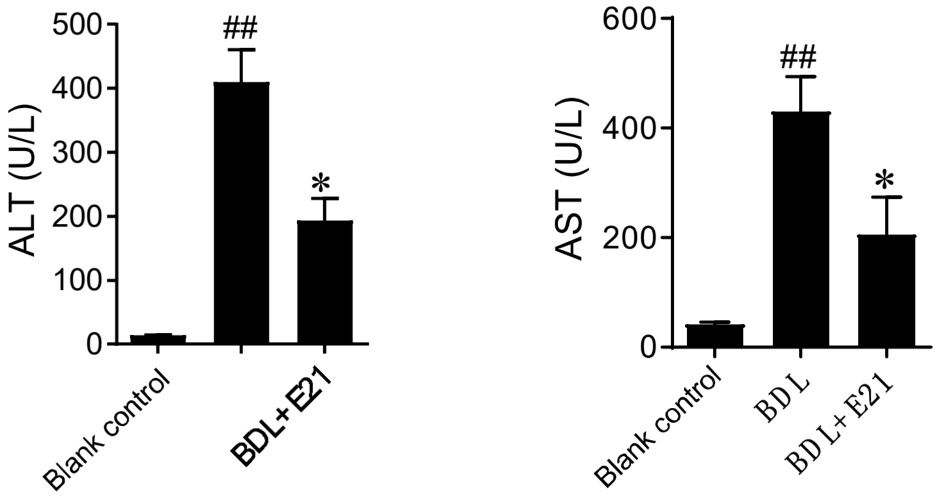


**Figure 5**

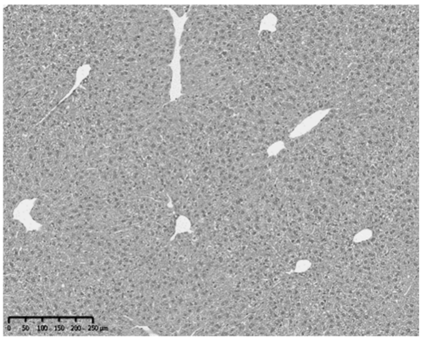

Blank control

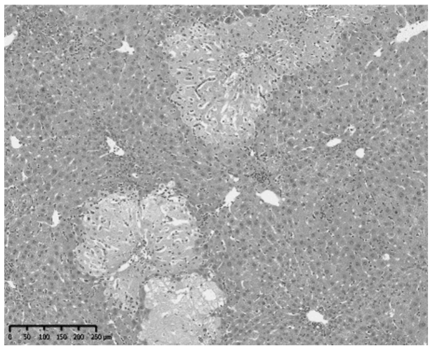

BDL model

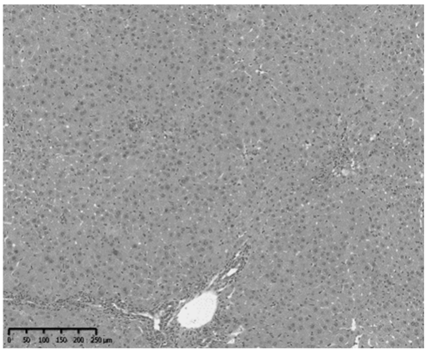

**BDL+E21**

**Figure 6**

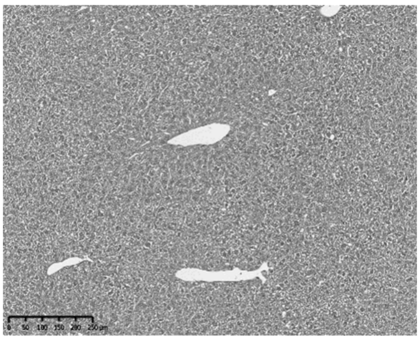

Blank control

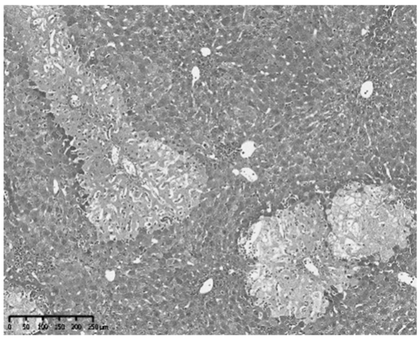

BDL model

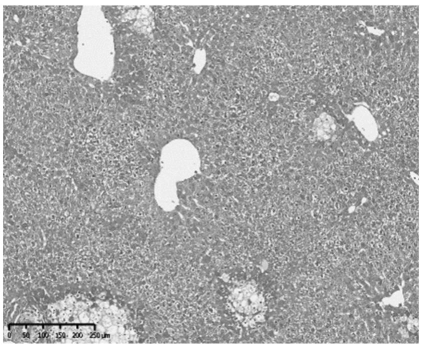

**BDL+E21**

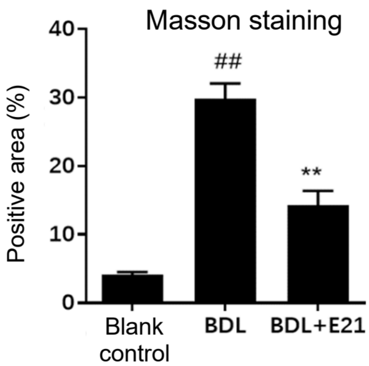


**Figure 7**

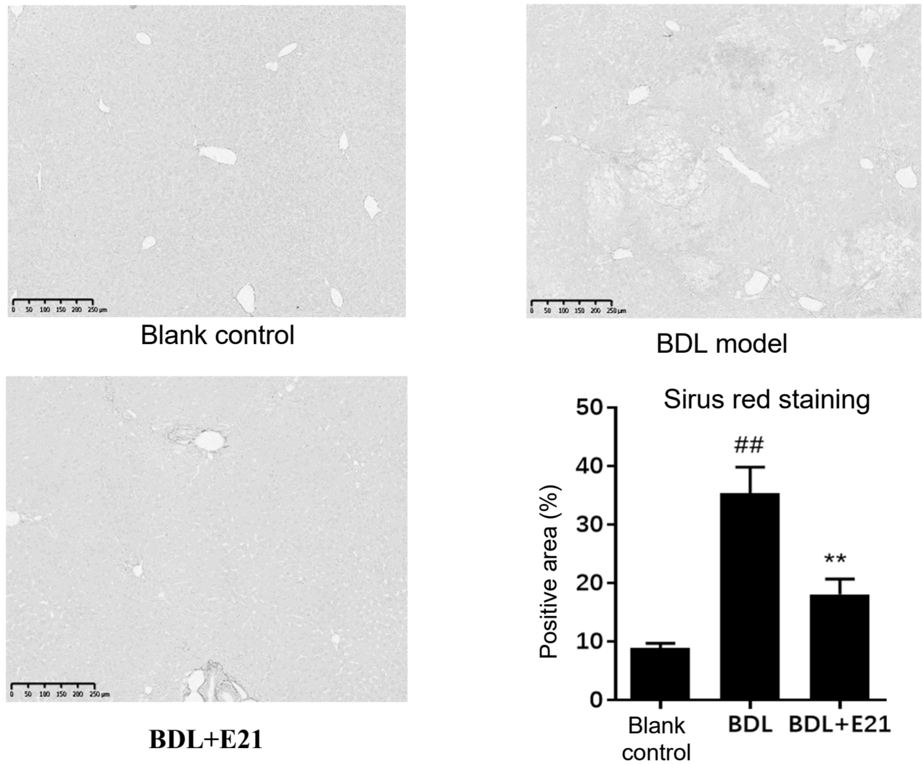


Figure 8

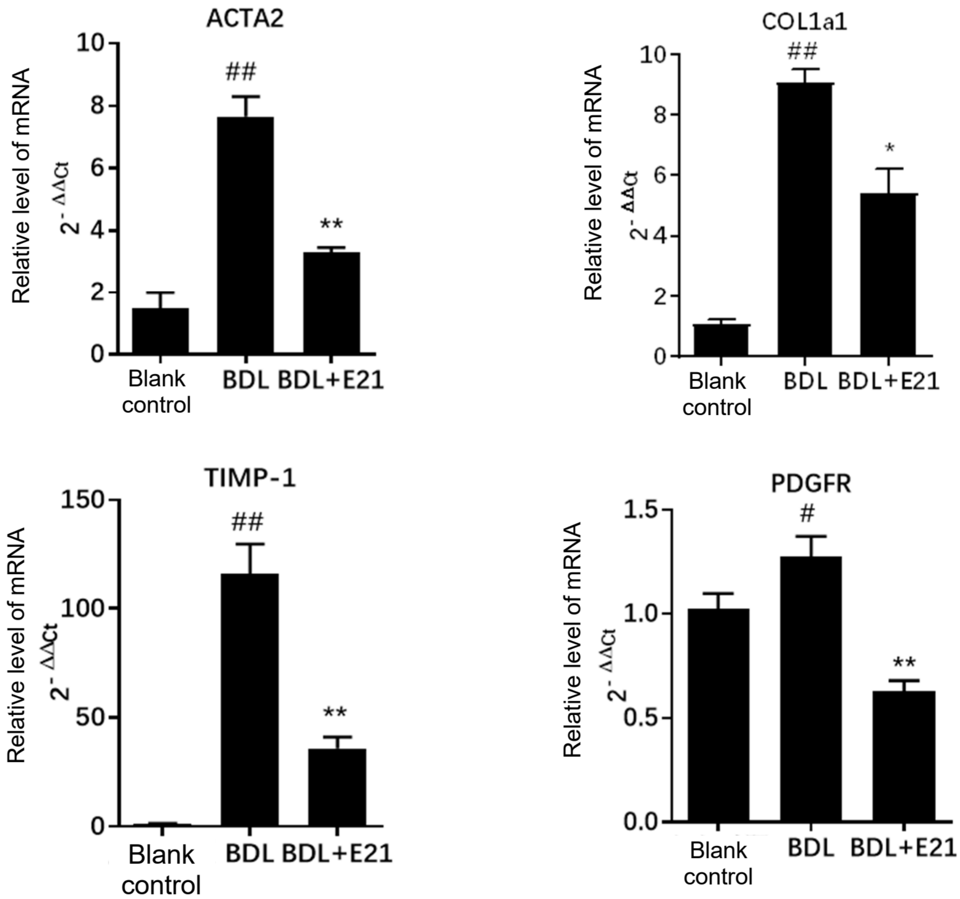


Figure 9

**INTERNATIONAL SEARCH REPORT**

International application No.
**PCT/CN2024/084776**

**A. CLASSIFICATION OF SUBJECT MATTER**

C07D487/04(2006.01)i; A61K31/4985(2006.01)i; A61P13/12(2006.01)i; A61P25/18(2006.01)i; A61P25/28(2006.01)i; A61P1/16(2006.01)i

According to International Patent Classification (IPC) or to both national classification and IPC

**B. FIELDS SEARCHED**

Minimum documentation searched (classification system followed by classification symbols)

IPC: C07D487/-; A61K31/-; A61P13/-; A61P1/-; A61P25/-

Documentation searched other than minimum documentation to the extent that such documents are included in the fields searched

Electronic data base consulted during the international search (name of data base and, where practicable, search terms used)

CNABS: CNTXT; VEN; WOTXT; USTXT; EPTXT; GBTXT; CATXT; CNKI; 万方: STN: 瞬时受体, TRPC, 哒嗪, Pyridazine, 哒嗪酮, Pyridazinone, 中国药科大学, 中国科学院上海药物研究所, 沈建华, 曹征宇, 王凯, 刘梦茹, 徐嫄嫄, 牛博, 许惕非, 赵芳, 胡一信, 任尤楠, 式(I)化合物, 式(I-a)化合物

**C. DOCUMENTS CONSIDERED TO BE RELEVANT**

| Category* | Citation of document, with indication, where appropriate, of the relevant passages | Relevant to claim No. |
| --- | --- | --- |
| PX | CN 116731013 A (SHENZHEN SALUBRIS PHARMACEUTICALS CO., LTD.) 12 September 2023 (2023-09-12)<br>description, paragraph [0058], structure formula 8, and paragraphs [0060], [0061], [0068], and [0069]-[0070] | 1, 3, 4, 6, 7, 8, 9 |
| A | CN 113880843 A (WUHAN LANGLAI TECHNOLOGY DEVELOPMENT CO., LTD.) 04 January 2022 (2022-01-04)<br>claim 14 | 1-10 |
| A | CN 114907408 A (WUHAN LANGLAI TECHNOLOGY DEVELOPMENT CO., LTD.) 16 August 2022 (2022-08-16)<br>entire document | 1-10 |
| A | US 2017073353 A1 (ABBVIE INC. et al.) 16 March 2017 (2017-03-16)<br>entire document | 1-10 |
| A | WO 2019055966 A2 (GOLDFINCH BIO INC.) 21 March 2019 (2019-03-21)<br>entire document | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **30 August 2024** | **30 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/084776**

**C. DOCUMENTS CONSIDERED TO BE RELEVANT**

| Category* | Citation of document, with indication, where appropriate, of the relevant passages | Relevant to claim No. |
|---|---|---|
| A | WO 2020191056 A1 (GOLDFINCH BIO INC.) 24 September 2020 (2020-09-24) entire document | 1-10 |
| A | WO 2023036312 A1 (MEDSHINE DISCOVERY INC.) 16 March 2023 (2023-03-16) entire document | 1-10 |

Form PCT/ISA/210 (second sheet) (July 2022)

**INTERNATIONAL SEARCH REPORT**

International application No. **PCT/CN2024/084776**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/084776**

| Patent document cited in search report | Publication date (day/month/year) | Patent family member(s) | Publication date (day/month/year) |
|---|---|---|---|
| CN 116731013 A | 12 September 2023 | None | |
| CN 113880843 A | 04 January 2022 | IL 299623 A | 01 March 2023 |
| | | JP 2023532577 A | 28 July 2023 |
| | | US 2023257382 A1 | 17 August 2023 |
| | | MX 2023000222 A | 13 April 2023 |
| | | AU 2021302090 A1 | 02 March 2023 |
| | | AU 2021302090 B2 | 18 January 2024 |
| | | CA 3184594 A1 | 06 January 2022 |
| | | WO 2022001767 A1 | 06 January 2022 |
| | | ZA 202300779 B | 25 October 2023 |
| | | EP 4174069 A1 | 03 May 2023 |
| | | TW 202202505 A | 16 January 2022 |
| | | KR 20230028522 A | 28 February 2023 |
| CN 114907408 A | 16 August 2022 | EP 4289846 A1 | 13 December 2023 |
| | | WO 2022166817 A1 | 11 August 2022 |
| | | WO 2022166817 A8 | 10 August 2023 |
| | | AU 2022216707 A1 | 14 September 2023 |
| | | TW 202233627 A | 01 September 2022 |
| | | TWI 824403 B | 01 December 2023 |
| US 2017073353 A1 | 16 March 2017 | US 2019367531 A1 | 05 December 2019 |
| | | US 10556914 B2 | 11 February 2020 |
| | | WO 2017046117 A1 | 23 March 2017 |
| | | EP 3350187 A1 | 25 July 2018 |
| WO 2019055966 A2 | 21 March 2019 | AU 2018334290 A1 | 02 April 2020 |
| | | EP 3684364 A2 | 29 July 2020 |
| | | EP 3684364 A4 | 02 June 2021 |
| | | AU 2023202125 A1 | 04 May 2023 |
| | | WO 2019055966 A3 | 18 April 2019 |
| | | CA 3075727 A1 | 21 March 2019 |
| | | US 2023203028 A1 | 29 June 2023 |
| WO 2020191056 A1 | 24 September 2020 | AU 2020240059 A1 | 28 October 2021 |
| | | WO 2020191056 A8 | 30 September 2021 |
| | | US 2022152031 A1 | 19 May 2022 |
| | | EP 3941475 A1 | 26 January 2022 |
| | | EP 3941475 A4 | 25 January 2023 |
| | | MX 2021011316 A | 13 October 2021 |
| | | SG 11202109568 TA | 28 October 2021 |
| | | JP 2022525506 A | 17 May 2022 |
| | | CA 3132580 A1 | 24 September 2020 |
| | | MA 55381 A | 26 January 2022 |
| | | IL 286481 A | 01 December 2021 |
| WO 2023036312 A1 | 16 March 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019055966 A
- WO 2020191056 A
- WO 2022001767 A


### Non-patent literature cited in the description


- *CHEMICAL ABSTRACTS*, 56-47-3